# EUROPÄISCHE PATENTSCHRIFT

(11) **EP 1 383 749 B1**
(45) Veröffentlichungstag und Bekanntmachung des Hinweises auf die Patenterteilung: **02.11.2005**
(21) Anmeldenummer: 02730119.1
(22) Anmeldetag: 08.04.2002
(51) Int. Cl.: C07D 271/06, C07D 413/04, C07D 417/04, C07D 498/04, A61K 31/425, A61K 31/4245, A61P 31/12

(54) **ARYLSULFONAMIDE ALS ANTIVIRALE MITTEL**
ARYLSULFONAMIDES AS ANTIVIRAL AGENTS
ARYLSULFONAMIDES EN TANT QU'AGENTS ANTIVIRAUX

(30) Priorität: 19.04.2001 DE 10119137; 02.10.2001 DE 10148598
(43) Veröffentlichungstag der Anmeldung: 28.01.2004
(73) Patentinhaber: Bayer HealthCare AG, 51368 Leverkusen (DE)
(72) Erfinder: WUNBERG, Tobias, 42699 Solingen (DE); BENDER, Wolfgang, 42113 Wuppertal (DE); ECKENBERG, Peter, 42115 Wuppertal (DE); HALLENBERGER, Sabine, 42115 Wuppertal (DE); HENNINGER, Kerstin, 42115 Wuppertal (DE); KELDENICH, Jörg, 42113 Wuppertal (DE); KERN, Armin, 42109 Wuppertal (DE); RADDATZ, Siegfried, 51065 Köln (DE); REEFSCHLÄGER, Jürgen, 26125 Oldenburg (DE); SCHMIDT, Gunter, 42115 Wuppertal (DE); ZIMMERMANN, Holger, 42113 Wuppertal (DE); ZUMPE, Franz, 42109 Wuppertal (DE); RADTKE, Martin, 40699 Erkrath (DE)
(86) Internationale Anmeldenummer: PCT/EP2002/003858
(87) Internationale Veröffentlichungsnummer: WO 2002/085869

(56) Entgegenhaltungen:
- WO-A-99/37291
- MISRA V S ET AL: "Search for New Antiviral Agents" JOURNAL OF THE INDIAN CHEMICAL SOCIETY, THE INDIAN CHEMICAL SOCIETY, CALCUTTA, IN, Bd. 51, Nr. 11, November 1974 (1974-11), Seiten 963-964, XP002108093 ISSN: 0019-4522
- DATABASE CROSSFIRE BEILSTEIN [Online] BEILSTEIN INSTITUT ZUR FOERDERUNG DER WISSENSCHAFTEN, FRANKFURT AM MAIN, DE; BRN 75129-34-9, XP002213430 & PATIL ET AL: JOURNAL OF THE INDIAN CHEMICAL SOCIETY, THE INDIAN CHEMICAL SOCIETY, Bd. 56, 1979, Seiten 2143-1245, CALCUTTA, IN
- DATABASE CROSSFIRE BEILSTEIN [Online] BEILSTEIN INSTITUT ZUR FOERDERUNG DER WISSENSCHAFTEN, FRANKFURT AM MAIN, DE; BRN 360648, XP002213431 & COOK ET AL: JOURNAL OF THE CHEMICAL SOCIETY, CHEMICAL SOCIETY, 1945, Seiten 182-185, LETCHWORTH, GB
- DATABASE WPI Section Ch, Week 200067 Derwent Publications Ltd., London, GB; Class B03, AN 2000-687149 XP002213432 & WO 00 63194 A (SHIONOGI & CO LTD), 26. Oktober 2000 (2000-10-26) -& EP 1 172 361 A (SHIONOGI & CO LTD) 16. Januar 2002 (2002-01-16)

## Beschreibung

Die vorliegende Erfindung betrifft neue Verbindungen, Verfahren zu ihrer Herstellung und ihre Verwendung als Arzneimittel, insbesondere als antivirale Mittel, insbesondere gegen Cytomegalieviren.

Die Verbindung 2,2-Dimethyl-N-[4-[[[4-(4-phenyl-2H-1,2,3-triazol-2-yl)phenyl]-sulfonyl]amino]phenyl]-propanamid ist als antiviral wirksam bekannt aus WO 99/37291.

Die vorliegende Erfindung betrifft Verbindungen der allgemeinen Formel (I) 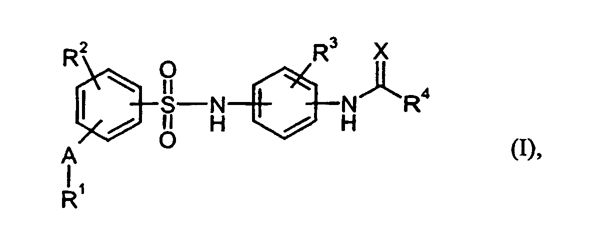 in der
- R² und R³: gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 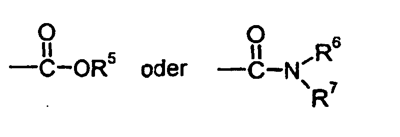 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
- A: für über ein C-Atom mit dem benachbarten Phenylring verknüpftes fünf- oder sechsgliedriges Heteroaryl mit ein bis drei Heteroatomen ausgewählt aus N, O und/oder S steht,
- R¹: für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
- R¹: substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heterocyclyl mit bis zu zwei Heteroatomen ausgewählt aus N, O und/oder S, 5- oder 6-gliedriges Heteroaryl mit einem oder mehreren Heteroatomen ausgewählt aus N, O und/oder S, -C(O)-O-R⁸, -C(O)-NR⁹R¹⁰,
-NH-C(O)-R¹¹, -NH-C(O)-C(O)-R¹² und -NH-SO₂-R¹³ besteht,
wobei
R⁸, R⁹ und R¹⁰ gleich oder verschieden sind und jeweils für Wasserstoff oder (C₁-C₆)-Alkyl stehen, oder
R⁹ und R¹⁰ zusammen mit dem Stickstoffatom, an das sie gebunden sind, einen 5- bis 6-gliedrigen Heterocyclus bilden, der ein weiteres Stickstoff- oder Sauerstoff-Heteroatom enthalten und ein- bis zweifach, gleich oder verschieden, durch (C₁-C₄)-Alkyl, welches seinerseits gegebenenfalls durch Hydroxy oder Amino substituiert ist, durch Amino, Hydroxy, (C₁-C₄)-Alkoxy, Oxo, Carboxyl oder durch (C₁-C₄)-Alkoxycarbonyl substituiert sein kann,
R¹¹ und R¹² gleich oder verschieden sind und jeweils für Trifluormethyl, (C₁-C₆)-Alkoxy, Hydroxy oder für (C₁-C₆)-Alkyl stehen, das gegebenenfalls ein- oder zweifach, gleich oder verschieden, durch Amino, (C₁-C₆)-Alkoxycarbonylamino, Mono-(C₁-C₆)-Acylamino, Hydroxy, Amidino, Guanidino, (C₁-C₆)-Alkoxycarbonyl, Carboxyl oder Phenyl substituiert ist, und
R¹³ für (C₁-C₆)-Alkyl oder (C₆-C₁₀)-Aryl, die jeweils durch Halogen, Amino, Hydroxy, (C₁-C₄)-Alkoxy oder (C₁-C₄)-Alkyl substituiert sein können, steht,
- R⁴: für (C₁-C₆)-Alkyl, das bis zu dreifach, gleich oder verschieden, durch Amino, Hydroxy, Halogen, (C₁-C₆)-Alkoxy, (C₁-C₅)-Alkanoyloxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
- X: für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

(C₁-C₆)-Alkyl steht im Rahmen der Erfindung für einen geradkettigen oder verzweigten Alkylrest mit 1 bis 6 Kohlenstoffatomen. Beispielsweise seien genannt: Methyl, Ethyl, n-Propyl, Isopropyl, n-Butyl, t-Butyl, n-Pentyl und n-Hexyl.

(C₃-C₇)-Cycloalkyl steht im Rahmen der Erfindung für eine Cycloalkylgruppe mit 3 bis 7 Kohlenstoffatomen. Beispielsweise seien genannt: Cyclopropyl, Cyclobutyl, Cyclopentyl und Cyclohexyl.

(C₁-C₆)-Alkoxy steht im Rahmen der Erfindung für einen geradkettigen oder verzweigten Alkoxyrest mit 1 bis 6 Kohlenstoffatomen. Beispielsweise seien genannt: Methoxy, Ethoxy, n-Propoxy, Isopropoxy, t-Butoxy, n-Pentoxy und n-Hexoxy. Bevorzugt sind Methoxy und Ethoxy.

(C₆-C₁₀)-Aryl steht im Rahmen der Erfindung für einen aromatischen Rest mit 6 bis 10 Kohlenstoffatomen. Bevorzugte Arylreste sind Phenyl und Naphthyl.

Aralkyl steht im Rahmen der Erfindung für (C₆-C₁₀)-Aryl, das seinerseits an (C₁-C₄)Alkyl gebunden ist. Bevorzugt ist Benzyl.

Mono-(C₁-C₆)-Alkylamino steht im Rahmen der Erfindung für eine Amino-Gruppe mit einem geradkettigen, verzweigten oder cyclischen Alkylsubstituenten, der 1 bis 6 Kohlenstoffatome aufweist. Beispielsweise seien genannt: Methylamino, Ethylamino, n-Propylamino, Isopropylamino, Cyclopropylamino, t-Butylamino, n-Pentylamino, Cyclopentylamino und n-Hexylamino.

Di-(C₁-C₆)-Alkylamino steht im Rahmen der Erfindung für eine Amino-Gruppe mit zwei gleichen oder verschiedenen geradkettigen, verzweigten oder cyclischen Alkylsubstituenten, die jeweils 1 bis 6 Kohlenstoffatome aufweisen. Beispielsweise seien genannt: *N,N*-Dimethylamino, *N,N*-Diethylamino, *N*-Ethyl-*N*-methylamino, *N*-Methyl-*N*-n-propylamino, *N*-Methyl-*N*-cyclopropylamino, *N*-Isopropyl-*N*-n-propyl-amino, *N*-t-Butyl-*N*-methylamino, *N*-Ethyl-*N*-n-pentylamino und *N*-n-Hexyl-*N*-methylamino.

(C₁-C₆)-Alkoxycarbonyl steht im Rahmen der Erfindung für einen geradkettigen oder verzweigten Alkoxyrest mit 1 bis 6 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.

(C₁-C₆)-Alkoxycarbonylamino steht im Rahmen der Erfindung für eine Amino-Gruppe mit einem geradkettigen oder verzweigten Alkoxycarbonylsubstituenten, der im Alkoxyrest 1 bis 6 Kohlenstoffatome aufweist und über die Carbonylgruppe verknüpft ist. Bevorzugt ist ein Alkoxycarbonylamino-Rest mit 1 bis 4 Kohlenstoffatomen im Alkoxyrest. Beispielhaft und vorzugsweise seien genannt: Methoxycarbonylamino, Ethoxycarbonylamino, n-Propoxycarbonylamino und t-Butoxycarbonylamino.

Mono-(C₁-C₆)-Acylamino steht im Rahmen der Erfindung für eine Amino-Gruppe mit einem geradkettigen oder verzweigten Alkanoylsubstituenten, der 1 bis 6 Kohlenstoffatome aufweist und über die Carbonylgruppe verknüpft ist. Bevorzugt ist ein Monoacylamino-Rest mit 1 bis 2 Kohlenstoffatomen. Beispielhaft und vorzugsweise seien genannt: Formamido, Acetamido, Propionamido, n-Butyramido und Pivaloylamido.

(C₁-C₅)-Alkanoyloxy steht im Rahmen der Erfindung vorzugsweise für einen geradkettigen oder verzweigten Alkylrest mit 1 bis 5 Kohlenstoffatomen, der in der 1-Position ein doppelt gebundenes Sauerstoffatom trägt und in der 1-Position über ein weiteres Sauerstoffatom verknüpft ist. Bevorzugt ist ein geradkettiger oder verzweigter Alkanoyloxy-Rest mit 1 bis 3 Kohlenstoffatomen. Beispielhaft und vorzugsweise seien genannt: Acetoxy, Propionoxy, n-Butyroxy, i-Butyroxy und Pivaloyloxy.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

5- bis 10-gliedriges Heteroaryl steht im Rahmen der Erfindung für 5- bis 10-gliedrige, Heteroatome enthaltende aromatische Ringe, die 1 bis 4 Heteroatome enthalten können, die ausgewählt werden aus O, S und N, und schließt beispielsweise ein: Pyridyl, Furyl, Thienyl, Pyrrolyl, Imidazolyl, Pyrazolyl, Pyrazinyl, Pyrimidinyl, Pyridazinyl, Indolicenyl, Indolyl, Benzo[b]thienyl, Benzo[b]furyl, Indazolyl, Chinolyl, Isochinolyl, Naphthyridinyl, Chinazolinyl, etc.

5- bis 10-gliedriges bzw. 5- bis 6-gliedriges gesättigtes oder teilweise ungesättigtes Heterocyclyl mit bis zu 3 Heteroatomen aus der Reihe S, N und/oder O steht im Rahmen der Erfindung im allgemeinen für einen mono- oder bicyclischen Heterocyclus, der eine oder mehrere Doppelbindungen enthalten kann und der über ein Ringkohlenstoffatom oder ein Ringstickstoffatom verknüpft ist. Beispielsweise seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-3-yl, 1,2-Dihydropyridin-1-yl, 1,4-Dihydropyridin-1-yl, Piperazin-1-yl, Morpholin-1-yl, Azepin-1-yl, 1,4-Diazepin-1-yl. Bevorzugt sind Piperidinyl, Morpholinyl und Pyrrolidinyl.

Ein 3- bzw. 5-verknüpftes 1,2,4-Oxadiazol steht für ein Oxadiazol, das über das 3- bzw. 5-Ringkohlenstoffatom an das Phenylsulfonamid gebunden ist.

Als Salze sind im Rahmen der Erfindung pharmakologisch verträgliche Salze der erfindungsgemäßen Verbindungen bevorzugt.

Pharmakologisch verträgliche Salze der erfindungsgemäßen Verbindungen können Säureadditionssalze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin, oder abgeleitet von natürlichen Aminosäuren wie beispielsweise Glycin, Lysin, Arginin oder Histidin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Die Erfindung betrifft auch Verbindungen der allgemeinen Formel (I),
in der
- R² und R³: gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 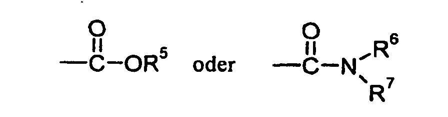 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
- A: für über ein C-Atom mit dem benachbarten Phenylring verknüpftes fünf- oder sechsgliedriges Heteroaryl mit ein bis drei Heteroatomen ausgewählt aus N, O und/oder S steht,
- R¹: für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
- R¹: substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heteroaryl mit einem oder mehreren Heteroatomen ausgewählt aus N, O und/oder S, -C(O)-O-R⁸, -C(O)-NR⁹R¹⁰ und -NH-C(O)-R¹¹ besteht,
wobei
R⁸, R⁹ und R¹⁰ gleich oder verschieden sind und jeweils für Wasserstoff oder (C₁-C₆)-Alkyl stehen, und
R¹¹ für (C₁-C₆)-Alkyl steht, das gegebenenfalls ein- oder zweifach, gleich oder verschieden, durch Amino, Hydroxy, Guanidino, Carboxyl oder Phenyl substituiert ist,
- R⁴: für (C₁-C₆)-Alkyl, das bis zu dreifach, gleich oder verschieden, durch Amino, Hydroxy, Halogen, (C₁-C₆)-Alkoxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
- X: für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

Die Erfindung betrifft bevorzugt Verbindungen der allgemeinen Formel (I),
in der
- R² und R³: gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 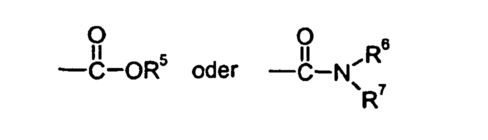 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
- A: für über ein C-Atom mit dem benachbarten Phenylring verknüpftes fünf- oder sechsgliedriges Heteroaryl mit ein bis drei Heteroatomen ausgewählt aus N, O und/oder S steht,
- R¹: für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
- R¹: substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heteroaryl mit einem oder mehreren Heteroatomen ausgewählt aus N, O und/oder S, -C(O)-O-R⁸, -C(O)-NR⁹R¹⁰ und -NH-C(O)-R¹¹ besteht,
wobei
R⁸, R⁹ und R¹⁰ gleich oder verschieden sind und jeweils für Wasserstoff oder (C₁-C₆)-Alkyl stehen, und
R¹¹ für (C₁-C₆)-Alkyl steht, das gegebenenfalls ein- oder zweifach, gleich oder verschieden, durch Amino, Hydroxy, Guanidino, Carboxyl oder Phenyl substituiert ist,
- R⁴: für (C₁-C₆)-Alkyl, das bis zu dreifach, gleich oder verschieden, durch Amino, Hydroxy, Halogen, (C₁-C₆)-Alkoxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
- X: für Sauerstoff steht,
und worin stickstoffhaltige Heterocyclen auch als N-Oxide vorliegen können,
sowie deren Tautomere, Stereoisomere, stereoisomere Gemische und deren pharmakologisch verträglichen Salze.

Die Erfindung betrifft bevorzugt auch Verbindungen der allgemeinen Formel (I),
in der
- R² und R³: gleich oder verschieden sind und für Wasserstoff oder Halogen stehen,
- A: für den Rest (A-I) 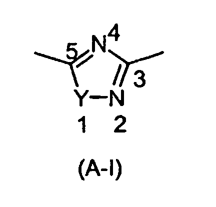 steht, der über eines der Kohlenstoffatome der Positionen 3 oder 5 mit dem benachbarten Phenylring verbunden ist,
und in dem
- Y: für Sauerstoff oder Schwefel steht,
oder
- A: für den Rest (A-II) 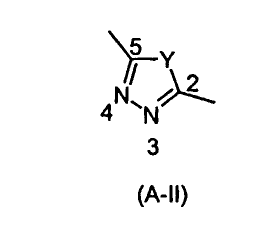 steht, der über eines der Kohlenstoffatome der Positionen 2 oder 5 mit dem benachbarten Phenylring verbunden ist,
und in dem
Y für Sauerstoff oder Schwefel steht,
- R¹: für 5- bis 10-gliedriges Heteroaryl oder 5- oder 10-gliedriges Heterocyclyl mit jeweils bis zu drei Heteroatomen ausgewählt aus N, O und/oder S oder für Phenyl steht, wobei
- R¹: substituiert sein kann mit ein bis drei Substituenten ausgewählt aus der Gruppe, die aus (C₁-C₄)-Alkyl, das seinerseits gegebenenfalls durch Hydroxy oder Amino substituiert ist, Hydroxy, Oxo, Halogen, Amino, Mono-(C₁-C₄)-alkylamino, Di-(C₁-C₄)-alkylamino und -NH-C(O)-R¹¹ besteht,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
- R⁴: für (C₁-C₄)-Alkyl, das bis zu dreifach, gleich oder verschieden, durch Amino, Hydroxy, Fluor, Chlor oder (C₁-C₄)-Alkoxy substituiert sein kann, oder
für (C₃-C₅)-Cycloalkyl, das bis zu dreifach, gleich oder verschieden, durch Amino, Hydroxy, Fluor, Chlor, (C₁-C₄)-Alkoxy oder (C₁-C₄)-Alkyl, welches seinerseits gegebenenfalls bis zu dreifach, gleich oder verschieden, durch Amino, Hydroxy, Fluor, Chlor oder (C₁-C₄)-Alkoxy substituiert ist, substituiert sein kann, steht,
und in der
- X: für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

Die Erfindung betrifft besonders bevorzugt Verbindungen der allgemeinen Formel (I),
in der
- R² und R³: gleich oder verschieden sind und für Wasserstoff oder Halogen stehen,
- A: für den Rest (A-I) 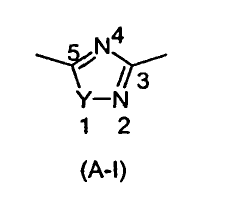 steht, der über eines der Kohlenstoffatome der Positionen 3 oder 5 mit dem benachbarten Phenylring verbunden ist,
und in dem
Y für Sauerstoff oder Schwefel steht, oder
- A: für den Rest (A-II) 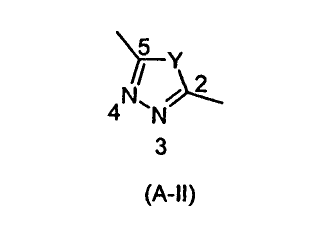 steht, der über eines der Kohlenstoffatome der Positionen 2 oder 5 mit dem benachbarten Phenylring verbunden ist,
und in dem
Y für Sauerstoff oder Schwefel steht,
- R¹: für 5- bis 10-gliedriges Heteroaryl oder 5- oder 10-gliedriges Heterocyclyl
mit jeweils bis zu drei Heteroatomen ausgewählt aus N, O und/oder S oder für Phenyl steht, wobei
- R¹: substituiert sein kann mit ein bis drei Substituenten ausgewählt aus der Gruppe, die aus (C₁-C₄)-Alkyl, das seinerseits gegebenenfalls durch Hydroxy oder Amino substituiert ist, Hydroxy, Oxo, Halogen, Amino, Mono-(C₁-C₄)-alkylamino, Di-(C₁-C₄)-alkylamino und -NH-C(O)-R¹¹ besteht,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
- R⁴: für (C₁-C₄)-Alkyl, das bis zu dreifach, gleich oder verschieden, durch Amino, Hydroxy, Fluor, Chlor oder (C₁-C₄)-Alkoxy substituiert sein kann, oder
für (C₃-C₅)-Cycloalkyl, das bis zu dreifach, gleich oder verschieden, durch Amino, Hydroxy, Fluor, Chlor, (C₁-C₄)-Alkoxy oder (C₁-C₄)-Alkyl, welches seinerseits gegebenenfalls bis zu dreifach, gleich oder verschieden, durch Amino, Hydroxy, Fluor, Chlor oder (C₁-C₄)-Alkoxy substituiert ist, substituiert sein kann, steht,
und in der
- X: für Sauerstoff steht,
sowie deren Tautomere, Stereoisomere, stereoisomere Gemische und deren pharmakologisch verträglichen Salze.

Die Erfindung betrifft besonders bevorzugt Verbindungen der allgemeinen Formel (I),
in der
- R² und R³: für Wasserstoff stehen,
- A: für einen der Reste 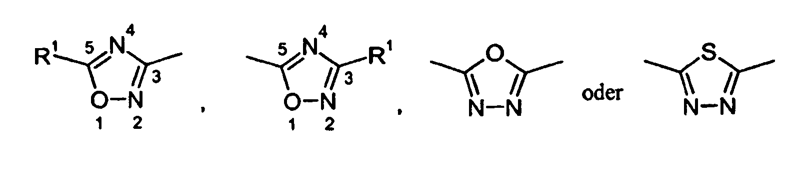 steht,
R¹ für einen Rest ausgewählt aus der Gruppe Phenyl, Pyridyl, Pyrazinyl, Thiazolyl, Thiadiazolyl, Chinolinyl, Isochinolinyl, Oxazolyl, Pyrazolyl, Imidazolyl, Pyrrolyl, Indolyl steht, wobei
R¹ substituiert sein kann mit ein bis zwei Substituenten ausgewählt aus der Gruppe Methyl, Aminomethyl, Hydroxy, Brom, Chlor, Fluor, Amino, Dimethylamino und -NH-C(O)-R¹¹,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
R⁴ für tert.-Butyl, das gegebenenfalls bis zu dreifach, gleich oder verschieden, durch Hydroxy, Fluor oder Chlor substituiert ist, oder
für Cyclopropyl oder Cyclobutyl steht, die durch Methyl substituiert sind, welches seinerseits gegebenenfalls durch Hydroxy, Fluor oder Chlor substituiert ist, und in der
- X: für Sauerstoff steht,
und worin stickstoffhaltige Heterocyclen auch als N-Oxide vorliegen können,
sowie deren Tautomere, Stereoisomere, stereoisomere Gemische und deren pharmakologisch verträglichen Salze.

Die Erfindung betrifft insbesondere bevorzugt Verbindungen der allgemeinen Formel (I),
in der
- R² und R³: für Wasserstoff stehen,
- A: für einen der Reste 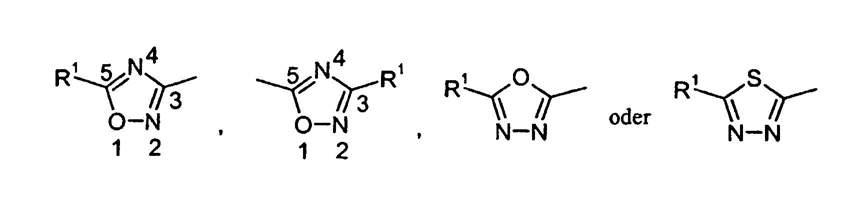 steht,
- R¹: für einen Rest ausgewählt aus der Gruppe Phenyl, Pyridyl, Pyrazinyl, Thiazolyl, Thiadiazolyl, Chinolinyl, Isochinolinyl, Oxazolyl, Pyrazolyl, Imidazolyl, Pyrrolyl, Indolyl steht, wobei
- R¹: substituiert sein kann mit ein bis zwei Substituenten ausgewählt aus der Gruppe Methyl, Aminomethyl, Hydroxy, Brom, Chlor, Fluor, Amino, Dimethylamino und -NH-C(O)-R¹¹,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
- R⁴: für tert.-Butyl, das gegebenenfalls bis zu dreifach, gleich oder verschieden, durch Hydroxy, Fluor oder Chlor substituiert ist, oder
für Cyclopropyl oder Cyclobutyl steht, die durch Methyl substituiert sind, welches seinerseits gegebenenfalls durch Hydroxy, Fluor oder Chlor substituiert ist,
und in der
- X: für Sauerstoff steht.

In einer bevorzugten Ausführungsform betrifft die Erfindung Verbindungen der allgemeinen Formel (Ia) 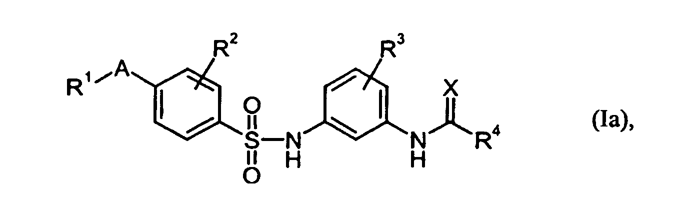 in der
- R¹, R⁴, A und X: die oben angegebenen Bedeutungen haben,
und
- R² und R³: gleich oder verschieden sind und für Wasserstoff Hydroxy, Halogen, Nitro, Cyano, Trifluormethyl, Trifluormethoxy, (C₁-C₆)-Alkyl oder (C₁-C₆)-Alkoxy stehen.

In einer weiteren bevorzugten Ausführungsform betrifft die Erfindung solche Verbindungen der allgemeinen Formel (I),
in denen
- R⁴: für einen der Reste 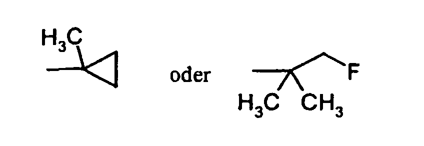 steht.

In einer weiteren bevorzugten Ausführungsform betrifft die Erfindung solche Verbindungen der allgemeinen Formel (I),
in denen
- A: für ein 3-verknüpftes 1,2,4-Oxadiazol steht.

Ganz besonders bevorzugte Verbindungen der vorliegenden Erfindung sind Sulfonamide, die ausgewählt sind aus der Gruppe der folgenden Verbindungen:

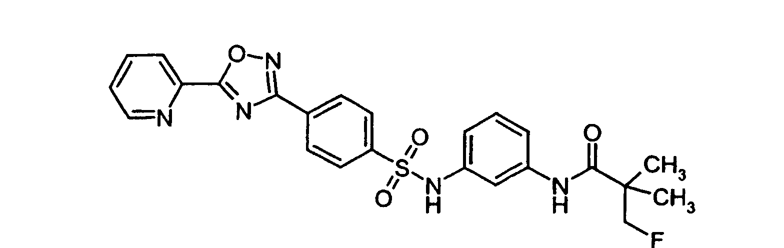

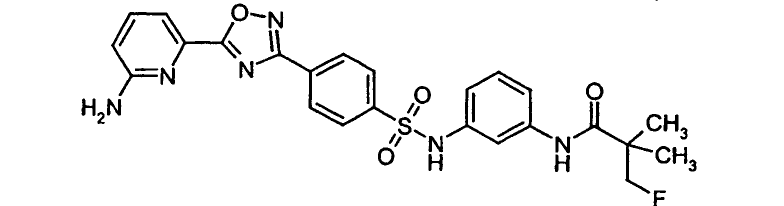

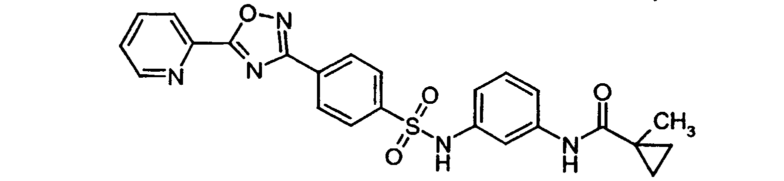

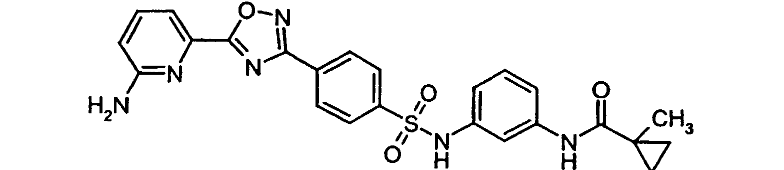

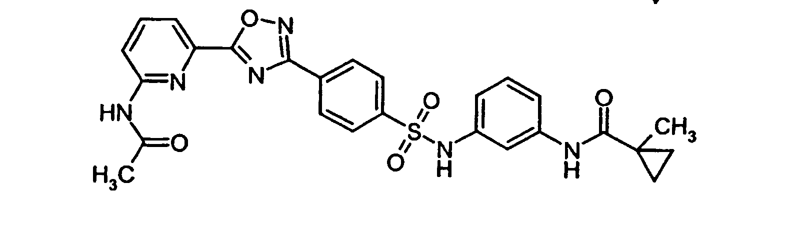

Die Erfindung betrifft weiter Verfahren zur Herstellung von Verbindungen der allgemeinen Formel (I), dadurch gekennzeichnet, dass man
[A] Nitro-Aniline der allgemeinen Formel [A-1] 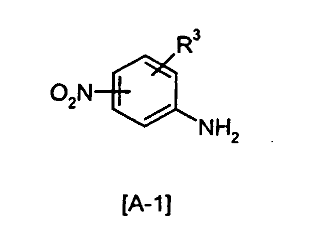 worin
   - R³: die oben angegebene Bedeutung hat,
   mit Verbindungen der allgemeinen Formel [A-2] 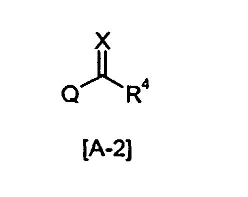 worin
   - X und R⁴: eine der oben angegebenen Bedeutungen haben,
   und
   - Q: für eine Abgangsgruppe, z.B. Halogen, vorzugsweise für Chlor oder Brom steht,
   in inerten Lösemitteln in Gegenwart einer Base zu Verbindungen der allgemeinen Formel [A-3] 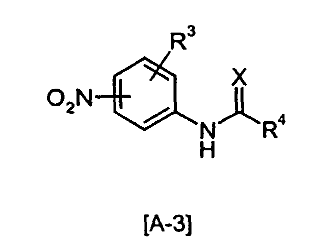 worin
   - X, R³ und R⁴: eine der oben angegebenen Bedeutungen haben,
   umsetzt,
   und
[B] die Nitro-Aromaten der allgemeinen Formel [A-3] in inerten Lösemitteln beispielsweise in Gegenwart von Übergangsmetallkatalysatoren und Wasserstoff zu aromatischen Aminen der allgemeinen Formel [B-1] 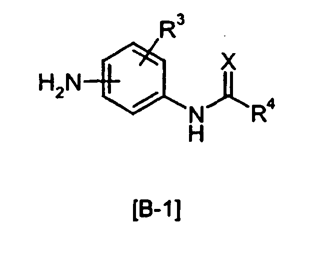 worin
   - X, R³ und R⁴: eine der oben angegebenen Bedeutungen haben,
   reduziert,
   und
[C] Amine der allgemeinen Formel [B-1] mit Sulfonsäurederivaten der allgemeinen Formel [C-1] 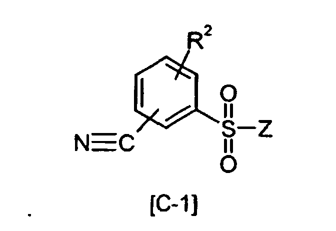 worin
   - R²: die oben angegebene Bedeutung hat,
   und
   - Z: für eine Abgangsgruppe, z.B. Halogen, vorzugsweise für Chlor oder Brom steht,
   in inerten Lösemitteln, in Gegenwart einer Base, zu Verbindungen der allgemeinen Fomel [C-2] 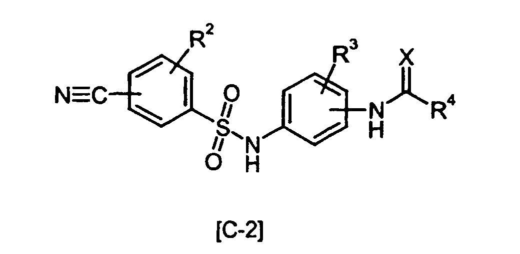 worin
   - X, R², R³ und R⁴: eine der oben angegebenen Bedeutungen haben,
   umsetzt,
   und
[D] die Nitrile der allgemeinen Formel [C-2] in polaren protischen Lösemitteln, beispielsweise Alkoholen, bei erhöhter Temperatur, vorzugsweise der Siedetemperatur des Lösemittels, in Gegenwart einer Base mit Hydroxylamin zu Amidoximen der allgemeinen Formel [D-1] 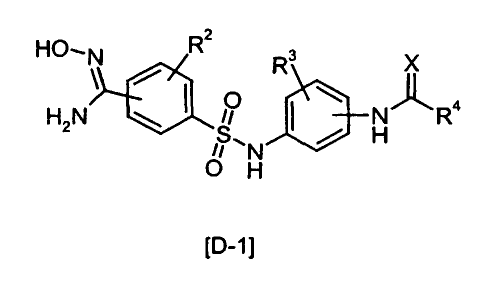 worin
   - X, R², R³ und R⁴: eine der oben angegebenen Bedeutungen haben,
   umsetzt,
   und
[E] Amidoxime der allgemeinen Formel [D-1] mit einer Carbonsäure der allgemeinen Formel [E-1]

   R¹-COOH [E-1]

   worin
   - R¹: die oben angegebene Bedeutung hat,
   in Gegenwart eines Kondensationsmittels, beispielsweise Benzotriazolyl-N-Oxi-tris(dimethylamino)phosphonium-Hexafluorophosphat (PyBOP), oder anderen aus der Peptidchemie bekannten Aktivierungsreagenzien sowie Säurechloriden, und einer Base in einem polaren, aprotischen Lösemittel, beispielsweise Tetrahydrofuran, acyliert, das acylierte Amidoxim als Rohprodukt isoliert und nachfolgend in einem hochsiedenden, polaren Lösemittel, beispielweise DMF, bei erhöhter Temperatur zum 1,2,4-Oxadiazol cyclisiert.
   Das erfindungsgemäße Verfahren zur Herstellung von über die Position 3 verknüpften 1,2,4-Oxadiazolen wird durch das folgende Formelschema beispielhaft erläutert: 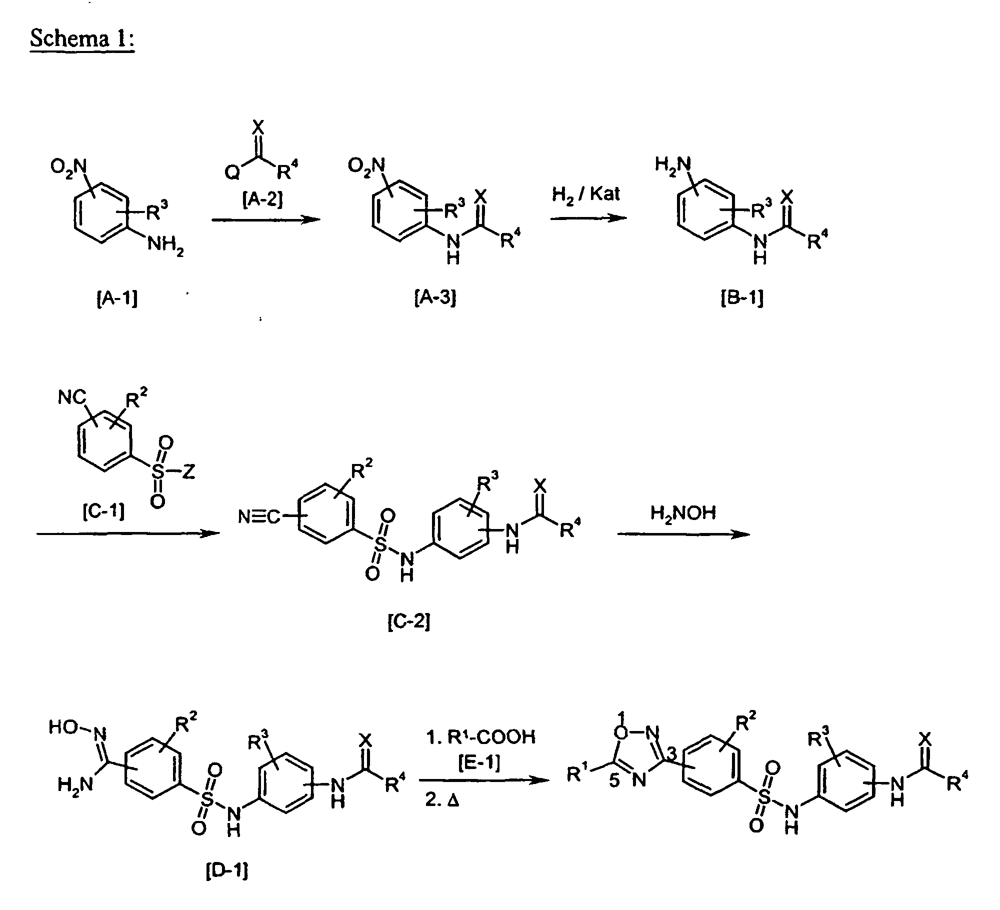
   Die Erfindung betrifft weiter Verfahren zur Herstellung von Verbindungen der allgemeinen Formel (I), dadurch gekennzeichnet, dass man
[F] Sulfonylhalogenide der allgemeinen Formel [F-1] 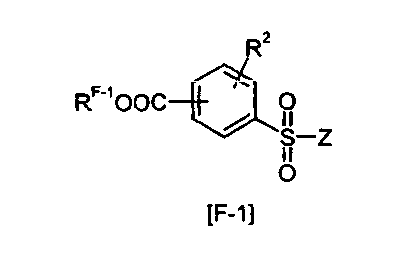 worin
   - R² und Z: die oben angegebene Bedeutung haben,
   und
   - R^{F-1}: für (C₁-C₄)-Alkyl, Aralkyl oder eine Carbonsäureschutzgruppe steht,
   in Gegenwart einer Base mit Anilinen der allgemeinen Formel [B-1] zu Sulfonamiden der allgemeinen Formel [F-2] 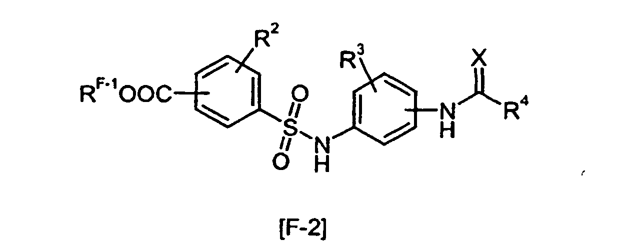 worin
   - R^{F-1}, R², R³, R⁴ und X: die oben angegebene Bedeutung haben,
   umsetzt,
   und nachfolgend aus den Verbindungen der allgemeinen Formel [F-2] die Gruppe R^{F-1} beispielsweise in Gegenwart von Hydroxyl-Anionen abspaltet und zu Sulfonamiden der allgemeinen Formel [F-3] umsetzt, 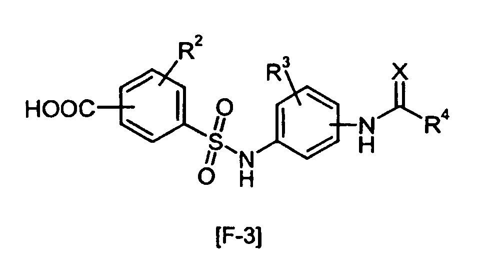 und
[G] Amid-Oxime der allgemeinen Formel [G-1] 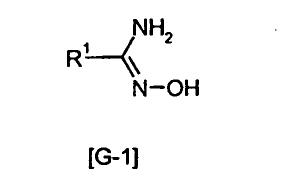 worin
   - R¹: die oben angegebene Bedeutung hat,
   mit Verbindungen der allgemeinen Formel [F-3] zu Verbindungen der allgemeinen Formel [G-2] kondensiert, 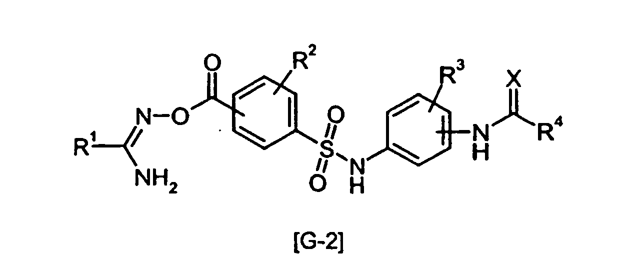 worin
   - R¹, R², R³, R⁴ und X: die oben angegebene Bedeutung haben,
   und
[H] Verbindungen der allgemeinen Formel [G-2] thermisch zu den erfindungsgemäßen 5-verknüpften 1,2,4-Oxadiazolen der allgemeinen Formel [H-1] 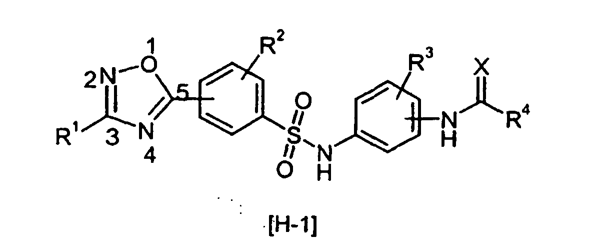 worin
   - R¹, R², R³, R⁴ und X: die oben angegebene Bedeutung haben,
   cyclisiert.

Die erfindungsgemäßen Verbindungen können nach folgenden Formelschemata beispielhaft hergestellt werden:

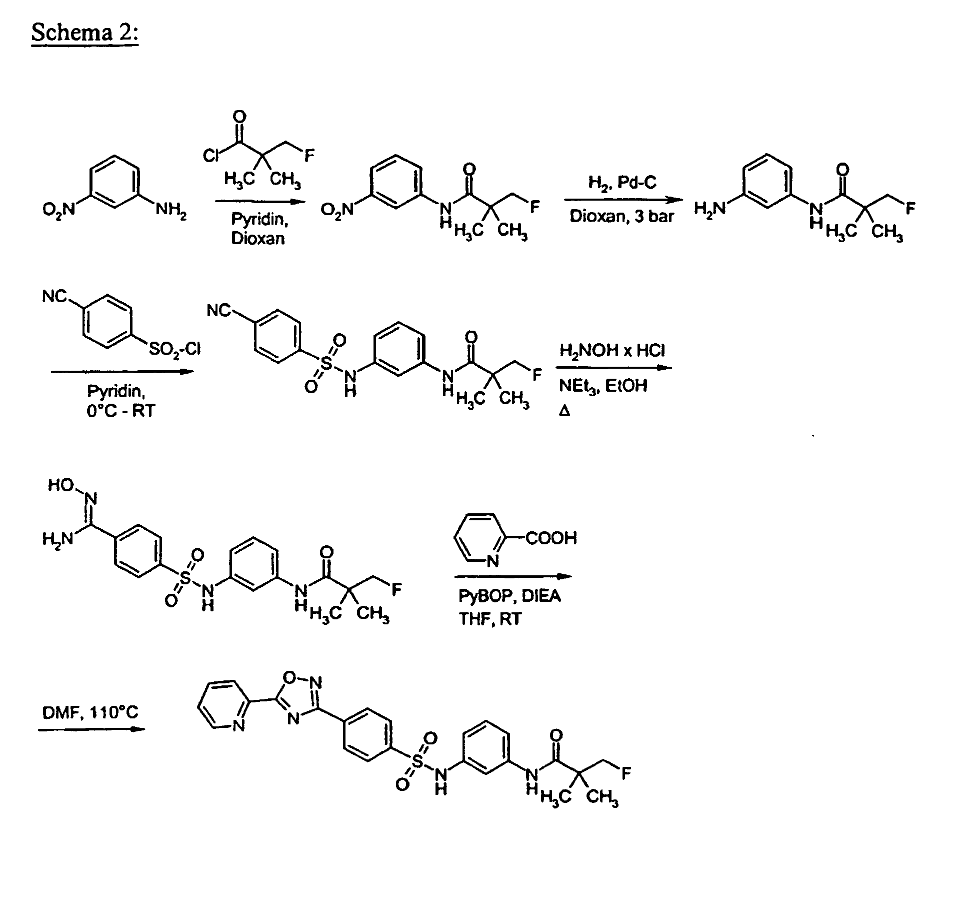

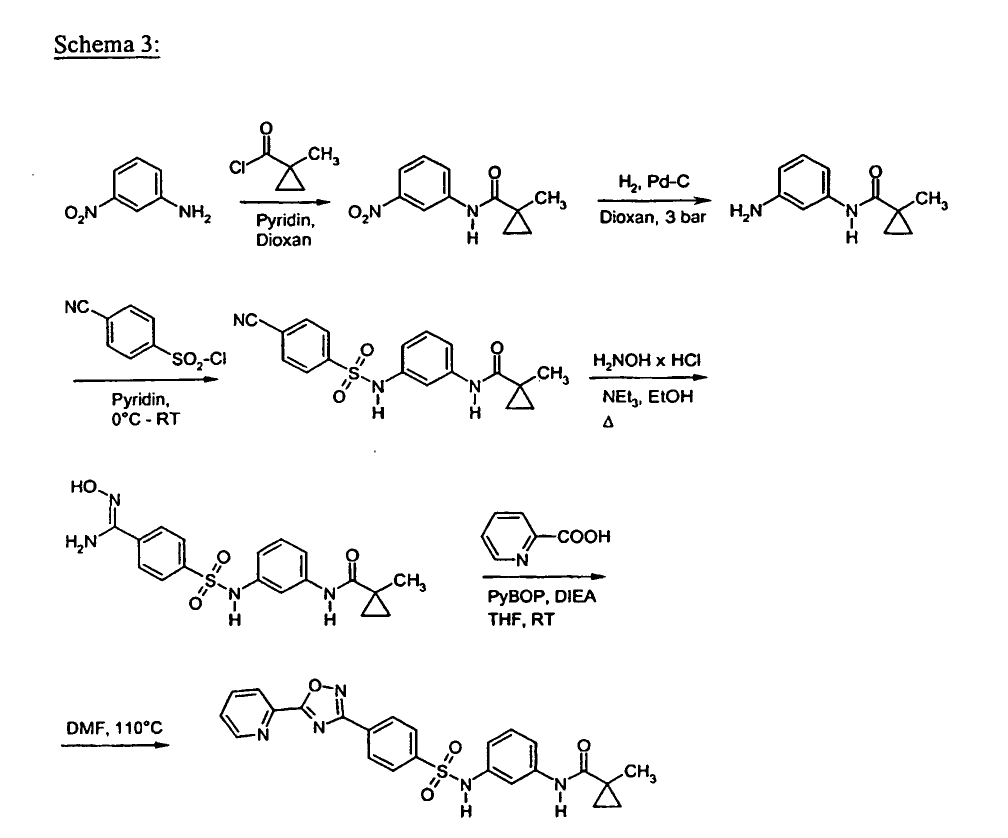

Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether, z.B. Diethylether, Glykolmono- oder -dimethylether, Dioxan oder Tetrahydrofuran, oder Alkohole wie Methanol, Ethanol, n-Propanol, iso-Propanol, n-Butanol oder tert.-Butanol, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Dioxan und Dioxan/Wasser und insbesondere die im Textabschnitt "Allgemeine Arbeitsvorschriften" erwähnten Lösemittel.

Als Basen eignen sich organische Amine wie Tri-(C₁-C₆)-alkylamine, beispielsweise Triethylamin, oder Heterocyclen wie Pyridin, Methylpiperidin, Piperidin oder N-Methylmorpholin. Bevorzugt sind Triethylamin und Pyridin.

Die Basen werden im allgemeinen in einer Menge von 0,1 mol bis 5 mol, bevorzugt von 1 mol bis 3 mol jeweils bezogen auf 1 mol der Verbindungen der allgemeinen Formeln [A-1], [B-1], [C-2], [D-1] und [E-1] eingesetzt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0°C bis 150°C, vorzugsweise bei 0°C bis 30°C und bei Normaldruck durchgeführt. Die Umsetzung der Verbindungen [G-2] zu [H-1] erfolgt bei erhöhter Temperatur, vorzugsweise bei Temperaturen oberhalb von 100°C.

Die Reduktionen können im allgemeinen durch Wasserstoff in inerten organischen Lösemitteln wie Dimethylformamid, Alkoholen, Ethern oder Essigsäureestern, oder deren Gemischen, mit Katalysatoren wie Raney-Nickel, Palladium, Palladium auf Kohle oder Platin, oder mit Hydriden oder Boranen, oder mit anorganischen Reduktionsmitteln wie beispielsweise Zinn(II)chlorid, in inerten Lösemitteln, gegebenenfalls in Anwesenheit eines Katalysators durchgeführt werden. Bevorzugt ist Palladium auf Kohle.

Die Umsetzung kann bei normalem oder bei erhöhtem Druck durchgeführt werden (z.B. 1 bis 5 bar). Im allgemeinen arbeitet man bei Normaldruck. Hydrierungen werden bevorzugt unter erhöhtem Druck, im allgemeinen bei 3 bar, durchgeführt.

Die Reduktionen werden im allgemeinen in einem Temperaturbereich von 0°C bis +60°C, vorzugsweise bei +10°C bis +40°C durchgeführt.

Als Lösemittel für die Acylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylformamid, Acetonitril oder Aceton. Ebenso ist es möglich, Gemische der genannten Lösemittel zu verwenden. Bevorzugt sind Dichlormethan, Tetrahydrofuran und Pyridin.

Die Acylierung wird in den oben aufgeführten Lösemitteln bei Temperaturen von 0°C bis +150°C, vorzugsweise bei Raumtemperatur bis +100°C und bei Normaldruck durchgeführt.

Die Verbindungen der allgemeinen Formeln [A-1], [A-2], [C-1], [E-1], [F-1] und [G-1] sind an sich bekannt oder nach literaturbekannten Methoden herstellbar.

Weitere Verbindungen der allgemeinen Formel (I), in denen A für ein 1,3,4-Oxadiazol steht, können beispielsweise wie nachfolgend gemäß dem Schema 4 angefiihrt an einem polymeren Träger mit dem IRORI-System nach der "Split & Mix"-Methode hergestellt werden:

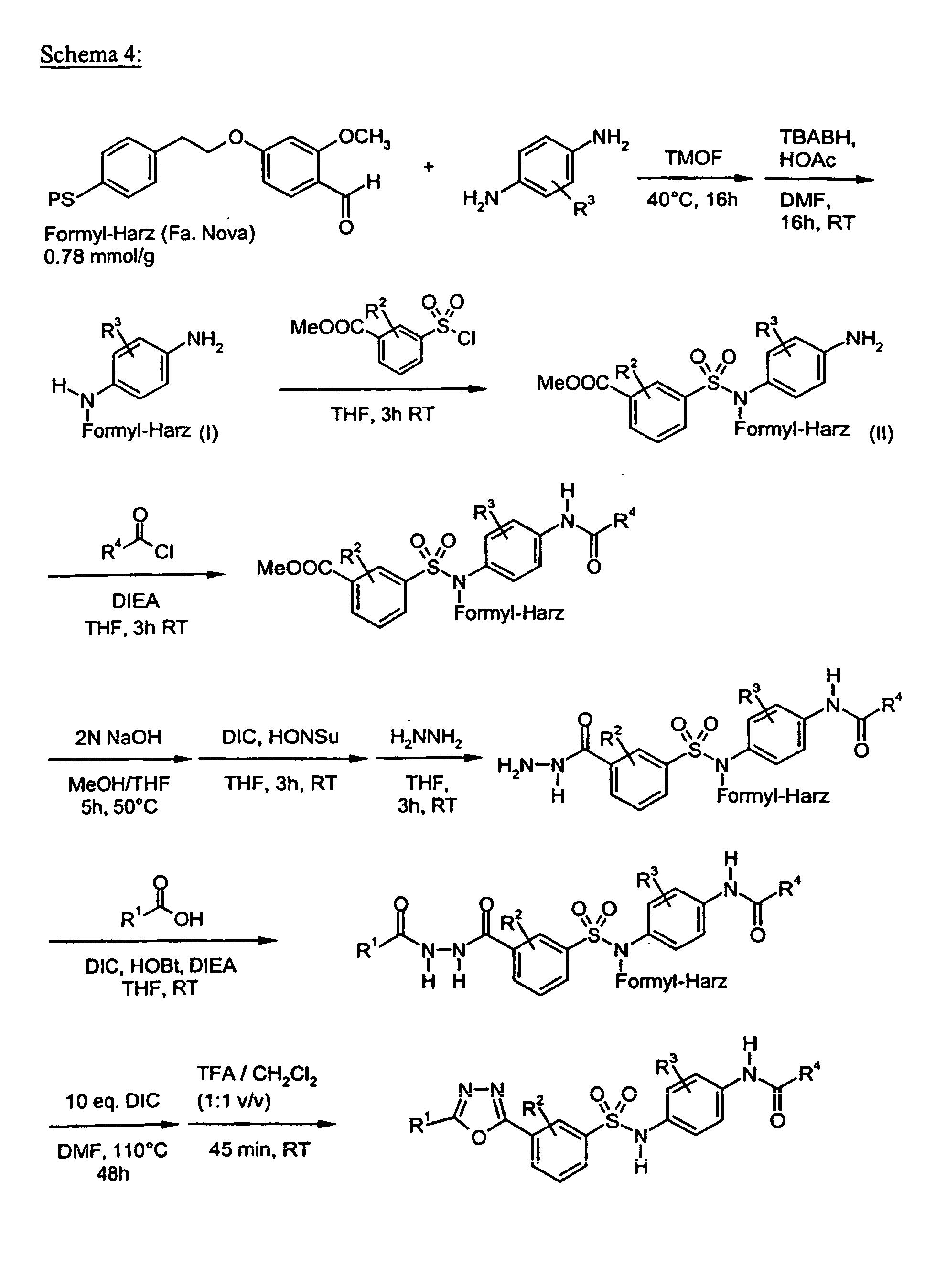

Weiter werden Verbindungen der allgemeinen Formel (I) beispielsweise durch ein Verfahren gemäß Schema 5 erhalten, das in einem gemischten Ansatz aus Festphasensynthese und Synthese in Lösung durchgeführt wird.

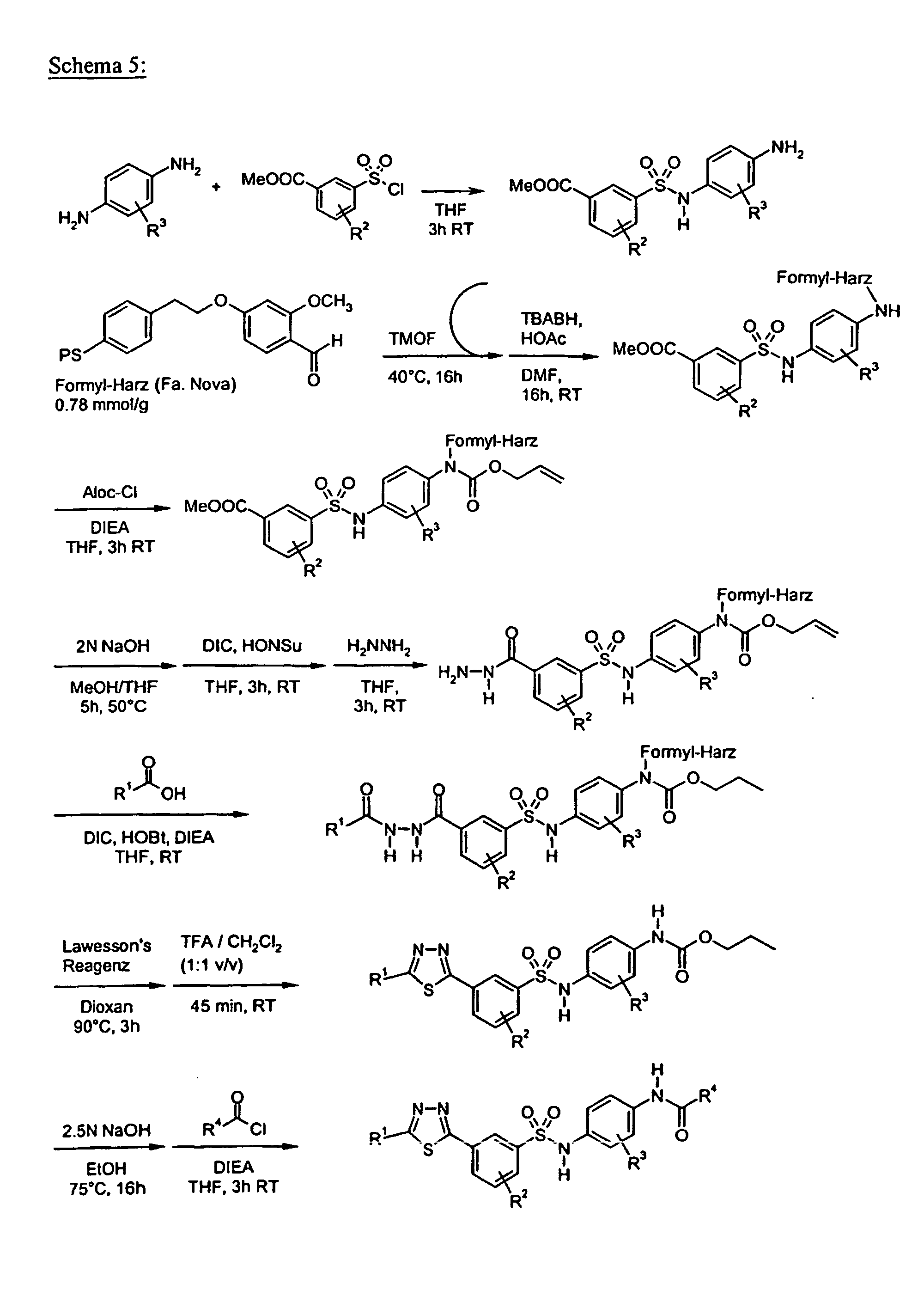

Die gemäß Schema 4 und 5 aufgezeigten Verfahren erlauben also die Herstellung weiterer erfindungsgemäßer Verbindungen der allgemeinen Formel (I), worin
- X: für Sauerstoff
und
- A: für den Rest (A-II) 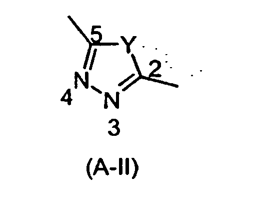 steht, der über eines der Kohlenstoffatome der Positionen 2 oder 5 mit dem benachbarten Phenylring verbunden ist,
und in dem
Y für Sauerstoff steht,
indem Hydrazide der allgemeinen Formel [H-2] 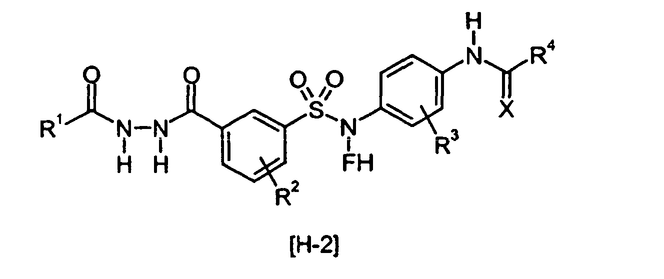 worin X, R¹, R², R³, R⁴ eine der oben angegebenen Bedeutungen haben,
und
- FH: für Wasserstoff, eine Amino-Schutzgruppe oder einen polymeren Träger steht,
unter Wasserabspaltung zu den Verbindungen der allgemeinen Formel (I) cyclisiert werden.

Sie erlauben weiter, Verbindungen der allgemeinen Formel (I) herzustellen, worin
- X: für Sauerstoff
und
- A: für den Rest (A-II) 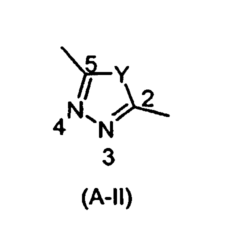 steht, der über eines der Kohlenstoffatome der Positionen 2 oder 5 mit dem benachbarten Phenylring verbunden ist,
und in dem
Y für Schwefel steht,
indem man Hydrazide der allgemeinen Formel [H-3] 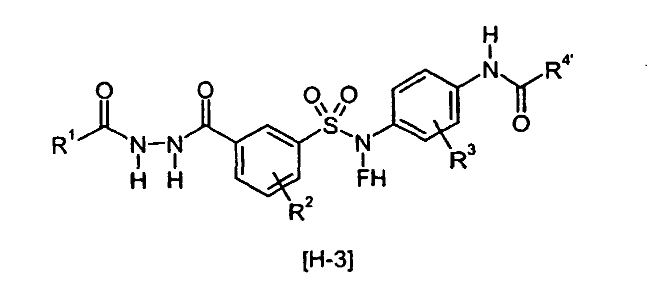 worin R¹, R², R³ die oben angegebene Bedeutung haben,
- FH: für Wasserstoff, eine Amino-Schutzgruppe oder einen polymeren Träger steht,
und
- R^{4'}: für (C₁-C₆)-Alkoxy, (C₁-C₆)-Alkenoxy oder Aralkoxy steht,
in Gegenwart eines Thio-Donors, vorzugsweise Lawesson's Reagenz, zu solchen Verbindungen der allgemeinen Formel (I) cyclisiert, in denen Y für Schwefel steht, dann die Gruppe -C(O)-R^{4'} abspaltet, und abschließend mit Verbindungen der allgemeinen Formel 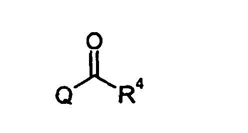 worin R⁴ und Q die oben angegebene Bedeutung haben,
umsetzt.

Die erfindungsgemäßen Verbindungen der allgemeinen Formel (I) zeigen ein nicht vorhersehbares überraschendes Wirkspektrum. Sie zeigen eine antivirale Wirkung gegenüber Vertretern der Gruppe der Herpes viridae, besonders gegenüber dem humanen Cytomegalievirus (HCMV). Sie sind somit zur Behandlung und Prophylaxe von Erkrankungen, die durch Herpes viridae, insbesondere von Erkrankungen, die durch humane Cytomegalieviren hervorgerufen werden, geeignet.

Die Verbindungen der allgemeinen Formel (I) können aufgrund ihrer besonderen Eigenschaften zur Herstellung von Arzneimitteln, die zur Prophylaxe oder Behandlung von Krankheiten, insbesondere viraler Erkrankungen, geeignet sind, verwendet werden.

Die erfindungsgemäßen Verbindungen stellen aufgrund ihrer Eigenschaften wertvolle Wirkstoffe zur Behandlung und Prophylaxe von humanen Cytomegalievirus-Infektionen und dadurch hervorgerufenen Erkrankungen dar. Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.

Die neuen Wirkstoffe können alleine und bei Bedarf auch in Kombination mit anderen antiviralen Wirkstoffen wie beispielsweise Gancyclovir oder Acyclovir eingesetzt werden.

### Biologische Testbeschreibungen:

### in vitro-Wirkung:

### Anti-HCMV- (Anti-Humanes Cytomegalie-Virus) und Anti-MCMV- (Anti-Murines Cytomegalie-Virus) Zytopathogenitätstests:

Die Testverbindungen wurden als 50 millimolare (mM) Lösungen in Dimethylsulfoxid (DMSO) eingesetzt. Ganciclovir, Foscarnet und Cidofovir dienten als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung wurden 1:2-Verdünnungen mit je 50 µl Medium bis zur Reihe 11 der 96-Well-Platte durchgeführt. Die Wells in den Reihen 1 und 12 enthielten je 50 µl Medium. In die Wells wurden dann je 150 µl einer Suspension von 1 x 10⁴ Zellen (humane Lungenfibroblasten [HELF]) pipettiert (Reihe 1 = Zellkontrolle) bzw. in die Reihen 2-12 ein Gemisch von HCMV-infizierten und nichtinfizierten HELF-Zellen (M.O.I. = 0,001 - 0,002), d.h. 1-2 infizierte Zellen auf 1000 nicht-infizierte Zellen. Die Reihe 12 (ohne Substanz) diente als Viruskontrolle. Die End-Testkonzentrationen lagen bei 250 - 0,0005 µM. Die Platten wurden 6 Tage bei 37°C / 5 % CO₂ inkubiert, d.h. bis in den Viruskontrollen alle Zellen infiziert waren (100 % cytopathogener Effekt [CPE]). Die Wells wurden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach wurden die Platten mit einem Overhead-Mikroskop (Plaque multiplier der Firma Technomara) visuell ausgewertet.

Die folgenden Daten konnten von den Testplatten ermittelt werden:
CC₅₀ (HELF) = Substanzkonzentration in µM, bei der im Vergleich zur unbehandelten Zellkontrolle keine sichtbaren cytostatischen Effekte auf die Zellen erkennbar waren;
EC₅₀ (HCMV) = Substanzkonzentration in µM, die den CPE (cytopathischen Effekt) um 50 % im Vergleich zur unbehandelten Viruskontrolle hemmt;
SI (Selektivitätsindex) = CC₅₀ (HELF) / EC₅₀ (HCMV).

Der Anti-MCMV-Test wurde in Abweichung zum vorstehend für HCMV beschriebenen Verfahren mit folgenden Veränderungen durchgeführt: Eine zellfreie Virussuspension wurde mit einer konzentrierten Zellsuspension (3T3-Mauszellen) gemischt und 15 Minuten zur Adsorption der Viren inkubiert, bevor auf 1,3 x 10⁵ Zellen/ml mit Medium verdünnt wurde mit einer End-Multiplizität der Infektion (M.O.I.) von 0,05 - 0,1 und mit je 150 µl in die Wells verteilt wurde. Die Inkubationszeit betrug 5 Tage.

Repräsentative Wirkdaten für die erfindungsgemäßen Verbindungen sind in Tabelle 1 wiedergegeben:

**Tabelle 1**

| **Beispiel Nr.** | **HELF CC**_{**50**} **[µM]** | **HCMV EC**_{**50**} **[µM]** | **SI HCMV** | **3T3 CC**_{**50**} **[µM]** | **MCMV EC**_{**50**} **[µM]** | **SI MCMV** |
|---|---|---|---|---|---|---|
| **1** | 110 | 0,055 | 2000 | 33 | 0,019 | 1737 |
| **2** | <16 | 0,05 | <320 | 0,9 | 0,045 | 20 |
| **3** | <140 | 0,018 | <7778 | 23 | 0,008 | 2875 |
| **4** | >63 | 0,01 | >6300 | 11 | 0,015 | 733 |
| **5** | >16 | 0,016 | >1000 | >31 | 0,014 | >2214 |
| **6** | 39 | 0,02 | 2053 | 2,9 | 0,041 | 71 |
| **7** | 47 | 0,025 | 1880 | 12 | 0,025 | 480 |
| **8** | >2,2 | 0,02 | >110 | >3,9 | 0,024 | >163 |
| **9** | 39 | 0,018 | 2167 | 4 | 0,068 | 59 |
| **10** | >3,9 | 0,008 | >488 | >7,8 | 0,007 | >975 |
| **11** | >16 | 0,015 | >1040 | >12 | 0,002 | >5850 |
| **12** | 14 | 0,058 | 241 | 7 | 0,058 | 121 |
| **13** | <8 | 0,04 | <200 | <16 | 0,03 | <533 |
| **131** | >28 | 0,02 | >1555 | | | |
| **132** | 47 | 0,006 | 7833 | 2,3 | 0,003 | 767 |
| **134** | 94 | 0,009 | 10444 | 8 | 0,0047 | 1617 |
| **135** | 47 | 0,0052 | 9039 | 3 | 0,011 | 273 |

### In vivo-Wirkung:

### MCMV-Letalitätstest:

### Tiere:

2-3 Wochen alte weibliche immunkompetente Mäuse (12-14 g), Stamm Balb/C AnN oder CD1 wurden von kommerziellen Züchtern (Bomholtgaard, Iffa, Credo) bezogen. Die Tiere wurden nicht steril gehalten.

### Virusanzucht:

Murines Cytomegalievirus (MCMV), Stamm Smith, wurde *in vivo* wiederholt in weiblichen CD1-Mäusen passagiert. 21 Tage nach intraperitonealer Infektion (2 x 10⁴ Plaque Forming Units / 0,2 ml/Maus) wurden die Speicheldrüsen entnommen, im dreifachen Volumen Minimal Essential Medium (MEM) + 10 % foetalem Kälberserum (FKS) aufgenommen und mit Hilfe eines Ultrathurax homogenisiert. Es wurde 10 % DMSO v/v zugegeben, 1 ml-Aliquots hergestellt und die Virussuspension bei -140°C aufbewahrt. Nach serieller Verdünnung des Speicheldrüsenisolats in Zehnerschritten erfolgte die Titerbestimmung in Zellkultur auf NIH 3T3-Zellen nach Färbung mit Giemsalösung, sowie die Bestimmung der letalen Dosis *in vivo* in 2-3 Wochen alten Balb/C Mäusen.

### Virusinfektion der Versuchstiere, Behandlung und Auswertung:

2-3 Wochen alte weibliche immunkompetente Balb/C Mäuse (12-14 g) wurden mit 3 x 10⁵ PFU / 0.2 ml/Maus intraperitoneal infiziert. 6 Stunden nach der Infektion beginnend wurden die Mäuse über einen Zeitraum von 5 Tagen zweimal täglich (8.00 und 16.00 Uhr) peroral mit Substanz behandelt. Die Dosis betrug 3, 10, 30 oder 90 mg/kg Körpergewicht, das Applikationsvolumen 10 ml/kg Körpergewicht. Die Formulierung der Substanzen erfolgte in Form einer 0,5 %-igen Tylosesuspension mit 2 % DMSO. Im Zeitraum von 4-8 Tagen nach Infektion sterben die placebobehandelten Kontrolltiere. Die Auswertung erfolgt durch die Ermittlung des Prozentsatzes überlebender Tiere nach Substanzbehandlung im Vergleich zur placebobehandelten Kontrollgruppe.

### HCMV Xenograft-Gelfoam®-Modell:

### Tiere:

3-4 Wochen alte weibliche immundefiziente Mäuse (16-18 g), Fox Chase SCID oder Fox Chase SCID-NOD wurden von kommerziellen Züchtern (Bomholtgaard, Jackson) bezogen. Die Tiere wurden unter sterilen Bedingungen (einschließlich Streu und Futter) in Isolatoren gehalten.

### Virusanzucht:

Humanes Cytomegalievirus (HCMV), Stamm DavisSmith, wurde *in vitro* auf humanen embryonalen Vorhautfibroblasten (NHDF-Zellen) angezüchtet. Nach Infektion der NHDF-Zellen mit einer Multiplizität der Infektion (M.O.I) von 0.01 wurden die virusinfizierten Zellen 5-7 Tage später geerntet und in Gegenwart von Minimal Essential Medium (MEM), 10 % foetalem Kälberserum (FKS) mit 10 % DMSO bei -140°C aufbewahrt. Nach serieller Verdünnung der virusinfizierten Zellen in Zehnerschritten erfolgte die Titerbestimmung auf 24-Well-Platten konfluenter NHDF-Zellen nach Vitalfärbung mit Neutralrot.

### Vorbereitung der Schwämme, Transplantation, Behandlung und Auswertung:

1x1x1 cm große Kollagenschwämme (Gelfoam®; Fa. Peasel & Lorey, Best.-Nr. 407534; K.T. Chong et al., Abstracts of 39^{th} Interscience Conference on Antimicrobial Agents and Chemotherapy, 1999, S. 439) werden zunächst mit Phosphatgepufferter Saline (PBS) benetzt, die eingeschlossenen Luftblasen durch Entgasen entfernt und dann in MEM + 10 % FKS aufbewahrt. 1 x 10⁶ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. 12-13 Stunden später werden die infizierten Schwämme mit 25 µl PBS / 0,1 % BSA /1 mM DTT mit 5 ng/µl basic Fibroblast Growth Factor (bFGF) inkubiert. Zur Transplantation werden die immundefizienten Mäuse mit Avertin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation wurden die Mäuse über einen Zeitraum von 8 Tagen zweimal täglich (8.00 und 16.00 Uhr) peroral mit Substanz behandelt. Die Dosis betrug 10 oder 30 mg/kg Körpergewicht, das Applikationsvolumen 10 ml/kg Körpergewicht. Die Formulierung der Substanzen erfolgte in Form einer 0,5 %-igen Tylosesuspension mit 2 % DMSO. 10 Tage nach Transplantation und 16 Stunden nach der letzten Substanzapplikation wurden die Tiere schmerzlos getötet und der Schwamm entnommen. Die virusinfizierten Zellen wurden durch Kollagenaseverdau (330 U / 1,5 ml) aus dem Schwamm freigesetzt und in Gegenwart von MEM, 10 % foetalem Kälberserum, 10 % DMSO bei -140°C aufbewahrt: Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wurde die Anzahl infektiöser Viruspartikel nach Substanzbehandlung im Vergleich zur placebobehandelten Kontrollgruppe.

Der im folgenden beschriebene Test dient der Untersuchung der erfindungsgemäßen Substanzen im Hinblick auf ihr Nebenwirkungspotential bezüglich einer Induktion von Cytochrom P450-Enzymen.

### Untersuchung der Induktion von Cytochrom P450-Enzymen in humanen Leberzellkulturen:

Primäre humane Hepatozyten wurden mit einer Zelldichte von 2,5 x 10⁵ Zellen zwischen zwei Schichten von Collagen in 24 well-Mikrotiterplatten bei 37°C bei 5 % CO₂ 8 Tage kultiviert. Das Zellkulturmedium wurde täglich gewechselt.

Nach 48 Std. in Kultur wurden die Hepatozyten für 5 Tage in Doppelbestimmung mit unterschiedlichen Konzentrationen der Testsubstanzen im Vergleich mit den Induktoren Rifampicin (50 µM) und Phenobarbital (2 mM) behandelt. Die Endkonzentrationen der Testsubstanzen lagen bei 0,1 - 10 µg/ml.

Von den Zellkulturen wurde der induktive Effekt der Testsubstanzen auf die Cytochrom (CYP) P450-Enzyme 1A2, 2B6, 2C19 und 3A4 durch Zugabe der Substrate 7-Ethoxyresorufin (CYP1A2), [¹⁴C]S-Mephenytoin (CYP2B6 und 2C19) und [¹⁴C]Testosteron (CYP3A4) am Tag 8 bestimmt. Von den so gemessenen Enzymaktivitäten CYP1A2, 2B6, 2C19 und 3A4 behandelter Zellen im Vergleich zu unbehandelten Zellen wurde das induktive Potential der Testsubstanzen ermittelt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- Aloc-Cl: Chlorameisensäureallylester
- DCM: Dichlormethan
- DIC: N,N'-Diisopropylcarbodiimid
- DIEA: Diisopropylethylamin
- DMF: Dimethylformamid
- eq.: Äquivalente
- ges.: gesättigt
- HOAc: Essigsäure
- HOBt: Hydroxybenzotriazol
- HONSu: N-Hydroxysuccinimid
- i. Vak.: im Vakuum
- MTP: Microtiterplatte
- PS-: Polystyrol-Harz-
- PyBOP: Benzotriazolyl-N-Oxi-tris(dimethylamino)phosphonium-Hexafluorophosphat
- Rt: Retentionszeit
- RT: Raumtemperatur
- TBABH: Tetrabutylammoniumborhydrid
- TFA: Trifluoressigsäure
- THF: Tetrahydrofuran
- TMOF: Trimethylorthoformiat

### Allgemeine Arbeitsvorschrift für die Umsetzung von Verbindungen der Formel [A-1] mit Verbindungen der Formel [A-2] (AAV 1):

1,0 eq [A-1] werden in Dioxan gelöst (0,2 M Lösung), mit 2,5 eq. Pyridin versetzt, die Lösung auf 5°C abgekühlt und danach 1,1 eq. [A-2], worin Q vorzugsweise für Chlor steht, als 1,0 M Lösung zugetropft. Der Ansatz wird 30 min. bei 5°C weitergerührt, anschließend die Kühlung entfernt und 16 h bei Raumtemperatur nachgerührt. Der Ansatz wird auf H₂O gegeben, das ausgefallene Produkt abgesaugt, mit H₂O gewaschen und i. Hochvak. getrocknet.

### Allgemeine Arbeitsvorschrift für die Hydrierung von Verbindungen der Formel [A-3] (AAV 2):

0,14 mol der Verbindungen [A-3] werden in 500 ml DMF oder Ethanol gelöst und unter Argon mit einer Suspension von 6,0 g 10 %-igem Pd-C versetzt. Anschließend wird bei 3 bar Wasserstoff-Druck hydriert. Sobald der Umsatz vollständig ist (DC- oder HPLC-Kontrolle), wird der Pd-C-Katalysator abfiltriert und das Lösungsmittel im Vakuum entfernt. Die Rohprodukte der allgemeinen Formel [B-1] werden ohne weitere Reinigung weiter umgesetzt.

### Allgemeine Arbeitsvorschrift für die Sulfonylierung der Verbindungen der allgemeinen Formel [B-1] (AAV 3):

Unter Argon werden 1,0 eq. der Verbindungen [B-1] in Dioxan (0,2 M Lösung) gelöst und mit 2,5 eq. Pyridin versetzt. Nachdem 30 min. bei Raumtemperatur gerührt wurde, werden 1,1 eq. der Verbindungen der allgemeinen Formel [C-1], worin Z vorzugsweise für Chlor steht, gelöst in Dioxan (1,0 M Lösung) zugegeben und das Gemisch 16 h bei Raumtemperatur gerührt. Anschließend wird die Lösung auf H₂O gegeben und dreimal mit DCM extrahiert. Die organische Phase wird mit ges. NaHCO₃-Lösung gewaschen, über Na₂SO₄ getrocknet, filtriert und das Lösungsmittel im Vakuum entfernt. Der Rückstand [C-2] wird i. Hochvak. getrocknet und anschließend ohne weitere Reinigung weiter umgesetzt.

### Allgemeine Arbeitsvorschrift für die Synthese von Verbindungen der allgemeinen Formel [D-1] aus Verbindungen der allgemeinen Formel [C-2] (AAV 4):

Die Verbindungen der Formel [C-2] (1,0 eq.) werden in Ethanol gelöst (0,1 M Lösung), die Lösung mit Hydroxylamin-Hydrochlorid (1,5 eq.) sowie Triethylamin (1,6 eq.) versetzt, anschließend 4 h unter Rückfluss erhitzt sowie 16 h bei Raumtemperatur nachgerührt. Das Lösungsmittel wird im Vakuum entfernt, der Rückstand in Ethylacetat aufgenommen und 3 x mit Wasser extrahiert, die organische Phase über MgSO₄ getrocknet, filtriert und im Vakuum vom Lösungsmittel befreit. Der Rückstand [D-1] wird i. Hochvak. getrocknet.

### Allgemeine Arbeitsvorschrift für die Umsetzung der Verbindungen mit der allgemeinen Formel [D-1] mit Verbindungen [E-1] (AAV 5):

1,0 eq. der Verbindungen der allgemeinen Formel [D-1], 1,05 eq. Carbonsäure [E-1] und 1,1 eq. PyBOP werden in THF vorgelegt (0,1 M Lösung), die Suspension mit 1,1 eq. *N*,*N*-Diisopropylethylamin versetzt und die resultierende Lösung 16 h bei Raumtemperatur gerührt. Anschließend wird der Ansatz mit 10 ml DCM verdünnt und je einmal mit 1 N HCl, ges. NaHCO₃-Lösung sowie ges. NaCl-Lösung extrahiert. Die organische Phase wird über Na₂SO₄ getrocknet, filtriert und das Lösungsmittel im Vakuum entfernt. Das Rohprodukt wird direkt weiter umgesetzt.

### Allgemeine Arbeitsvorschrift für die Synthese eines 1,2,4-Oxadiazols aus dem gemäß AAV 5 erhaltenen Rohprodukt (AAV 6):

1,0 mmol von gemäß AAV 5 erhaltenem Rohprodukt werden in 10 ml DMF aufgenommen und die Lösung auf 110°C erhitzt. Sobald der Umsatz vollständig ist (DC- oder HPLC-Kontrolle, ca. 2-16 h), wird der Ansatz mit DCM verdünnt und zweimal mit H₂O extrahiert. Die vereinigten wässrigen Phasen werden zweimal mit DCM extrahiert, die organischen Phasen vereinigt und über Na₂SO₄ getrocknet, filtriert und das Lösungsmittel im Vakuum entfernt. Die so erhaltenen Verbindungen der allgemeinen Formel (I) werden durch Chromatographie an Kieselgel (Cyclohexan/Ethylacetat) oder durch präparative HPLC gereinigt.

### Allgemeine Arbeitsvorschriften für die Synthesen unter Verwendung von polymeren Trägern:

### Allgemeine Arbeitsvorschrift für die Synthese der 1,3,4-Oxadiazole gemäß Schema 4:

Die Reaktionen gemäß Schema 4 wurden an einem polymeren Träger mit dem IRORI-System nach der dem Festphasenchemiker geläufigen "Split & Mix"-Methode mit 4 Carbonsäurechloriden, 24 Carbonsäuren sowie beiden meta- bzw. para-Isomeren des Phenylendiamins bzw. Sulfonsäurechlorids durchgeführt. Dabei wurden die ersten beiden Stufen in einem Kolben durchgeführt, die übrigen Stufen in den IRORI-Mini-Kans (100 mg Harz pro Kan).

### Synthese der Ausgangsharze (I) und (II) für die Synthesen am polymeren Träger entsprechend Schema 4:

### Reduktive Aminierung von Formyl-Harz (Fa. Nova Biochem, 0, 78 mmol/g):

In einem Kolben wird das Formyl-Harz (1,0 eq.) in TMOF/DMF (100 ml pro 12,5 g Harz) suspendiert und mit dem Diamin (6,0 eq.) versetzt. Die Suspension wird 16 h bei 40°C geschüttelt und anschließend mit einer frisch angesetzten Lösung von TBABH (4,0 eq.) und HOAc (16,0 eq.) in DMF versetzt. Nach 8 h bei RT wird das Lösungsmittel abfiltriert und das Harz erneut mit der Reduktionslösung versetzt. Nach weiteren 16 h bei RT wird das Lösungsmittel abgesaugt und das Harz (I) jeweils 2 x mit je 200 ml 50 %-iger HOAc, DMF, THF sowie DCM gewaschen und i. Hochvak. getrocknet.

### Sulfonylierung von polymergebundenem Phenylendiamin:

Das Harz (I) (1,0 eq.) wird in THF aufgenommen und mit dem Sulfonsäurechlorid (1,5 eq.) versetzt. Die Suspension wird 16 h bei RT geschüttelt und das Lösungsmittel abgesaugt. Anschließend wird das Harz (II) je 2 x mit je 100 ml 50 %-iger HOAc, DMF, THF sowie DCM gewaschen und i. Hochvak. getrocknet.

### Harzvorbereitung für das IRORI-System:

Die Harze vom Typ II werden als Suspension (pro 3,0g Harz: 30 ml DMF/DCM 2:1 v/v) in je 96 Mini-Kans verteilt (1 ml Suspension pro Kan), jeweils dreimal mit DCM gewaschen und die Kans i. Vak. getrocknet.

### Reaktionssequenz (IRORI):

### Acylierung mit Säurechloriden:

Die Kans werden sortiert, in THF aufgenommen und mit 5,0 eq. DIEA sowie 5,0 eq. Säurechlorid versetzt, kurz evakuiert, und 3 h bei RT geschüttelt. Danach werden die Reaktionslösungen abgetrennt, die Kans vereinigt und gewaschen (je 2 x 50 %-ige HOAc, DMF, THF, DCM).

### Hydrazid-Synthese:

Die vereinigten Kans werden in einem Gemisch 2 N NaOH / MeOH / THF (5:7:15 v/v) aufgenommen, kurz evakuiert, und 5 h bei 50°C gerührt. Anschließend werden die Kans gewaschen (je 2 x 50 %-ige HOAc, DMF, THF, DCM) und i. Vak. getrocknet. Danach werden die Kans mit THF aufgenommen, mit 5 eq. DIC sowie 10 eq. HONSu versetzt und 3 h bei RT geschüttelt. Es wird abfiltriert, 2 x mit THF gewaschen und anschließend erneut mit THF aufgenommen und mit 3 eq. Hydrazinhydrat versetzt. Nach weiteren 3 h bei RT wird abgesaugt und die Kans mit je 2 x 50 %-ige HOAc, DMF, THF, DCM, gewaschen.

### Acylierung mit Carbonsäuren / DIC / HOBt:

Die Carbonsäuren (3 eq.) werden in THF mit 3 eq. DIC, 6 eq. DIEA sowie 6 eq. HOBt versetzt. Nach 60 min Aktivierung bei RT wird die Lösung zu den zuvor sortierten Kans gegeben und 16 h bei RT geschüttelt. Anschließend werden die Kans vereinigt, gewaschen (je 2 x 50 %-ige HOAc, DMF, THF, DCM) und i. Vak. getrocknet.

### Cyclisierung zum 1,3,4-Oxadiazol:

Die vereinigten Kans werden in DMF aufgenommen, mit DIC (10 eq.) versetzt, kurz evakuiert und 48 h bei 110°C gerührt. Anschließend werden die Kans gewaschen (je 2 x 50 %-ige HOAc, DMF, THF, DCM) und i. Vak. getrocknet.

### Abspaltung vom polymeren Träger:

Nach dem Sortieren in IRORI-Abspaltblöcke werden die Kans aufgeschnitten, das Harz in FlexChem-Blöcke verteilt und die Produkte mit je 1,0 ml TFA/DCM (1:1 v/v) 45 min. bei RT in eine Deep-Well-MTP abgespalten. Das Harz wird mit 1 ml DCM nachgewaschen und das Lösungsmittel eingedampft.

### Allgemeine Arbeitsvorschrift für die Synthese der 1,3,4-Thiadiazole gemäß Schema 5:

Die Synthese wird in einem gemischten Ansatz aus Festphasensynthese und Synthese in Lösung durchgeführt.

### Synthese des monosulfonylierten Phenylendiamins:

Das Phenylendiamin (1,0 eq) wird in THF gelöst (0,4 M Lösung), mit 1,0 eq. Sulfonsäurechlorid versetzt und die Mischung 16 h bei RT gerührt. Anschließend wird mit DCM verdünnt, 2 x mit Wasser extrahiert, die wässrigen Phasen 1 x mit DCM re-extrahiert, die org. Phasen vereinigt, über Na₂SO₄ getrocknet und einrotiert. Das Rohprodukt wird ohne weitere Reinigung weiter umgesetzt.

### Anknüpfung an den polymeren Träger und Aufbau des Thiadiazols:

### Reduktive Aminierung von Formyl-Harz (Fa. Nova Biochem, 0, 78 mmol/g):

In einem Kolben wird das Formyl-Harz (1,0 eq.) in TMOF/DMF (100 ml pro 12,5 g Harz) suspendiert und mit dem sulfonylierten Phenylendiamin (6,0 eq.) versetzt. Die Suspension wird 16 h bei 40°C geschüttelt und anschließend mit einer frisch angesetzten Lösung von TBABH (4,0 eq.) und HOAc (16,0 eq.) in DMF versetzt. Nach 8 h bei RT wird das Lösungsmittel abfiltriert und das Harz erneut mit der Reduktionslösung versetzt. Nach weiteren 16 h bei RT wird das Lösungsmittel abgesaugt und das Harz jeweils 2 x mit je 200 ml 50 %-iger HOAc, DMF, THF sowie DCM gewaschen und i. Hochvak. getrocknet.

### Acylierung des Harzes:

In einer Spritze mit PE-Fritte (Fa. MultiSyntech) wird das Harz in THF suspendiert und mit 3,0 eq DIEA und 3,0 eq. Carbonsäurechlorid versetzt. Die Suspension wird 3 h bei RT geschüttelt, dann abgesaugt und das Harz jeweils 2 x mit 50%-iger HOAc, DMF, THF sowie DCM gewaschen.

### Hydrazid-Synthese:

In einer Spritze mit PE-Fritte (Fa. MultiSyntech) wird das Harz in einem Gemisch aus 2 N NaOH / MeOH / THF (5:7:15 v/v) aufgenommen, 5 h bei 50°C gerührt und anschließend gewaschen (je 2 x 50%-ige HOAc, DMF, THF, DCM). Danach wird das Harz mit THF aufgenommen, mit 5 eq. DIC sowie 10 eq. HONSu versetzt und 3 h bei RT geschüttelt. Es wird abfiltriert, 2 x mit THF gewaschen und anschließend erneut mit THF aufgenommen und mit 3 eq. Hydrazinhydrat versetzt. Nach weiteren 3 h bei RT wird abgesaugt und das Harz gewaschen (je 2 x 50%-ige HOAc, DMF, THF, DCM).

### Acylierung des Hydrazids mit Carbonsäuren / DIC / HOBt:

Die Carbonsäure (3 eq.) werden in THF mit 3 eq. DIC, 6 eq. DIEA sowie 6 eq. HOBt versetzt. Nach 60 min. Aktivierung bei RT wird die Lösung zu dem Harz gegeben (1 ml pro 100 mg Harz) und 16 h bei RT geschüttelt. Anschließend wird abgesaugt und das Harz gewaschen (je 2 x 50%-ige HOAc, DMF, THF, DCM). Das LC-MS der Probeabspaltung zeigt, dass die Doppelbindung der Allyloxycarbonyl-Gruppe bei dieser Reaktion hydriert wird.

### Thiadiazol-Synthese:

Das Harz wird in Dioxan (1 ml pro 100 mg Harz) vorgelegt, mit 5,0 eq. Lawesson's Reagenz versetzt und die Mischung 3 h bei 90°C gerührt. Anschließend wird abgesaugt und das Harz jeweils 2 x mit DMF, 50 %-iger HOAc, DMF, THF sowie DCM gewaschen.

### Abspaltung vom polymeren Träger, Abspaltung der Carbamatschutzgruppe und Synthese des Amids:

Das Harz wird mit TFA/DCM (1:1 v/v, 1 ml pro 100 mg Harz) behandelt, nach 45 min. abfiltriert und mit DCM (gleiches Volumen) nachgewaschen. TFA und DCM werden i. Vak. entfernt, der Rückstand in Ethanol / 2,5 N NaOH (1:1 v/v, 0,5 M Lösung) aufgenommen, 16 h bei 75°C gerührt, mit DCM verdünnt, 2 x mit Wasser extrahiert, die wässrige Phase mit 1 N HCl auf pH 7 gestellt, die wässrige Phase 3 x mit DCM extrahiert, alle org. Phasen vereinigt, 2 x mit Wasser gewaschen, die org. Phase über Na₂SO₄ getrocknet und einrotiert. Der Rückstand wird in THF aufgenommen, mit 1,05 eq. DIEA sowie 1,05 eq. Säurechlorid versetzt und die Mischung 16 h bei RT geschüttelt. Anschließend wird i. Vak. von flüchtigen Komponenten befreit und das Produkt mittels präparativer HPLC isoliert.

### Ausgangsverbindungen:

### Beispiel I

### 1-Methyl-N-(3-nitrophenyl)-cyclopropanamid

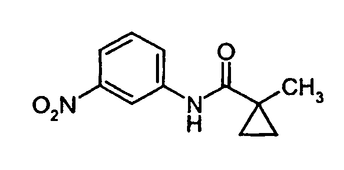

Diese Verbindung wird gemäß AAV 1 aus 80,0 g 3-Nitroanilin hergestellt. Ausbeute: 107 g (81 % d.Th.)

### Beispiel II

### 3-Fluor-2,2-dimethyl-N-(3-aminophenyl)-propanamid

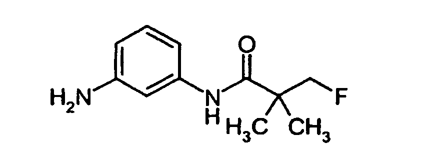

Diese Verbindung wird gemäß AAV 1 und AAV 2 aus 3-Nitroanilin ohne Reinigung der Zwischenstufe hergestellt.
Ausbeute: 85 % d.Th. (über 2 Stufen)

### Beispiel III

### 1-Methyl-N-(3-aminophenyl)-cyclopropanamid

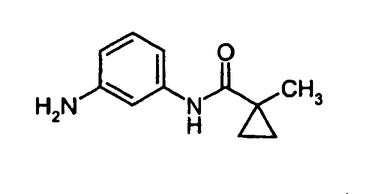

Diese Verbindung wird gemäß AAV 2 aus 107 g der Verbindung aus Beispiel I hergestellt.
Ausbeute: 80 g (87 % d.Th.)

### Beispiel IV

### 3-Fluor-2,2-dimethyl-N-(3-{[(4-methylphenyl)sulfonyl]amino}phenyl)-propanamid

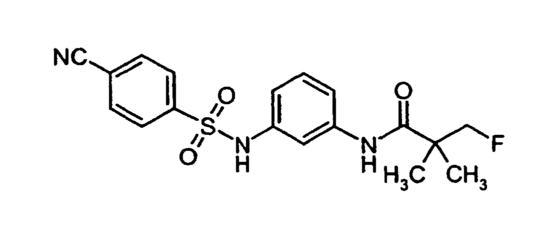

Diese Verbindung wird gemäß AAV 3 aus 18,68 g der Verbindung aus Beispiel II hergestellt.
Ausbeute: 19,96 g (78 % d.Th.)

### Beispiel V

### N-{3-[({4-[Amino(hydroxyimino)methyl]phenyl}sulfonyl)amino]phenyl}-3-fluor-2,2-dimethylpropanamid

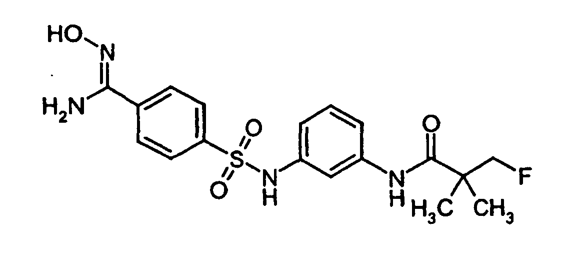

Diese Verbindung wird gemäß AAV 4 aus 10,0 g der Verbindung aus Beispiel IV hergestellt.
Ausbeute: 10,5 g (97 % d.Th.)

### Beispiel VI

### N-(3-{[(4-Cyanophenyl)sulfonyl]amino}phenyl)-1-methylcyclopropancarboxamid

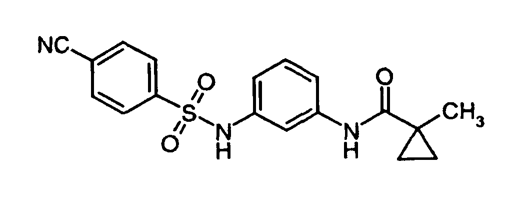

Diese Verbindung wird gemäß AAV 3 aus 90 g der Verbindung aus Beispiel III hergestellt.
Ausbeute: 150 g Rohprodukt (quant.)
HPLC: Rt = 2.87 min (HPLC-Methode/Instrument 9)

### Beispiel VII

### N- {3-[({4-[Amino(hydroxyimino)methyl]phenyl}sulfonyl)amino]phenyl}-1-methyl-cyclopropancarboxamid

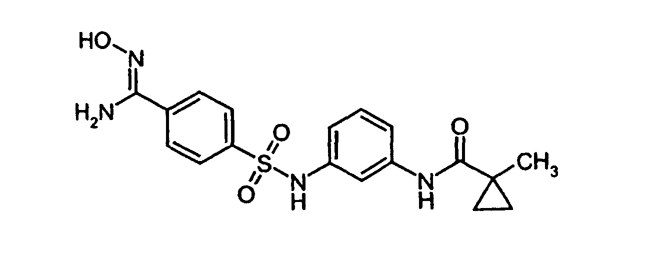

Diese Verbindung wird gemäß AAV 3 aus 168 g der Verbindung aus Beispiel VI (als Rohprodukt) hergestellt.
Ausbeute: 118 g (57 % d.Th.)
HPLC: Rt = 2.7 min (HPLC-Methode/Instnanent 5)
MW 388.45; m/z gef.: 389

### Beispiel VIII

### 2-Aminoacetyl-picolin

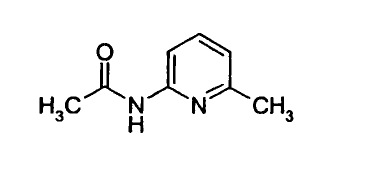

25,0 g (0,23 mol) 6-Aminopicolin werden in 250 ml Essigsäure gelöst und unter Rühren und Eiskühlung mit 47,2 g (0,46 mol) Essigsäureanhydrid versetzt. Man lässt zunächst bei Eiskühlung noch 30 min. rühren, dann wird das Eisbad entfernt und 16 h bei Raumtemperatur weitergerührt. Anschließend wird die klare Lösung i. Vak. eingeengt. Der ölige Rückstand wird im Eisbad kristallisiert und die Kristalle i. Vak. getrocknet.
Ausbeute: 28 g (80,6 % d.Th.)
¹H-NMR (200 MHz, DMSO-d₆): δ = 10.41 (s, 1H), 7.87 (d, 1H), 7.63 (t, 1H), 6.93 (d, 1H), 2.39 (s, 3H), 2.06 (s, 3H).

### Beispiel IX

### 2-Aminoacetyl-picolinsäure

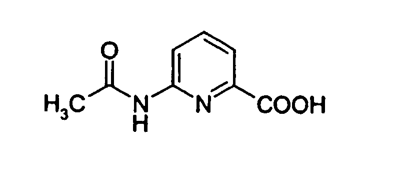

31,0 g (0,21 mol) 2-Aminoacetylpicolin werden in 310 ml Wasser gelöst, auf 75°C erhitzt und portionsweise über 3 h mit 60,0 g (0,38 mol) Kaliumpermanganat versetzt, so dass die violette Farbe jeweils wieder verschwindet. Es wird 5 h bei 75°C nachgerührt und anschließend das noch heiße Reaktionsgemisch filtriert. Die wässrige Phase wird viermal mit Dichlormethan extrahiert und anschließend die wässrige Phase mit 1 N Salzsäure auf pH = 4 angesäuert. Der Niederschlag wird abfiltriert, mit 0,1 N Salzsäure gewaschen und i. Vak. getrocknet.
Ausbeute: 15,5 g (42 % d.Th.)
HPLC: Rt = 1.11 min (HPLC-Methode/Instrument 3)
MW 180.16; m/z gef.: 181
¹H-NMR (200 MHz, DMSO-d₆): δ = 13.23 (br s, 1H), 10.81 (s, 1H), 8.28 (d, 1H), 7.94 (t, 1H), 7.73 (dd, 1H), 2.12 (s, 3H).

### Ausführungsbeispiele:

Die im folgenden aufgeführten Ausführungsbeispiele zu 3-verknüpften 1,2,4-Oxadiazolen wurden aus den Verbindungen vom Typ Beispiel V gemäß AAV 5 und AAV 6 hergestellt.

### Beispiel 1

### 3-Fluor-2,2-dimethyl-N-{3-[({4-[5-(2-pyridinyl)-1,2,4-oxadiazol-3-yl]phenyl}-sulfonyl)amino]phenyl}propanamid

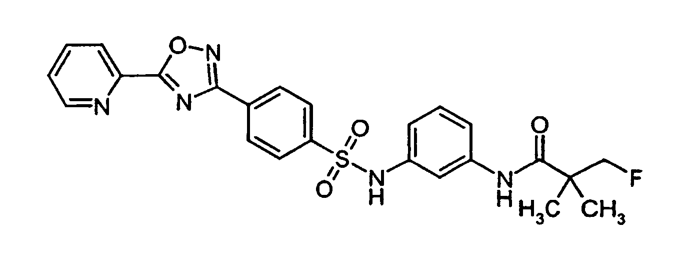

In 70 ml THF werden 5,93 g (45,92 mmol) *N,N*-Diisopropylethylamin, 5,65 g (45,92 mmol) Picolinsäure und 23,89 g (45,92 mmol) PyBOP vorgelegt, 30 min. bei Raumtemperatur gerührt, dann mit 17,05 g (41,74 mmol) des Amidoxims aus Beispiel V versetzt und die Lösung 16 h bei Raumtemperatur gerührt. Das Reaktionsgemisch wird i. Vak. eingeengt, der Rückstand in 50 ml DMF aufgenommen und die Lösung für 4 h bei 110°C gerührt. Anschließend wird der Ansatz mit 300 ml DCM verdünnt und die organische Phase dreimal mit je 200 ml 2 N H₂SO₄ sowie einmal mit ges. NaHCO₃-Lösung extrahiert. Die organische Phase wird über Na₂SO₄ getrocknet, filtriert und das Lösungsmittel i. Vak. entfernt (43,5 g Rohprodukt). Das Produkt wird durch Chromatographie an Kieselgel mit Cyclohexan/Ethylacetat (6:4 v/v) gereinigt und nach der Reinigung mit Cyclohexan verrührt, der Feststoff abgesaugt und i. Vak. getrocknet.
- Ausbeute :: 12,59 g (61 % d. Th.) eines weißen Feststoffs
- Schmp.:: 178,9°C
- MW 495,53;: m/z gef.: 496
- HPLC-Rt:: 4,38 min. (HPLC-Methode/Instrument: 3)
¹H-NMR (300 MHz, DMSO): δ = 1,20 (s, 3 H), 1,21 (s, 3 H), 4,48 (d, 2 H), 6,81 (d, 1 H), 7,14 (t, 1 H), 7,31 (d, 1 H), 7,58 (s, 1 H), 7,71-7,78 (m, 1 H), 7,99 (d, 2 H), 8,09-8,18 (m, 1 H), 8,27 (d, 2 H), 8,34 (d, 1 H), 8,86 (d, 1 H), 9,35 (s, 1 H), 10,43 (s, 1 H).

### Beispiel 2

### N-(3-{[(4-{5-[3-(Dimethylamino)phenyl]-1,2,4-oxadiazol-3-yl}phenyl)sulfonyl]-amino}phenyl)-1-methylcyclopropancarboxamid

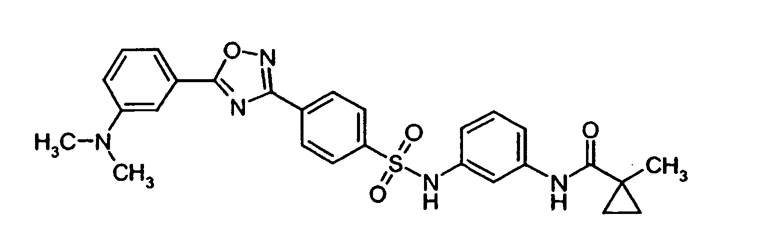

- MW 517,61;: m/z gef.: 518
- HPLC-Rt:: 3,25 min. (HPLC-Methode/Instrument: 3)
¹H-NMR (300 MHz, DMSO): δ = 0,54-0,64 (m, 2 H), 1,00-1,09 (m, 2 H), 1,36 (s, 3 H), 3,00 (s, 6 H), 6,79 (d, 1 H), 7,02-7,50 (m, 6 H), 7,57 (t, 1 H), 7,97 (d, 2 H), 8,24 (d, 2 H), 9,15 (s, 1 H), 10,34 (s, 1 H).

### Beispiel 3

### 1-Methyl-N-{3-[({4-[5-(2-pyridinyl)-1,2,4-oxadiazol-3-yl]phenyl}sulfonyl)-amino]-phenyl}cyclopropancarboxamid

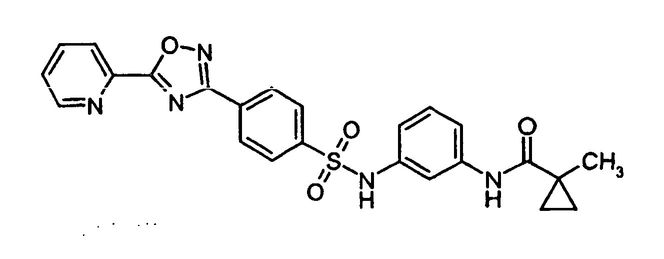

20,0 g (51,59 mmol) des entsprechenden Amidoxims, 6,66 g (54,06 mmol) Picolinsäure und 29,47 g (56,66 mmol) PyBOP werden in 60 ml THF vorgelegt, die Suspension bei Raumtemperatur mit 7,32 g (56,66 mmol) *N,N*-Diisopropylethylamin versetzt und die resultierende klare Lösung 16 h bei Raumtemperatur gerührt. Anschließend wird der Ansatz mit 250 ml DCM verdünnt und je einmal mit je 250 ml 1 N HCl, ges. NaHCO₃-Lösung sowie ges. NaCl-Lösung extrahiert. Die organische Phase wird über Na₂SO₄ getrocknet, filtriert und das Lösungsmittel i. Vak. entfernt. Das Rohprodukt (25,41 g) wird in 250 ml DMF aufgenommen und die Lösung für 2,5 h bei 110°C gerührt. Anschließend wird der Ansatz mit 250 ml DCM verdünnt und die organische Phase zweimal mit je 250 ml H₂O extrahiert. Die vereinigten wässrigen Phasen werden zweimal mit je 250 ml DCM extrahiert, die organischen Phasen vereinigt und über Na₂SO₄ getrocknet, filtriert und das Lösungsmittel i. Vak. entfernt (43,5 g Rohprodukt). Das Produkt wird durch Chromatographie an Kieselgel 60 mit Cyclohexan/Ethylacetat 1:1 v/v gereinigt.
- Ausbeute :: 18,35 g (75 % d. Th.) eines weißen Feststoffs
- Schmp.:: 202°C
- MW 475,53;: m/z ger.: 476
- HPLC-Rt:: 4,0 min. (HPLC-Methode/Instrument: 6)
¹H-NMR (200 MHz, DMSO): δ = 0,55-0,64 (m, 2 H), 1,00-1,10 (m, 2 H), 1,36 (s, 3 H), 6,78 (d, 1 H), 7,11 (t, 1 H), 7,27 (d, 1 H), 7,57 (s, 1 H), 7,70-7,79 (m, 1 H), 8,08 (d, 2 H), 8,09-8,19 (m, 1 H), 8,26 (d, 2 H), 8,34 (d, 1 H), 8,87 (d, 1 H), 9,17 (s, 1 H), 10,38 (s, 1 H).

### Beispiel 4

### N-{3-[({4-[5-(2-Amino-1,3-thiazol-4-yl)-1,2,4-oxadiazol-3-yl]phenyl}sulfonyl)-amino]phenyl}-1-methylcyclopropancarboxamid

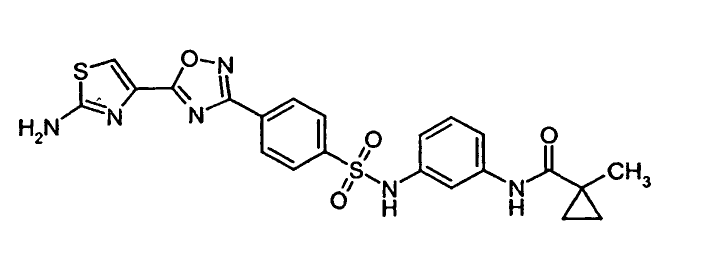

- MW 496,57;: m/z gef.: 497
- HPLC-Rt:: 2,508 min. (HPLC-Methode/Instrument: 8)
¹H-NMR (200 MHz, DMSO): δ = 0,54-0,64 (m, 2 H), 1,00-1,09 (m, 2 H), 1,36 (s, 3 H), 6,78 (d, 1 H), 7,12 (t, 1 H), 7,28 (d, 1 H), 7,52 (s, 1 H), 7,57 (s, 1 H), 7,82 (s, 1 H), 7,95 (d, 2 H), 8,19 (d, 2 H), 9,18 (s, 1 H), 10,38 (s, 1 H).

### Beispiel 5

### 1-Methyl-N-{3-[({4-[5-(6-methyl-2-pyridinyl)-1,2,4-oxadiazol-3-yl]phenyl}-sulfonyl)amino]phenyl}cyclopropancarboxamid

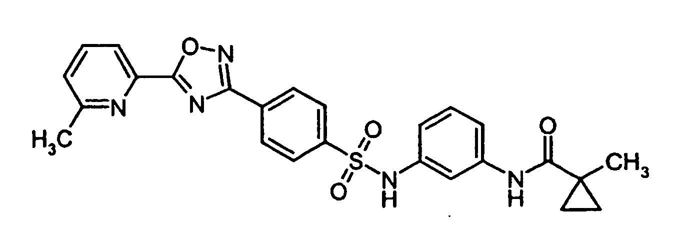

- MW 489,55;: m/z gef.: 490
- HPLC-Rt:: 4,76 min. (HPLC-Methode/Instrument: 6)
¹H-NMR (200 MHz, DMSO): δ = 0,54-0,63 (m, 2 H), 1,00-1,09 (m, 2 H), 1,36 (s, 3 H), 2,61 (s, 3 H), 6,77 (d, 1 H), 7,10 (t, 1 H), 7,26 (d, 1 H), 7,50-7,65 (m, 2 H), 7,92-8,06 (m, 3 H), 8,14 (d, 1 H), 8,25 (d, 2 H), 9,17 (s, 1 H), 10,39 (s, 1 H).

### Beispiel 6

### 1-Methyl-N-{3-[({4-[5-(3-methyl-1H-pyrazol-5-yl)-1,2,4-oxadiazol-3-yl]phenyl}-sulfonyl)amino]phenyl}cyclopropancarboxamid

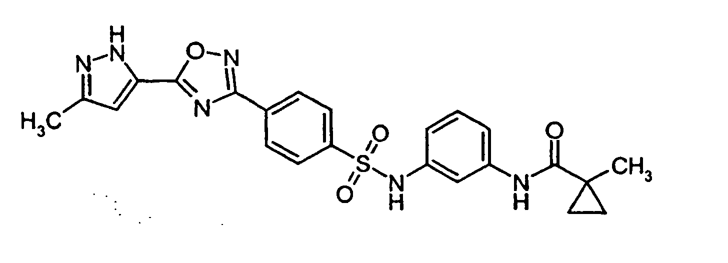

- MW 478,53;: m/z gef.: 479
- HPLC-Rt:: 3,77 min. (HPLC-Methode/Instrument: 6)
¹H-NMR (200 MHz, DMSO): δ = 0,54-0,65 (m, 2 H), 0,99-1,12 (m, 2 H), 1,36 (s, 3 H), 2,34 (s, 3 H), 6,77 (d, 1 H), 7,10 (t, 1 H), 7,25 (d, 1 H), 7,54 (s, 1 H), 7,95 (d, 2 H), 8,20 (d, 2 H), 9,16 (s, 1 H), 10,38 (s, 1 H), 13,58 (s, 1 H).

### Beispiel 7

### 1-Methyl-N-{3-[({4-[5-(1,3-thiazol-4-yl)-1,2,4-oxadiazol-3-yl]phenyl}sulfonyl)-amino]phenyl}cyclopropancarboxamid

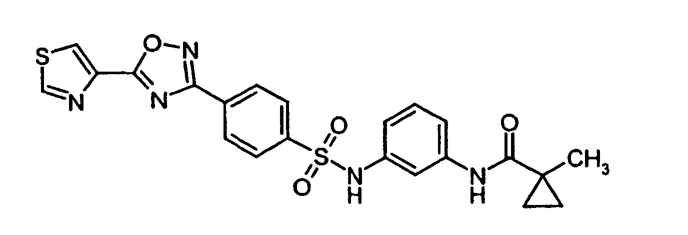

- MW 481,56;: m/z gef.: 482
- HPLC-Rt:: 2,689 min. (HPLC-Methode/Instrument: 8)
¹H-NMR (300 MHz, DMSO): δ = 0,55-0,63 (m, 2 H), 1,01-1,09 (m, 2 H), 1,36 (s, 3 H), 6,77 (d, 1 H), 7,12 (t, 1 H), 7,28 (d, 1 H), 7,58 (s, 1 H), 7,98 (d, 2 H), 8,24 (d, 2 H), 8,95 (d, 1 H), 9,19 (s, 1 H), 9,40 (d, 1 H), 10,39 (s, 1 H).

### Beispiel 8

### N- {3-[({4-[5-(1,5-Dimethyl-1H-pyrazol-3-yl)-1,2,4-oxadiazol-3-yl]phenyl}-sulfonyl)amino]phenyl}-1-methylcyclopropancarboxamid

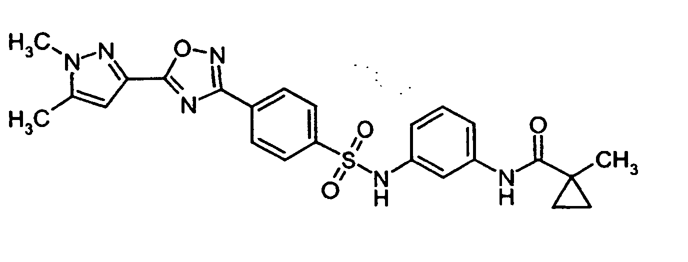

- MW 492,56;: m/z gef.: 493
- HPLC-Rt:: 2,788 min. (HPLC-Methode/Instrument: 8)
¹H-NMR (200 MHz, DMSO): δ = 0,53-0,64 (m, 2 H), 0,97-1,12 (m, 2 H), 1,36 (s, 3 H), 2,35 (s, 3 H), 3,89 (s, 3 H), 6,77 (d, 1 H), 6,85 (s, 1 H), 7,11 (t, 1 H), 7,27 (d, 1 H), 7,56 (s, 1 H), 7,95 (d, 2 H), 8,21 (d, 2 H), 9,18 (s, 1 H), 10,38 (s, 1 H).

### Beispiel 9

### 1-Methyl-N-{3-[({4-[5-(5-methyl-1H-pyrazol-3-yl)-1,2,4-oxadiazol-3-yl]phenyl}-sulfonyl)amino]phenyl}cyclopropancarboxamid

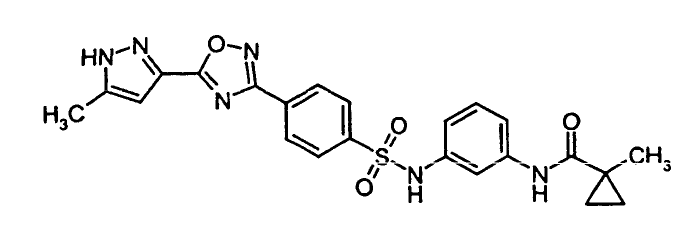

- MW 478,53;: m/z gef.: 479
- HPLC-Rt:: 2,614 min. (HPLC-Methode/Instrument: 8)
¹H-NMR (300 MHz, DMSO): δ = 0,57-0,63 (m, 2 H), 1,01-1,08 (m, 2 H), 1,36 (s, 3 H), 2,34 (s, 3 H), 6,75-6,80 (m, 2 H), 7,10 (t, 1 H), 7,26 (d, 1 H), 7,54 (s, 1 H), 7,96 (d, 2 H), 8,20 (d, 2 H), 9,16 (s, 1 H), 10,38 (s, 1 H), 13,58 (s, 1 H).

### Beispiel 10

### N-{3-[({4-[5-(1-Isochinolinyl)-1,2,4-oxadiazol-3-yl]phenyl}sulfonyl)amino]phenyl}-1-methylcyclopropancarboxamid

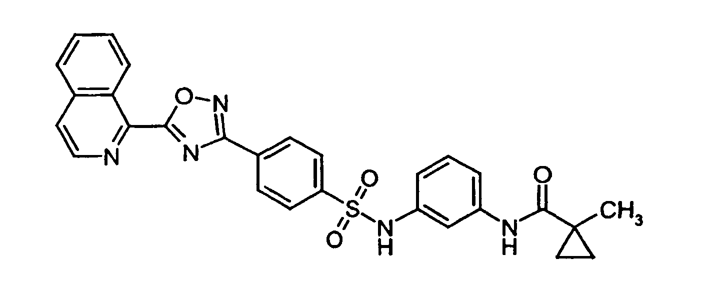

- MW 525,59;: m/z gef.: 526
- HPLC-Rt:: 4,34 min. (HPLC-Methode/Instrument: 5)
¹H-NMR (200 MHz, DMSO): δ = 0,54-0,67 (m, 2 H), 0,99-1,11 (m, 2 H), 1,36 (s, 3 H), 6,80 (d, 1 H), 7,12 (t, 1 H), 7,28 (d, 1 H), 7,58 (s, 1 H), 7,86-7,99 (m, 2 H), 8,01 (d, 2 H), 8,15-8,29 (m, 1 H), 8,26 (d, 1 H), 8,36 (d, 2 H), 8,82 (d, 1 H), 9,18 (s, 1 H), 9,26-9,36 (m, 1 H), 10,41 (s, 1 H).

### Beispiel 11

### 1-Methyl-N-{3-[({4-[5-(2-methyl-1,3-thiazol-4-yl)-1,2,4-oxadiazol-3-yl]phenyl}-sulfonyl)amino]phenyl}cyclopropancarboxamid

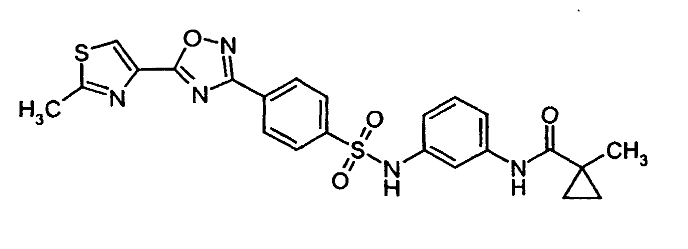

- MW 495,58;: m/z gef.: 496
- HPLC-Rt:: 2,813 min. (HPLC-Methode/Instrument: 8)
¹H-NMR (200 MHz, DMSO): δ = 0,55-0,63 (m, 2 H), 1,00-1,09 (m, 2 H), 1,36 (s, 3 H), 2,78 (s, 3 H), 6,78 (d, 1 H), 7,12 (t, 1 H), 7,28 (d, 1 H), 7,58 (s, 1 H), 7,97 (d, 2 H), 8,23 (d, 2 H), 8,73 (s, 1 H), 9,19 (s, 1 H), 13,39 (s, 1 H).

### Beispiel 12

### N-(3-{[(4-{5-[2-(Aminomethyl)-1,3-thiazol-4-yl]-1,2,4-oxadiazol-3-yl}phenyl)-sulfonyl]amino}phenyl)-1-methylcyclopropancarboxamid

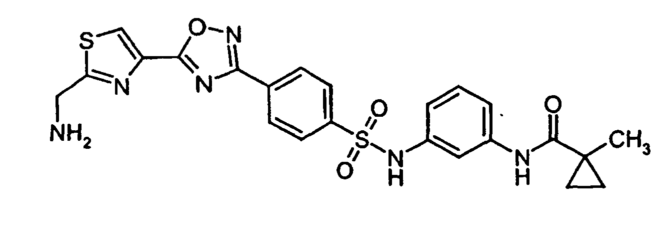

- MW 510,60;: m/z gef.: 511
- HPLC-Rt:: 1,71 min. (HPLC-Methode/Instrument: 8)
¹H-NMR (200 MHz, DMSO): δ = 0,54-0,64 (m, 2 H), 0,99-1,09 (m, 2 H), 1,36 (s, 3 H), 4,09 (s, 2 H), 6,77 (d, 1 H), 7,11 (t, 1 H), 7,27 (d, 1 H), 7,56 (s, 1 H), 7,96 (d, 2 H), 8,22 (d, 2 H), 8,75 (s, 1 H), 9,18 (s, 1 H).

### Beispiel 13

### 3-Fluor-2,2-dimethyl-N-[4-({[3-(5-phenyl-1,2,4-oxadiazol-3-yl)phenyl]sulfonyl}-amino)phenyl]propanamid

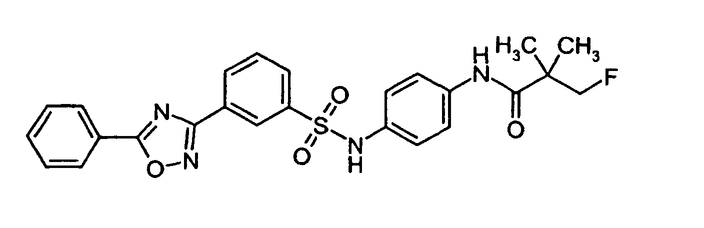

- MW 494,54;: m/z gef.: 495
- HPLC-Rt:: 4,8 min. (HPLC-Methode/Instrument: 3)
¹H-NMR (300 MHz, DMSO): δ = 1,17 (s, 6 H), 4,44 (d, 2 H), 7,04 (d, 2 H), 7,48 (d, 2 H), 7,63-7,81 (m, 4 H), 7,90 (d, 1 H), 8,22 (d, 2 H), 8,30 (d, 1 H), 8,45 (s, 1 H), 10,31 (s, 1 H).

Weitere gemäß den erfindungsgemäßen Verfahren hergestellte, über die Position 3 verknüpfte 1,2,4-Oxadiazol-Derivate sind in Tabelle 2 aufgeführt:

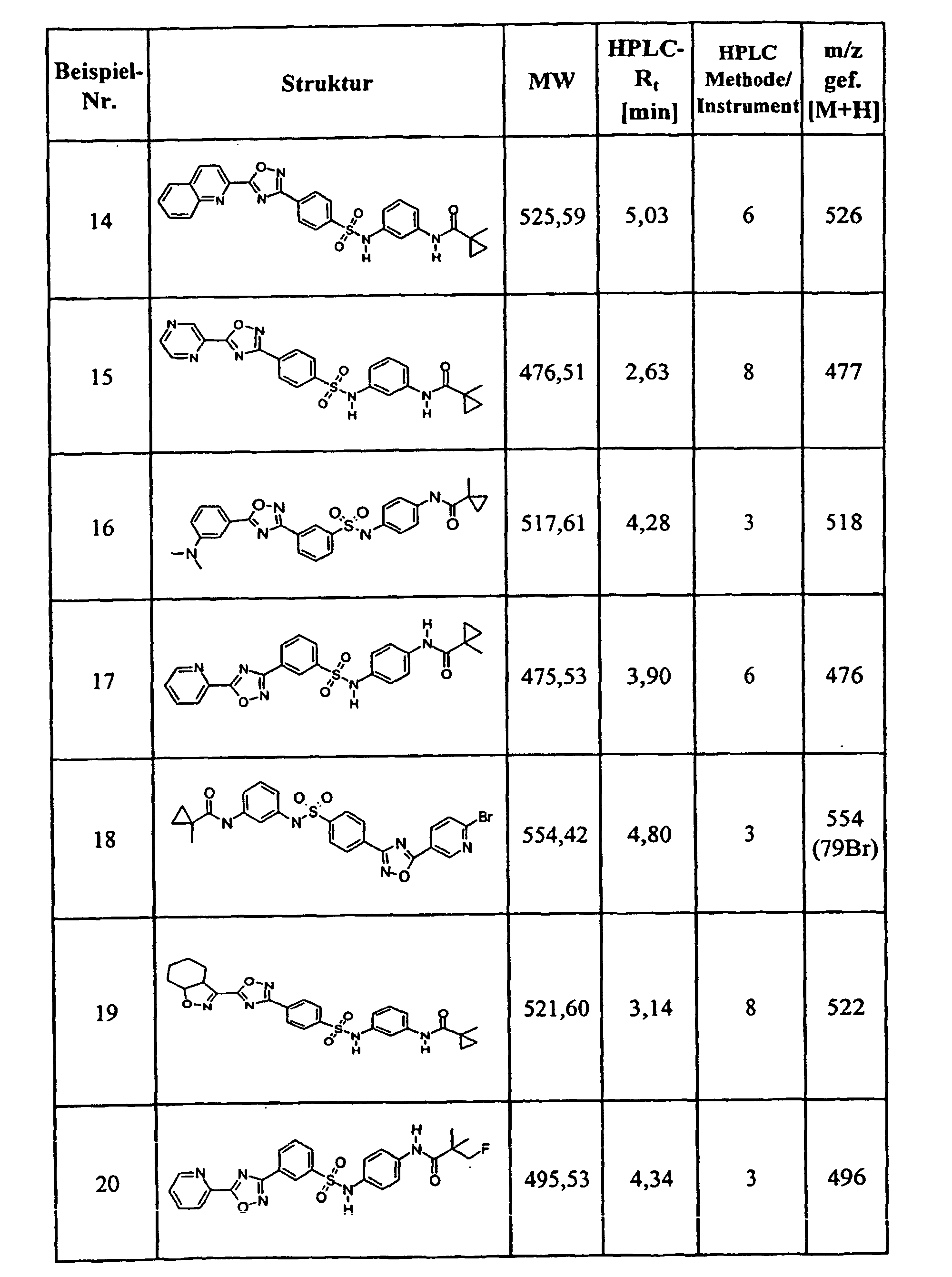

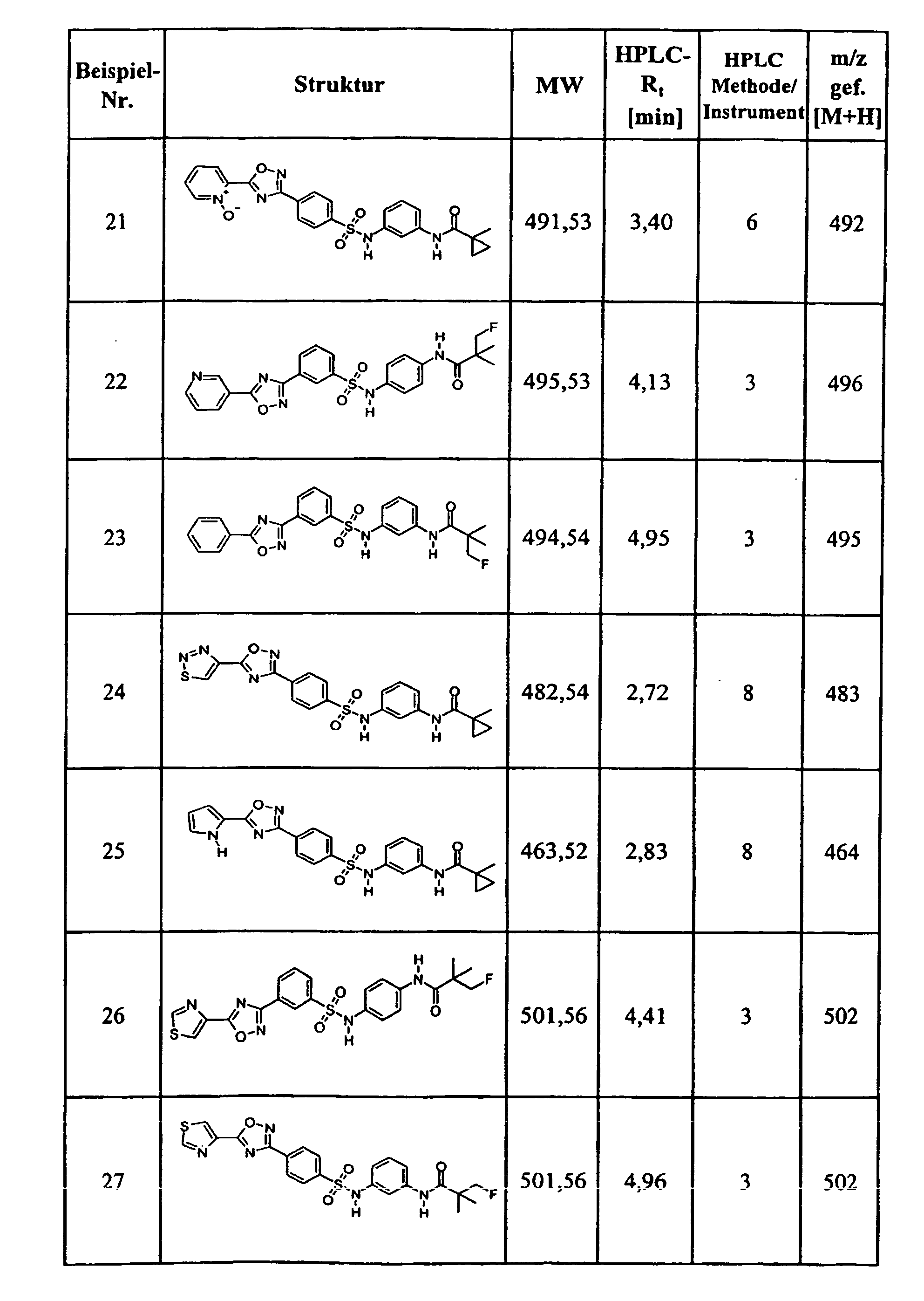

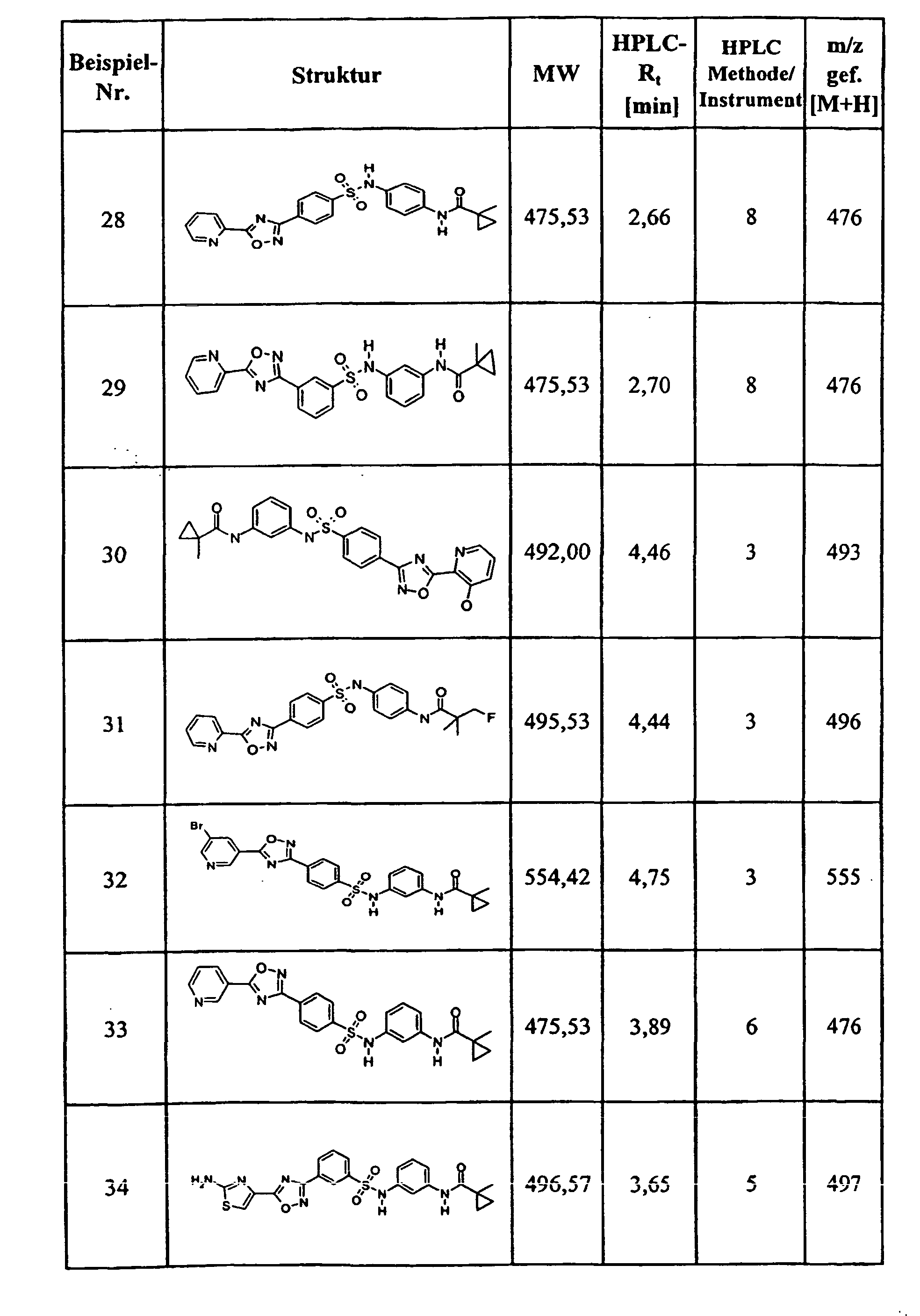

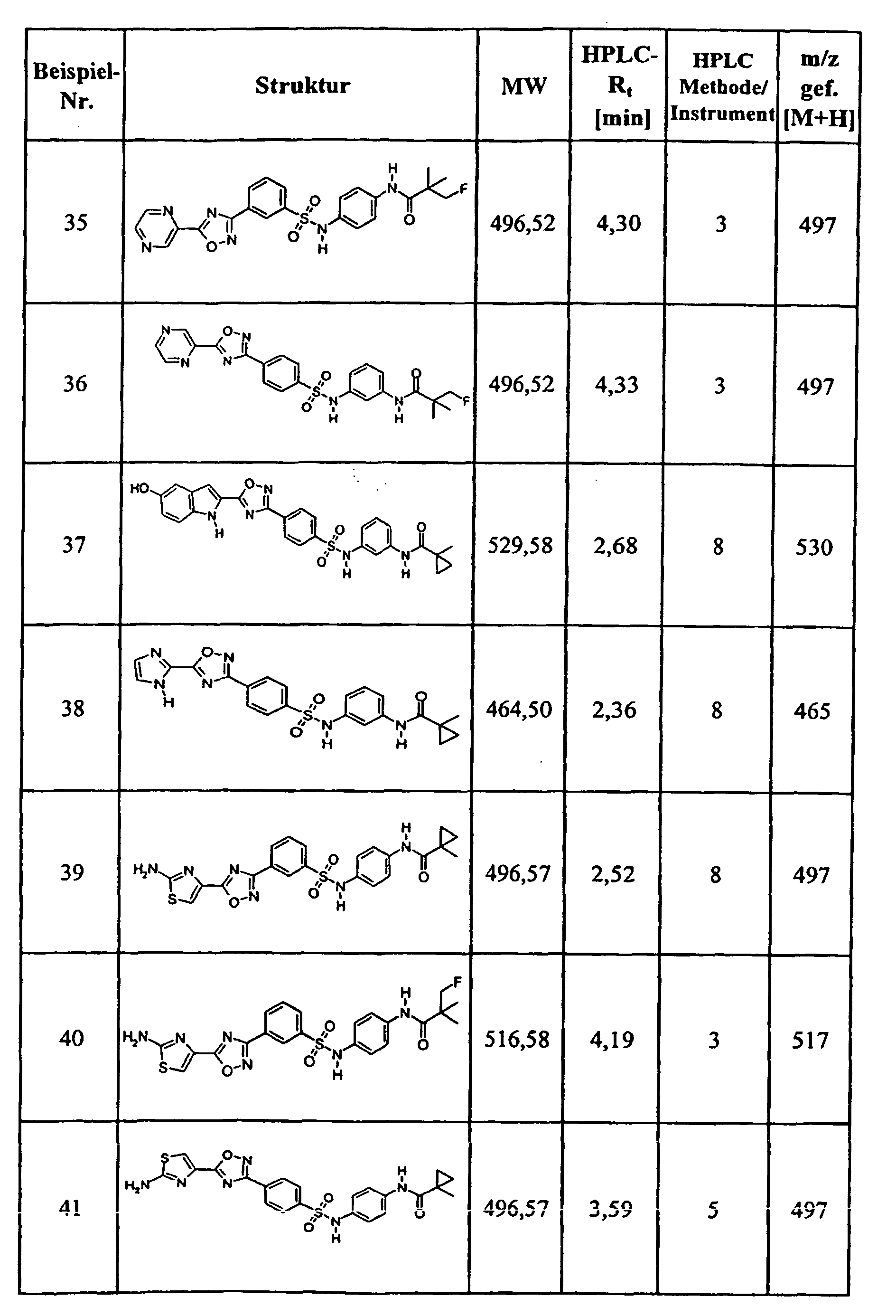

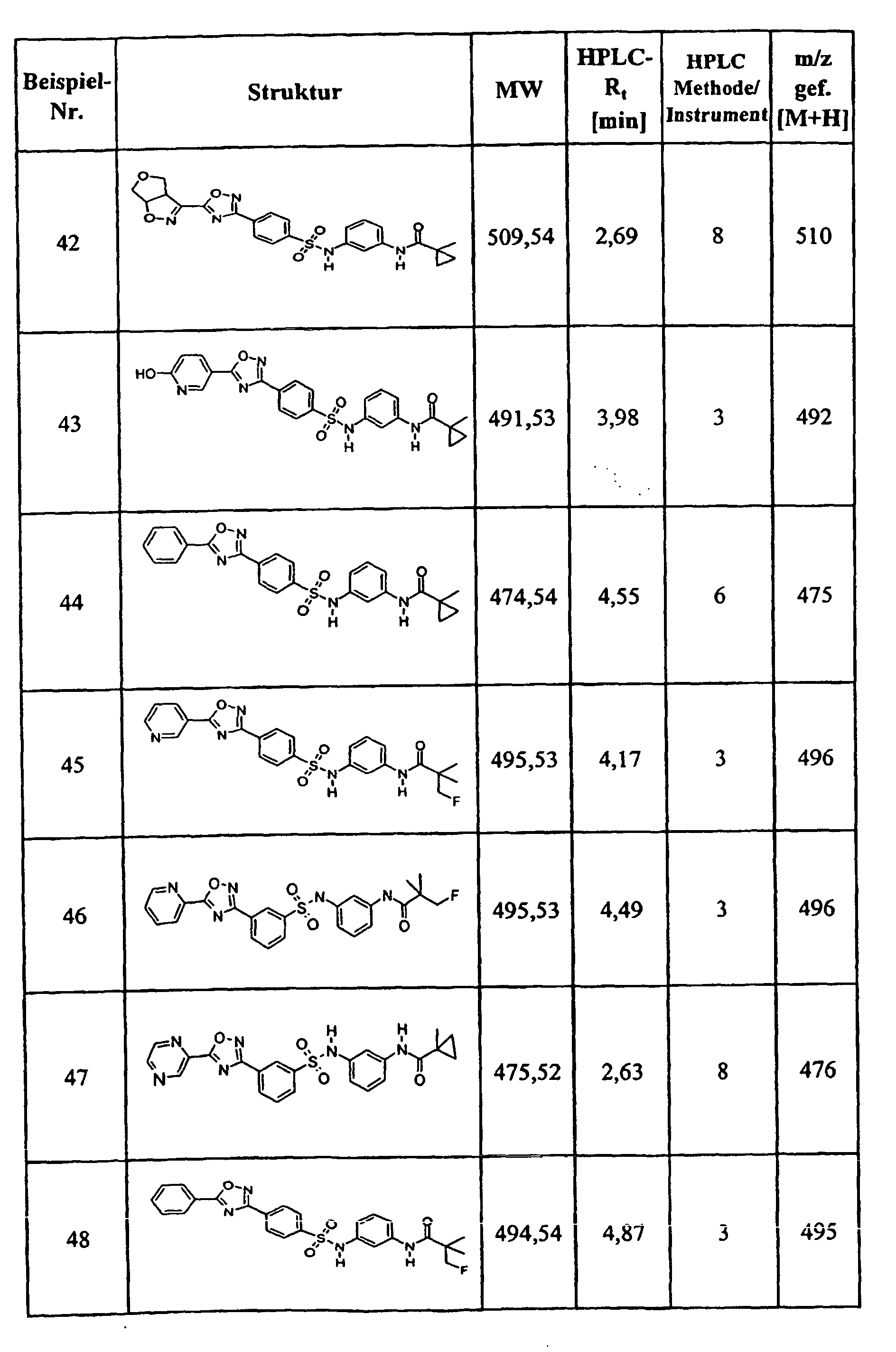

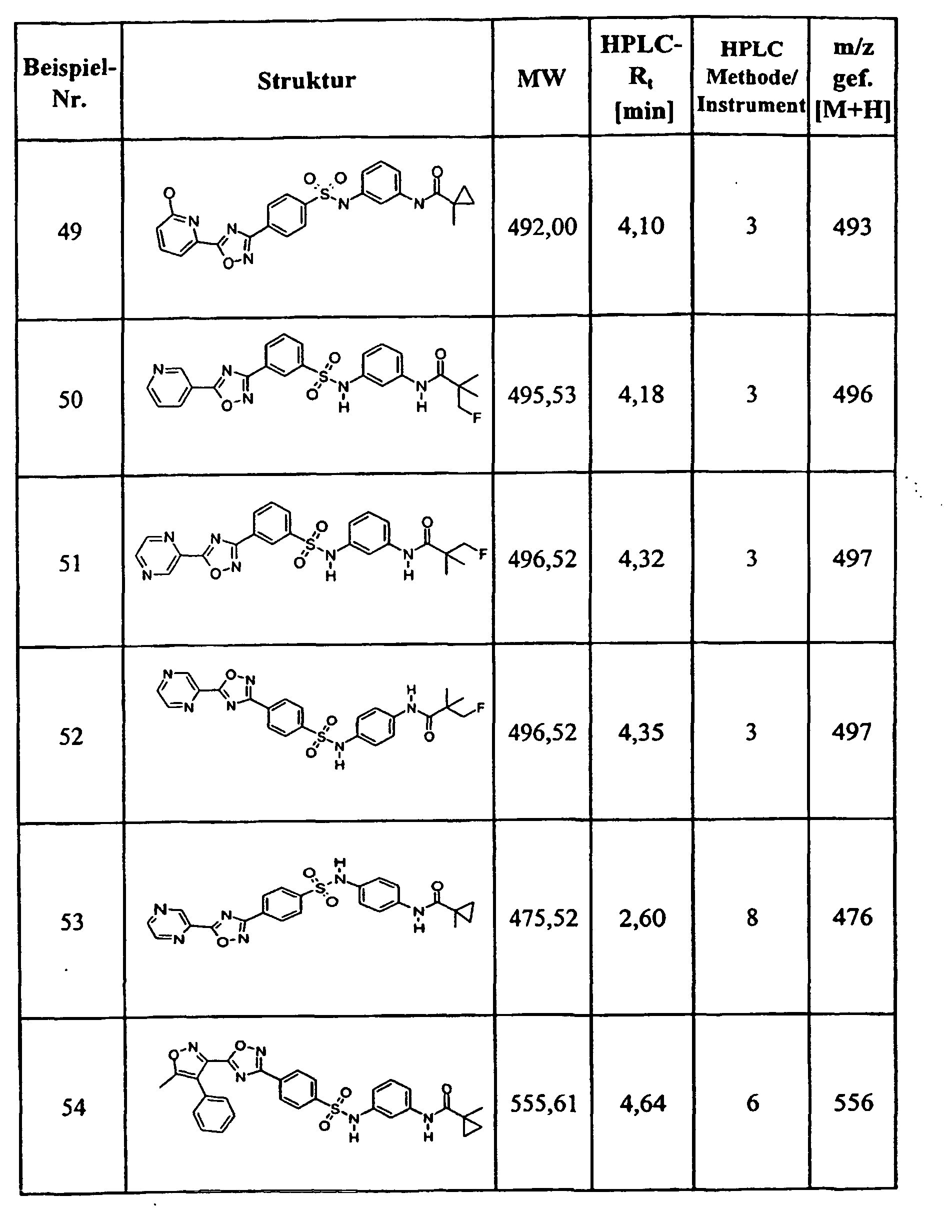

Weitere gemäß den erfindungsgemäßen Verfahren hergestellte, über die Position 5 verknüpfte 1,3,4-Oxadiazol-Derivate sind in Tabelle 3 aufgeführt:

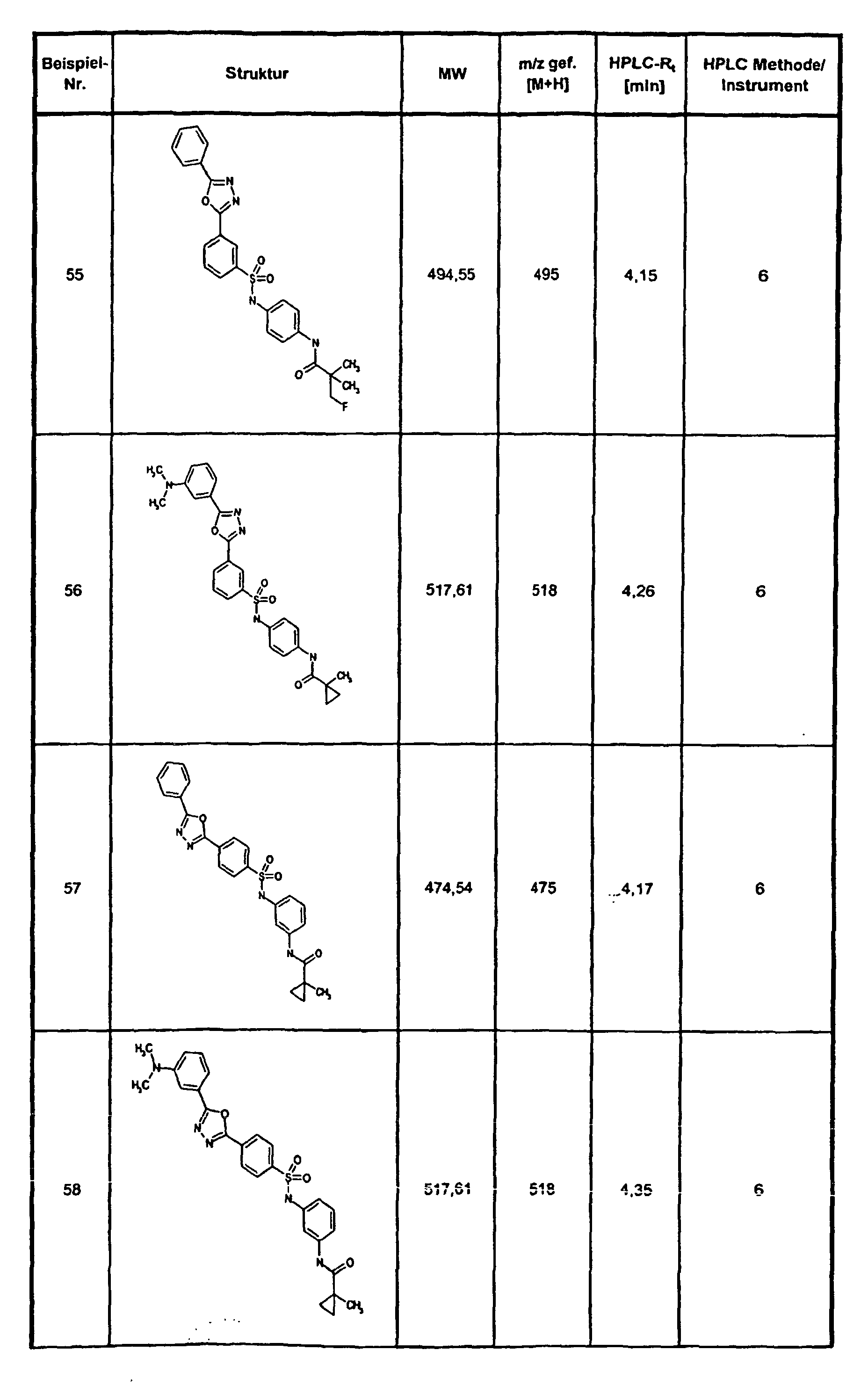

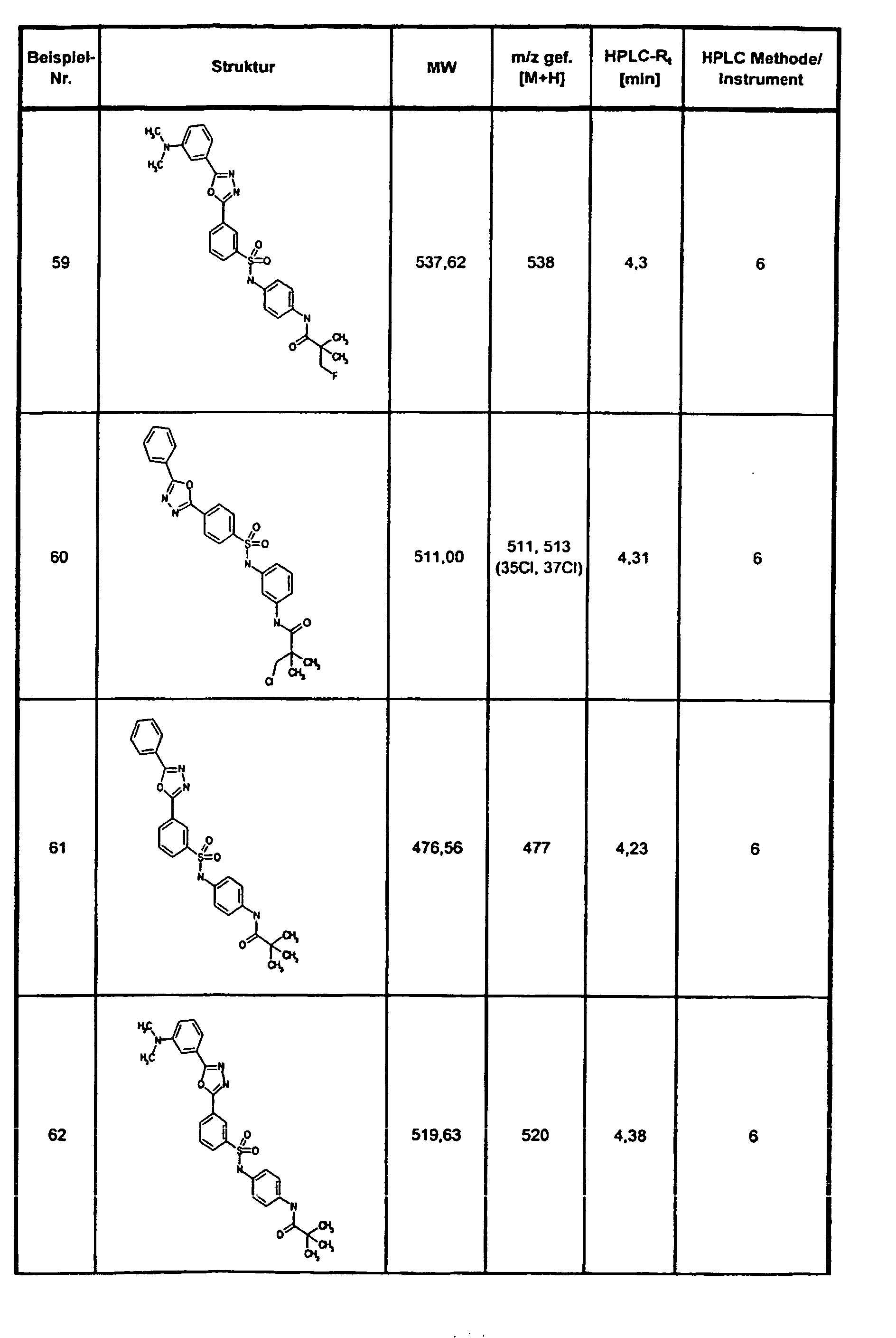

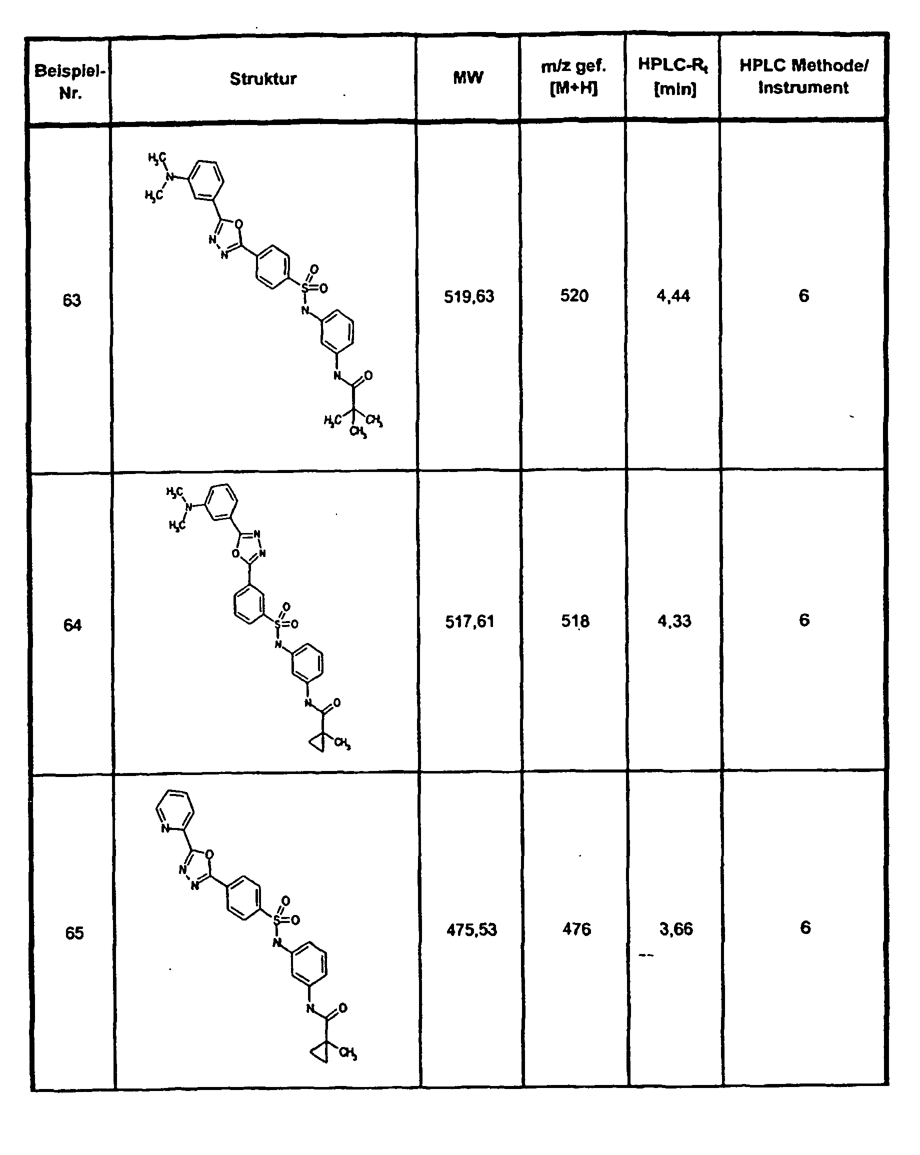

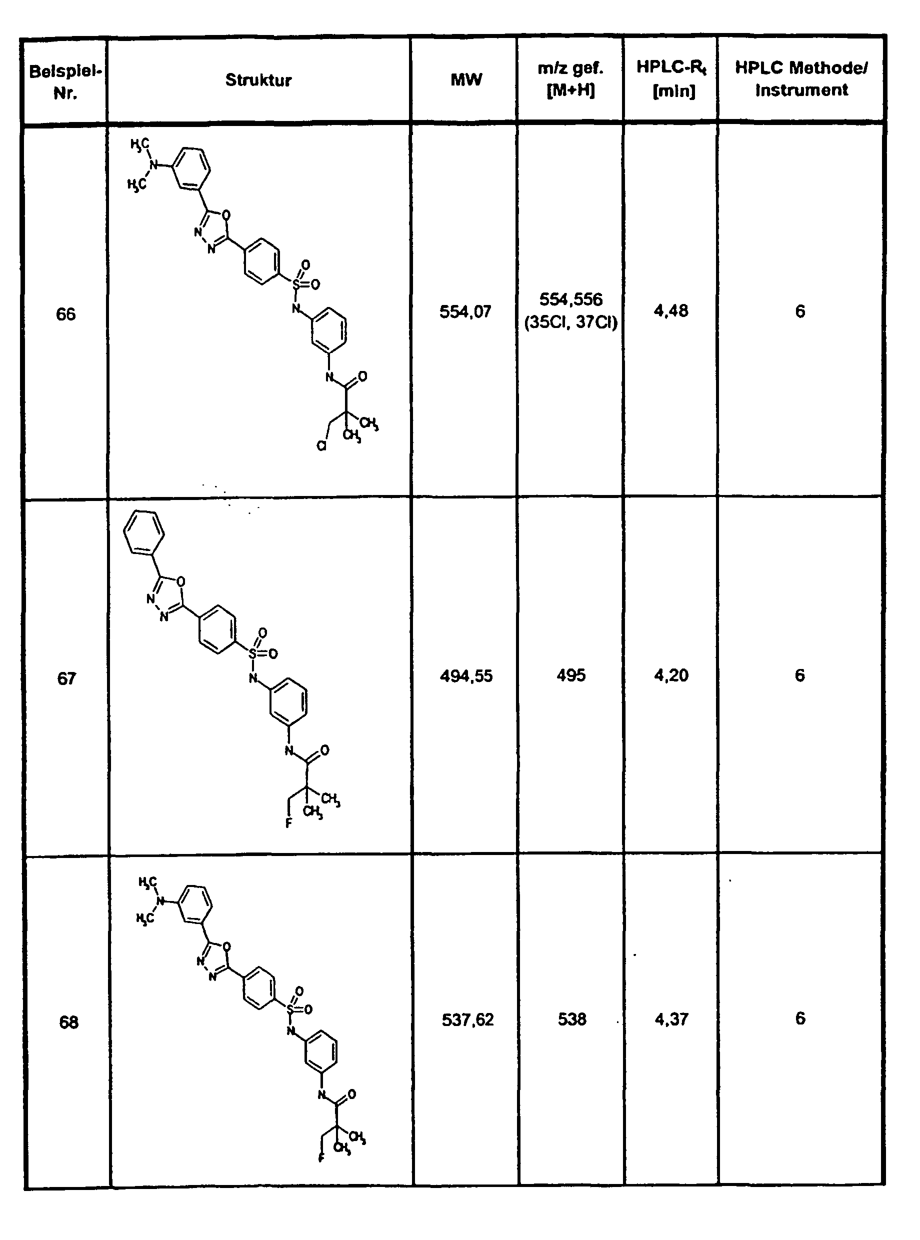

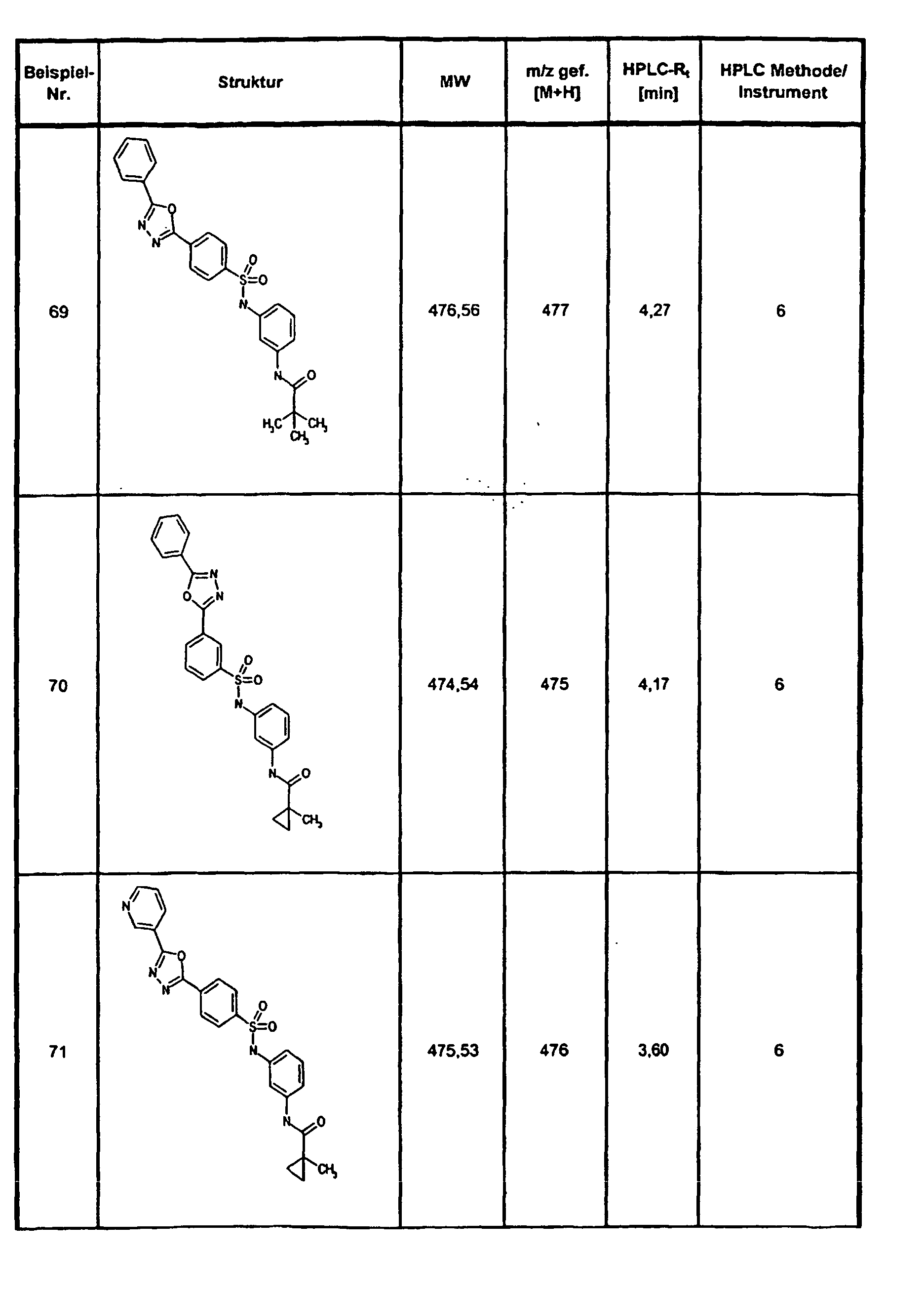

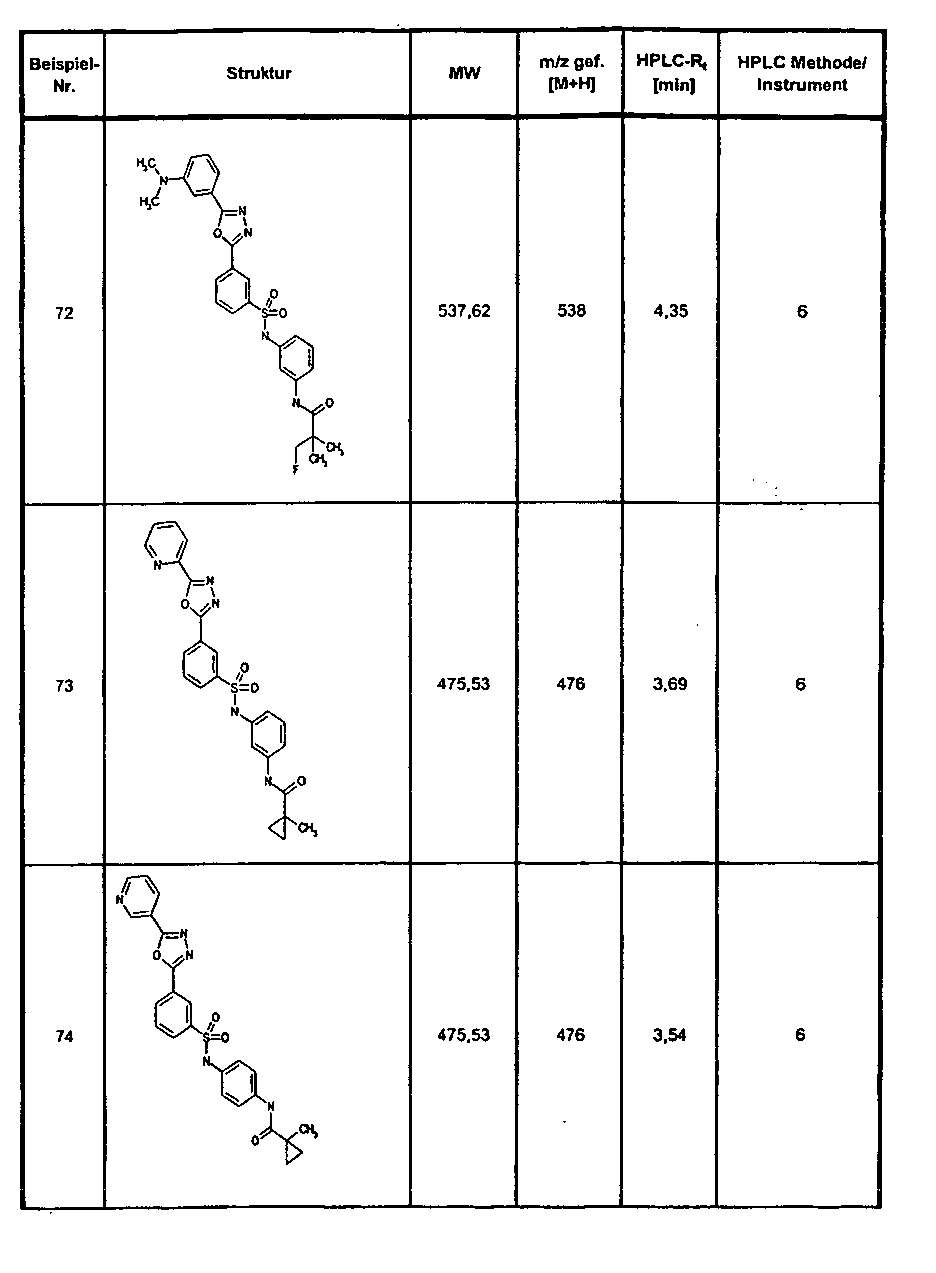

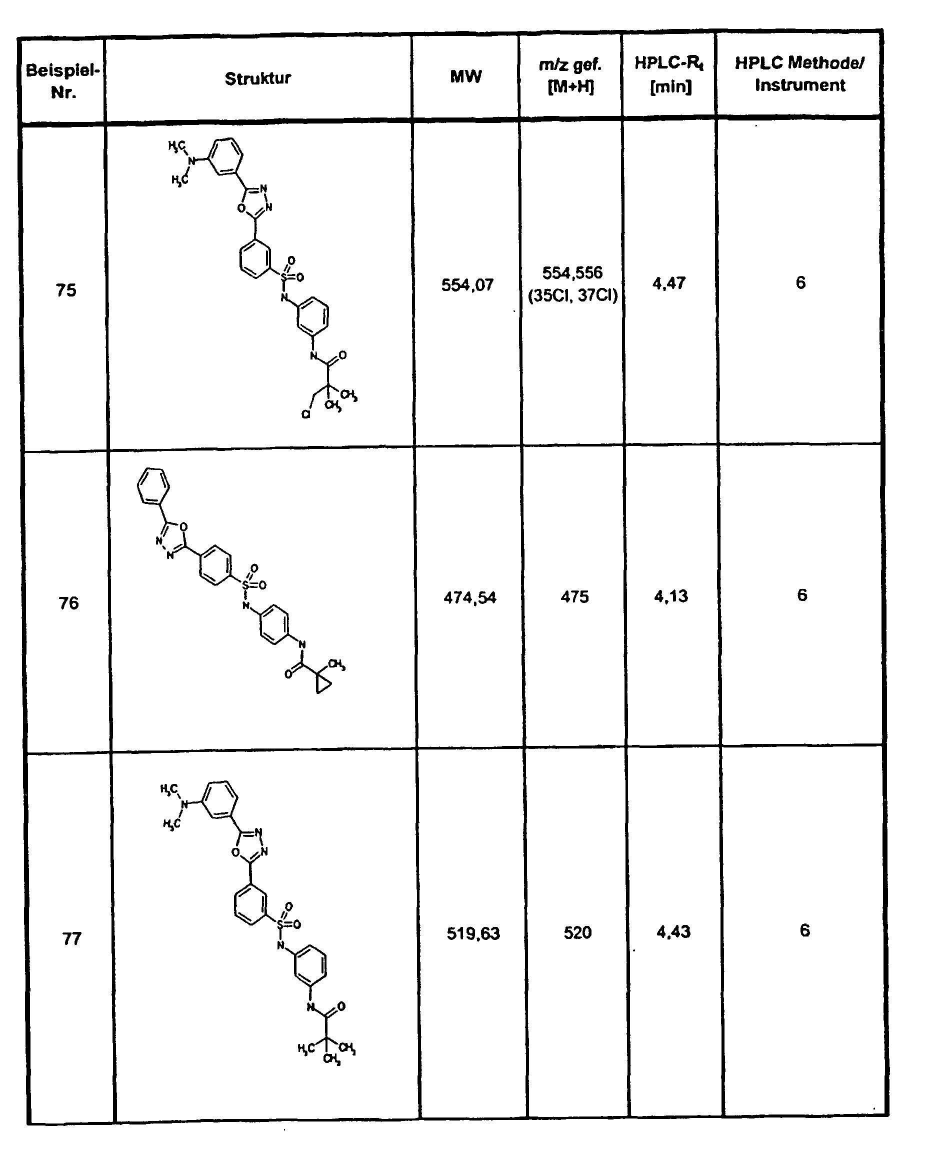

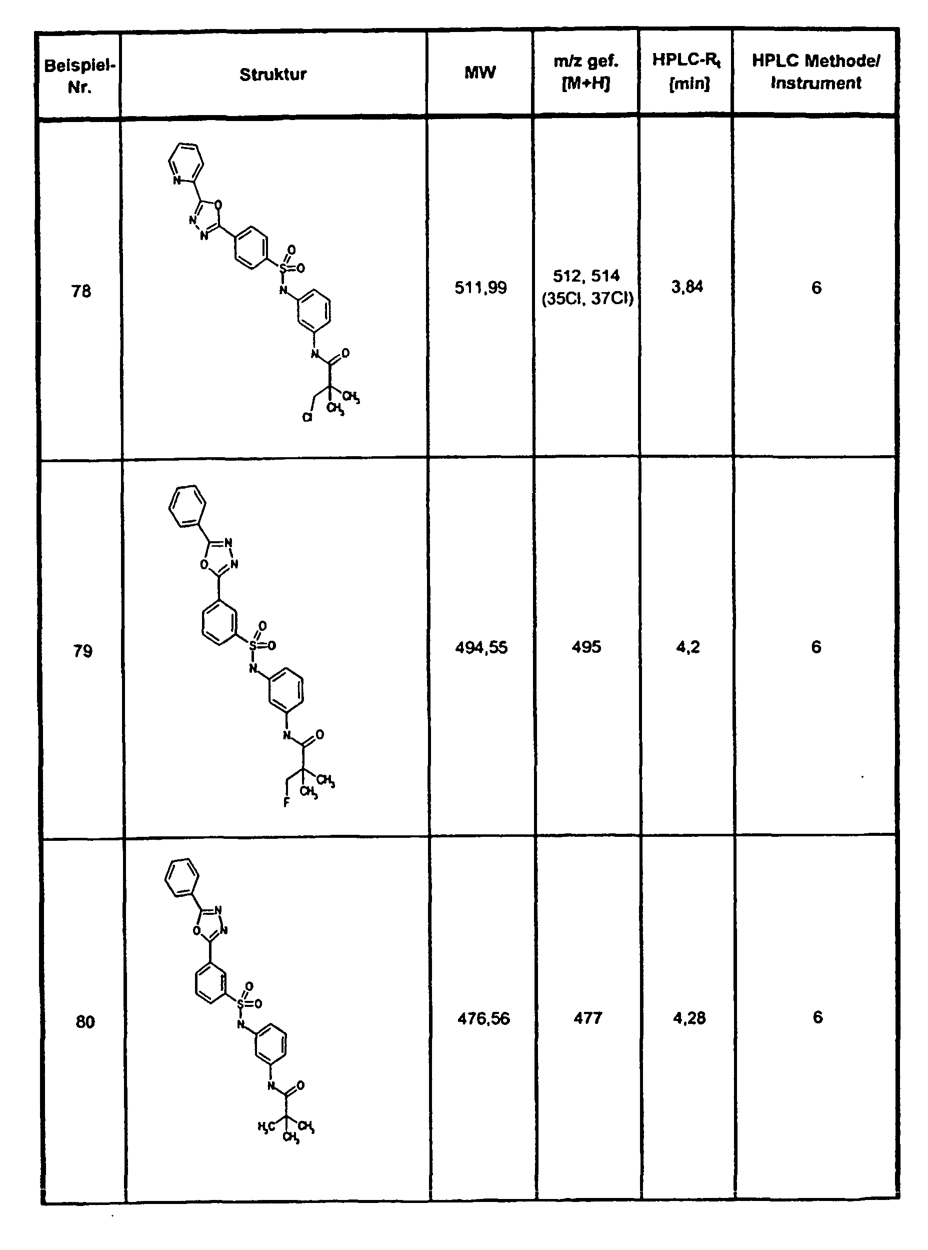

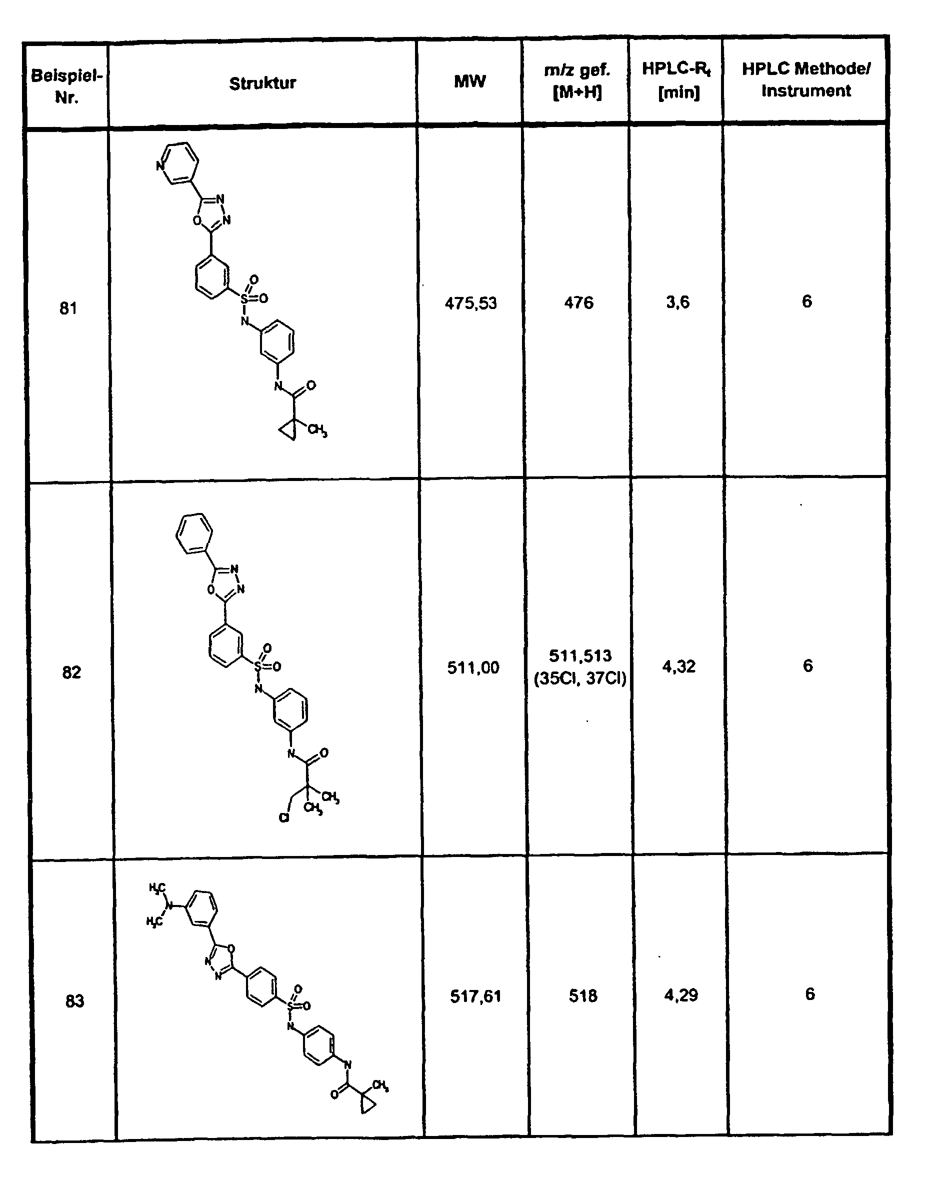

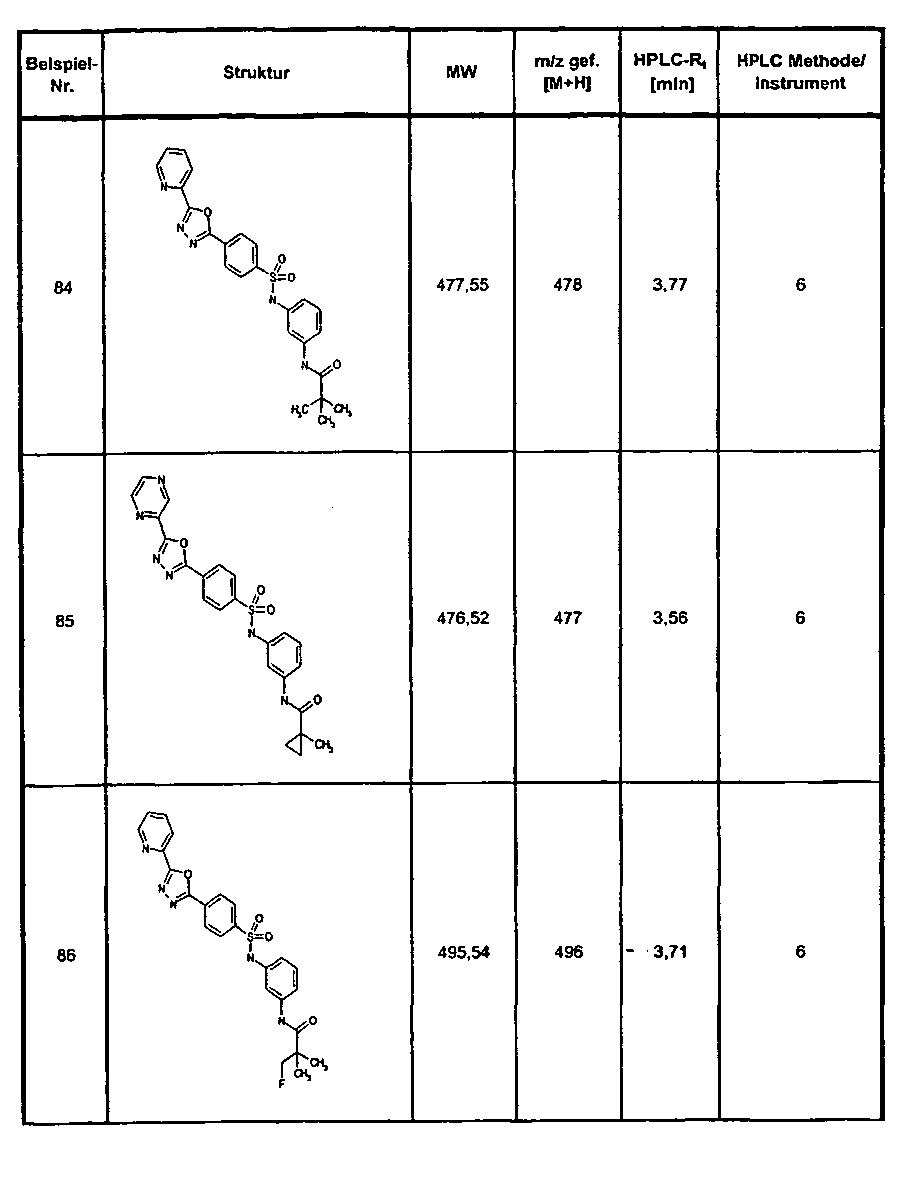

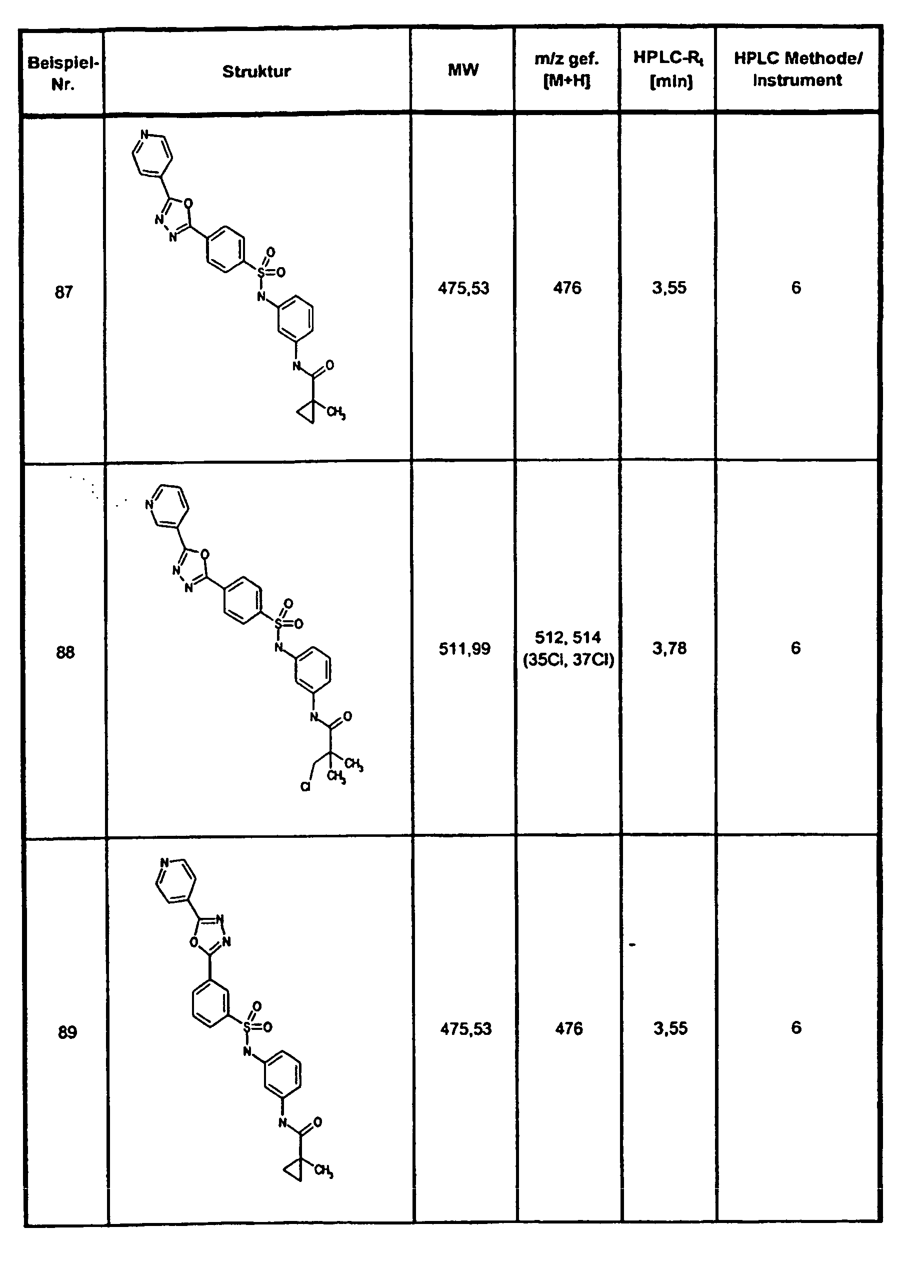

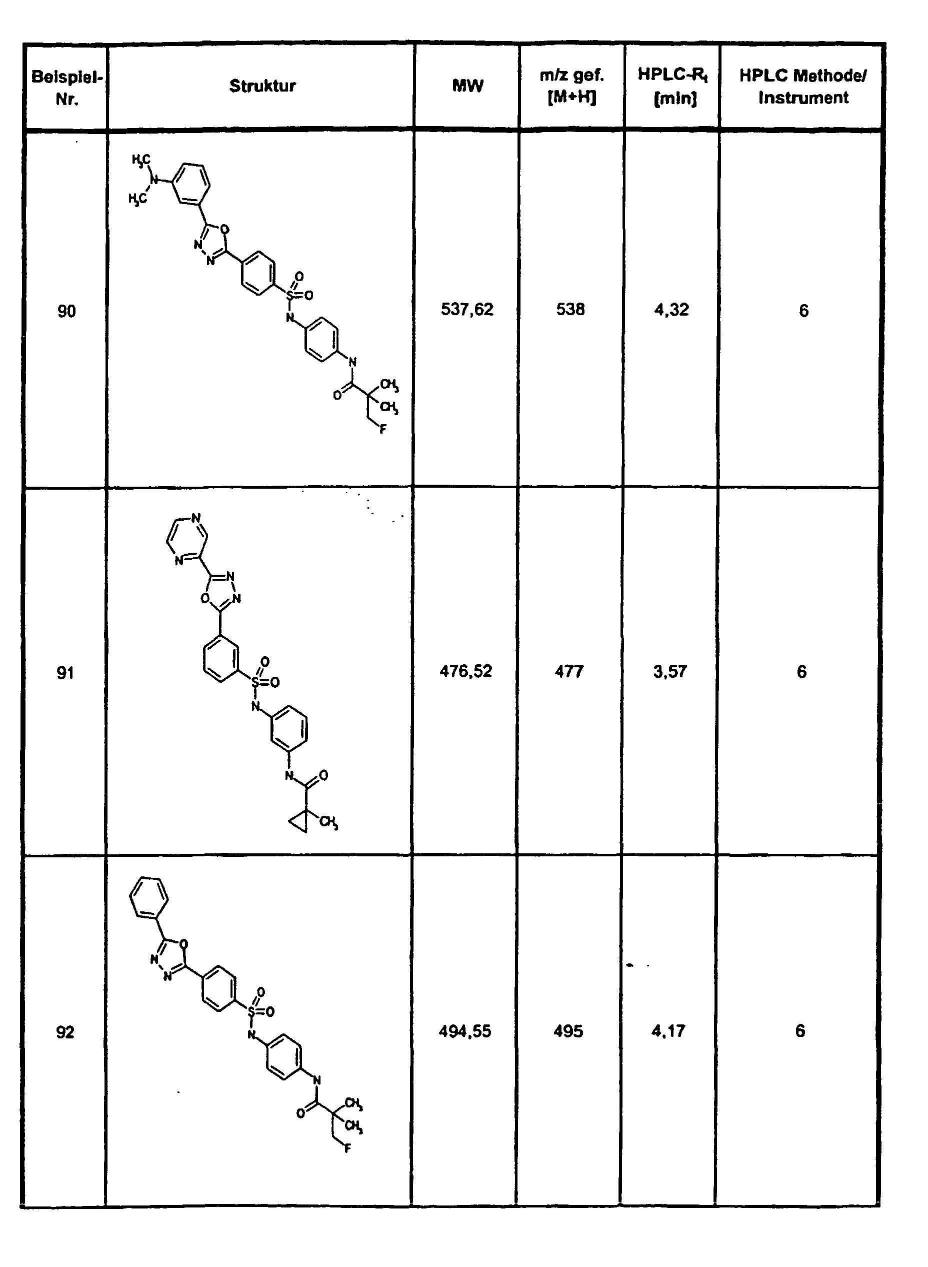

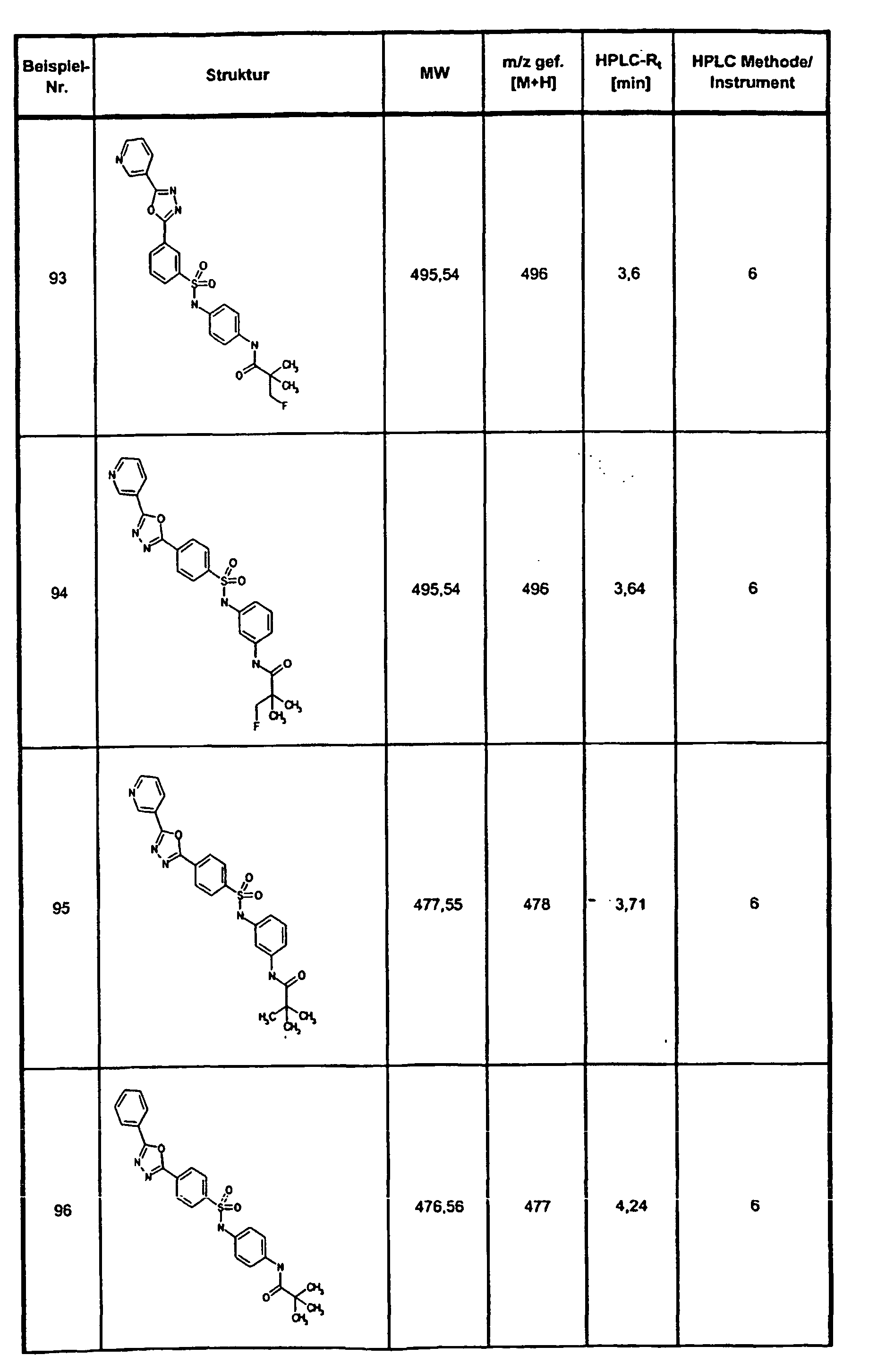

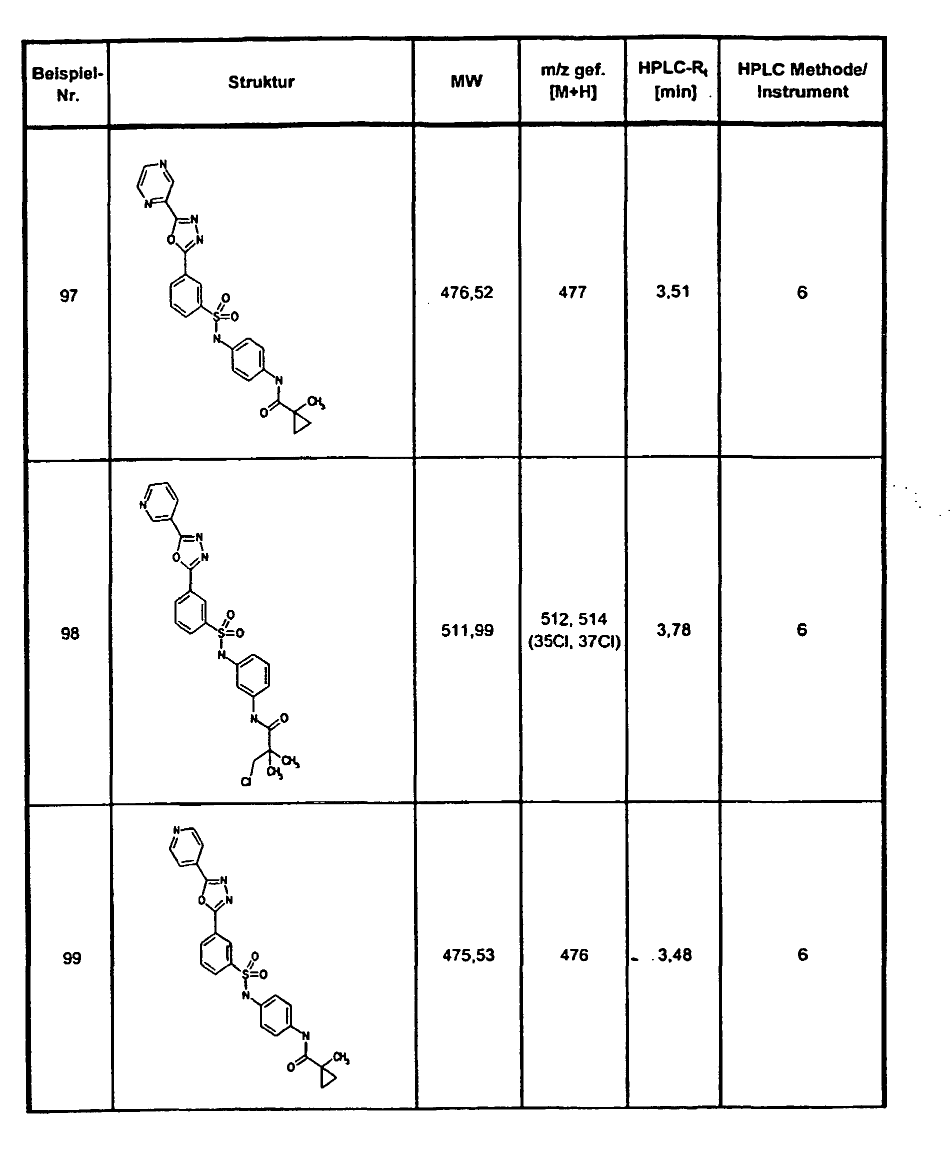

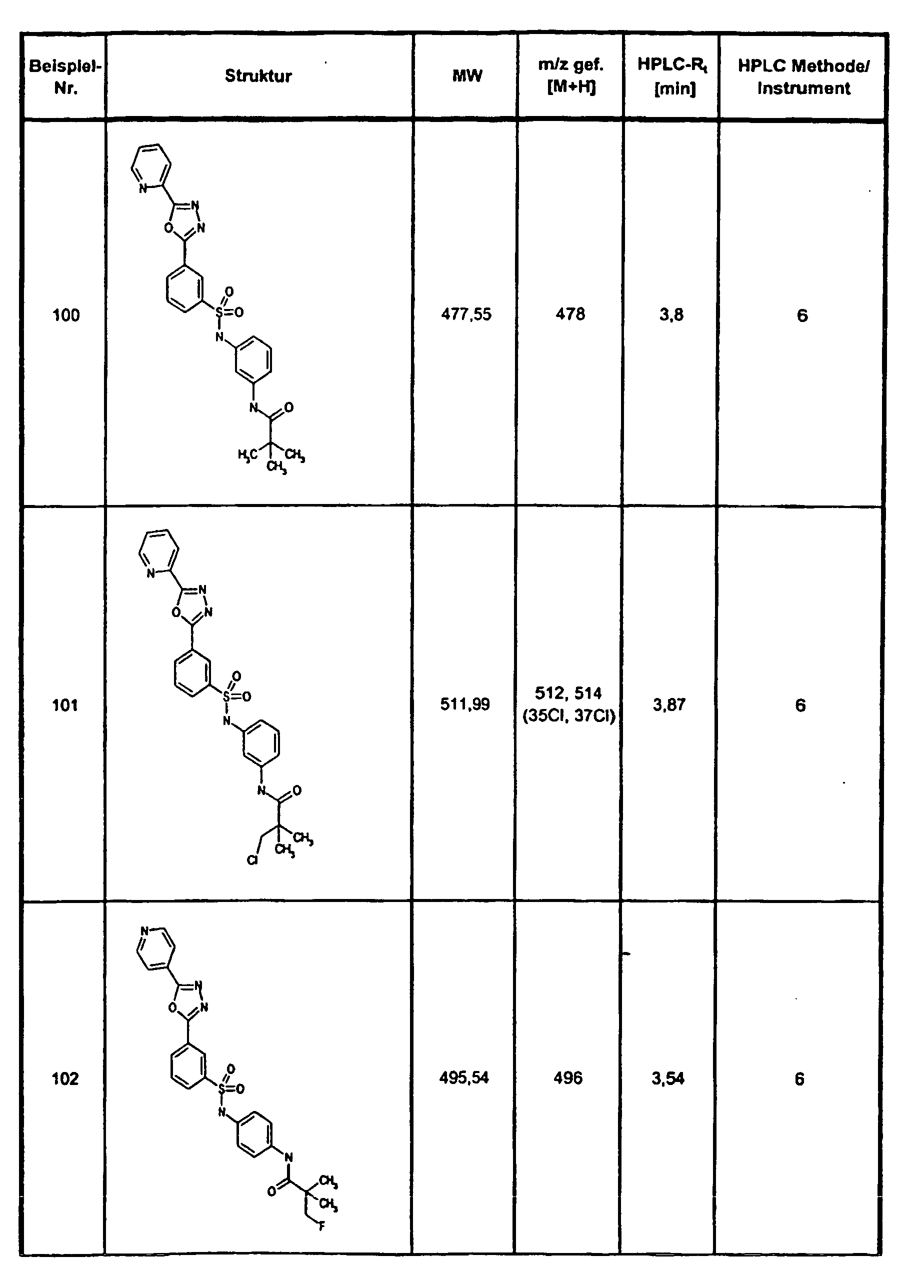

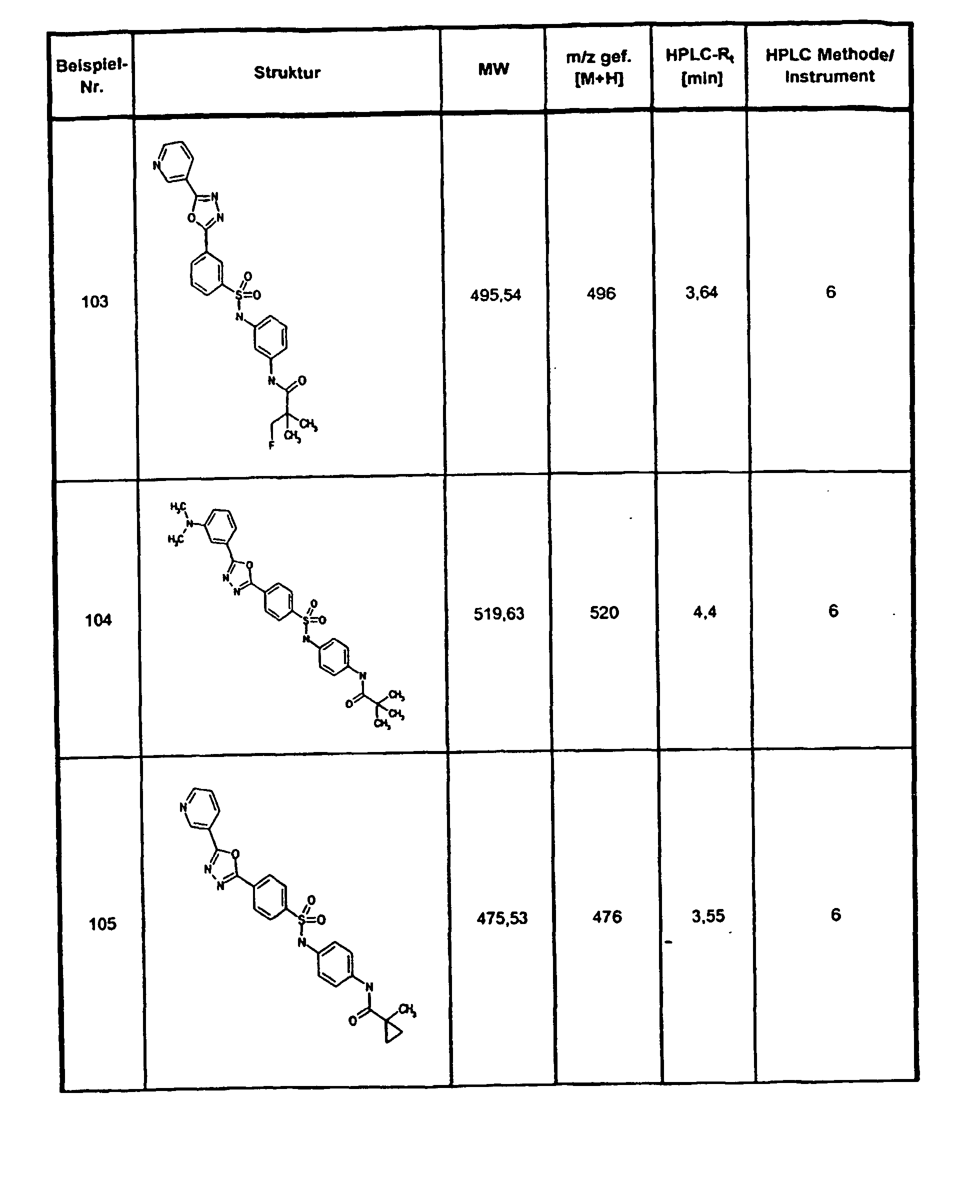

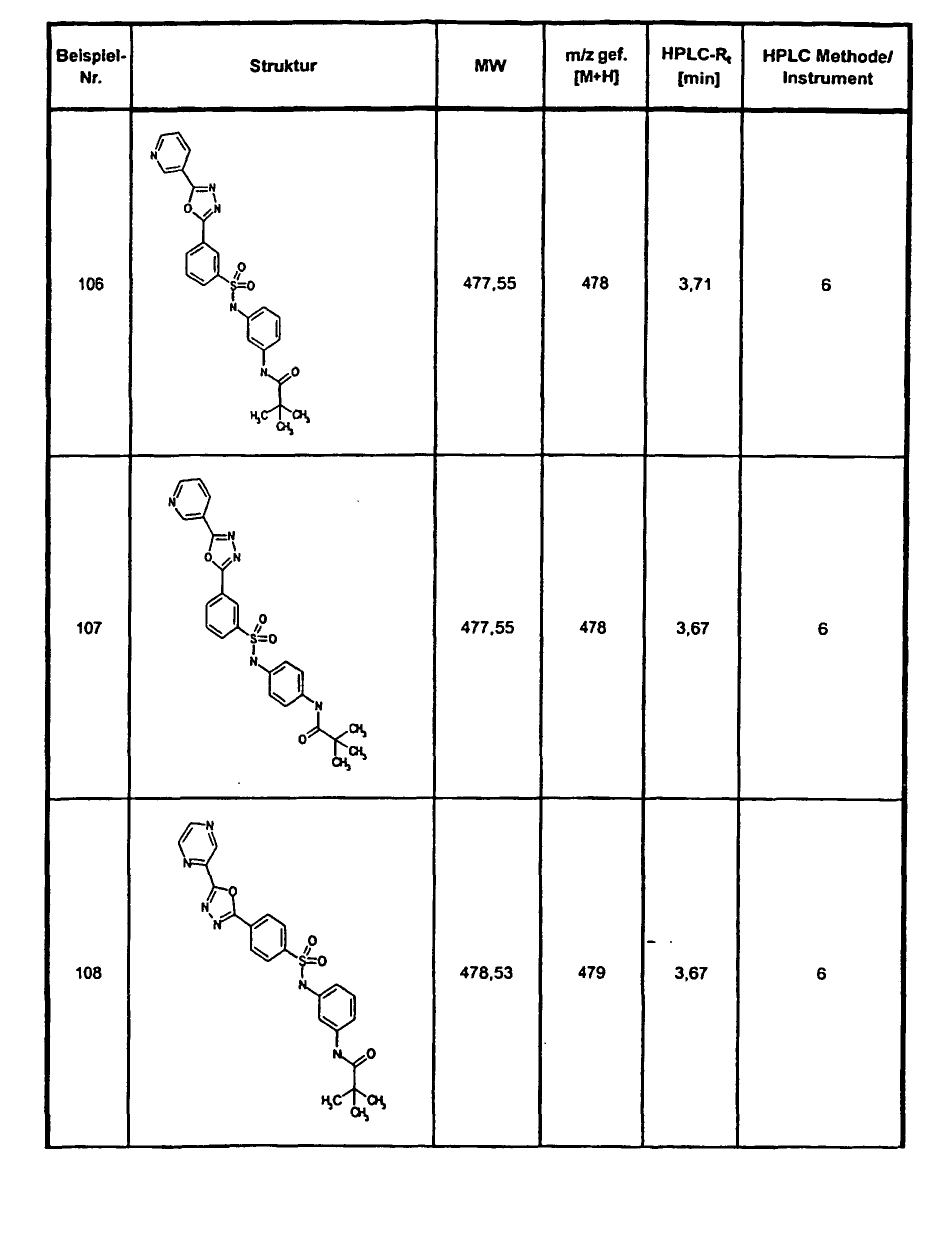

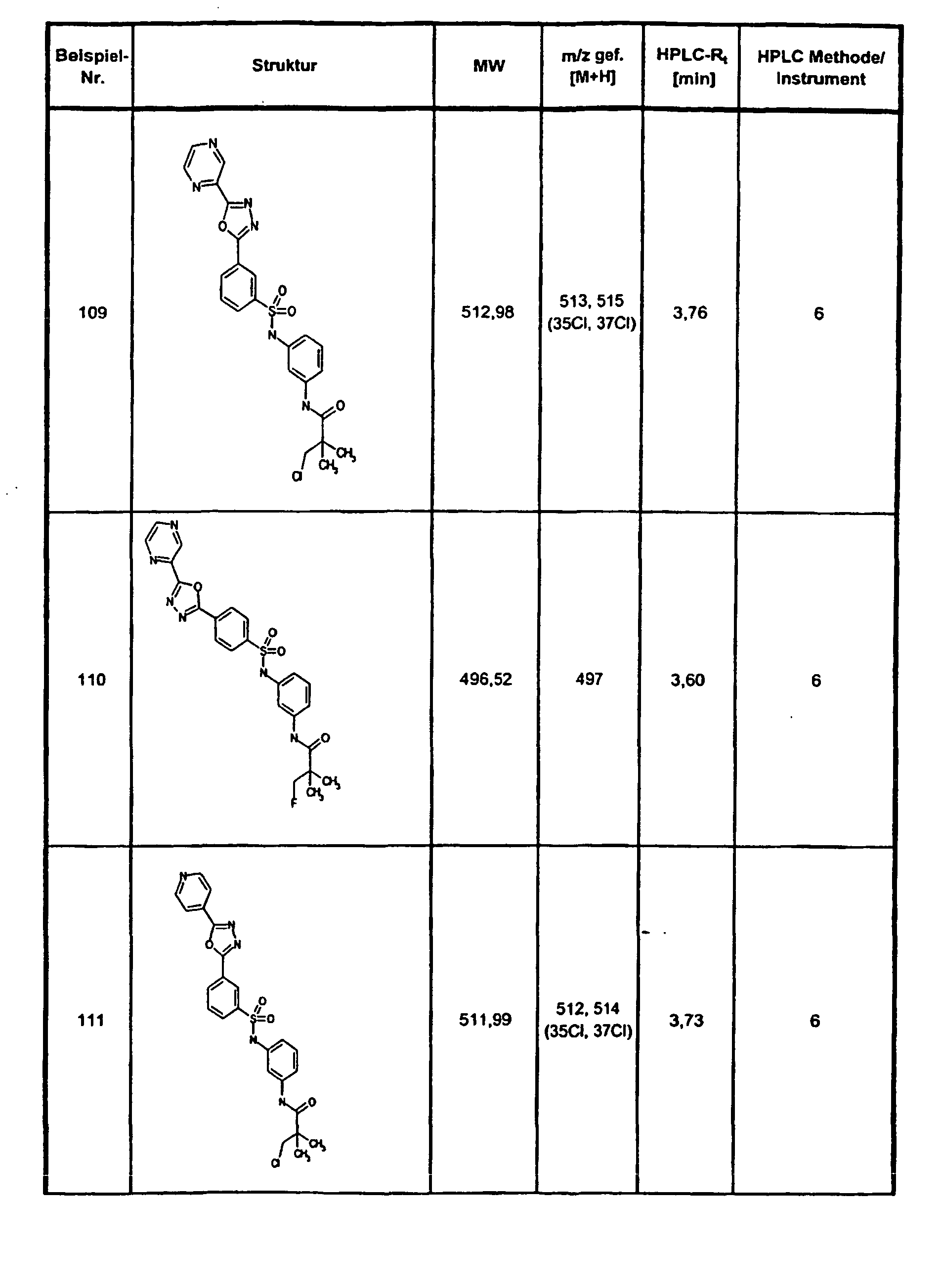

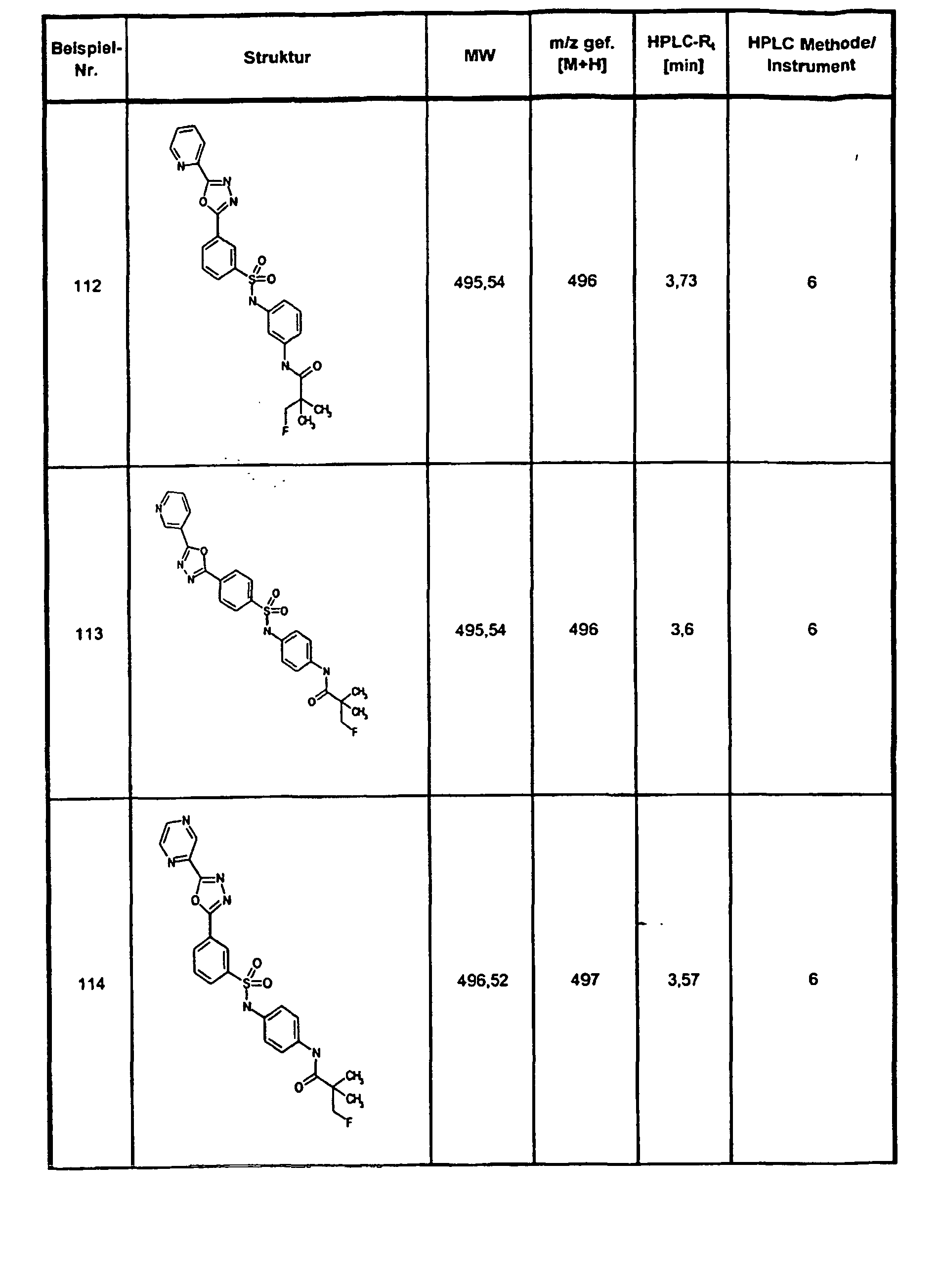

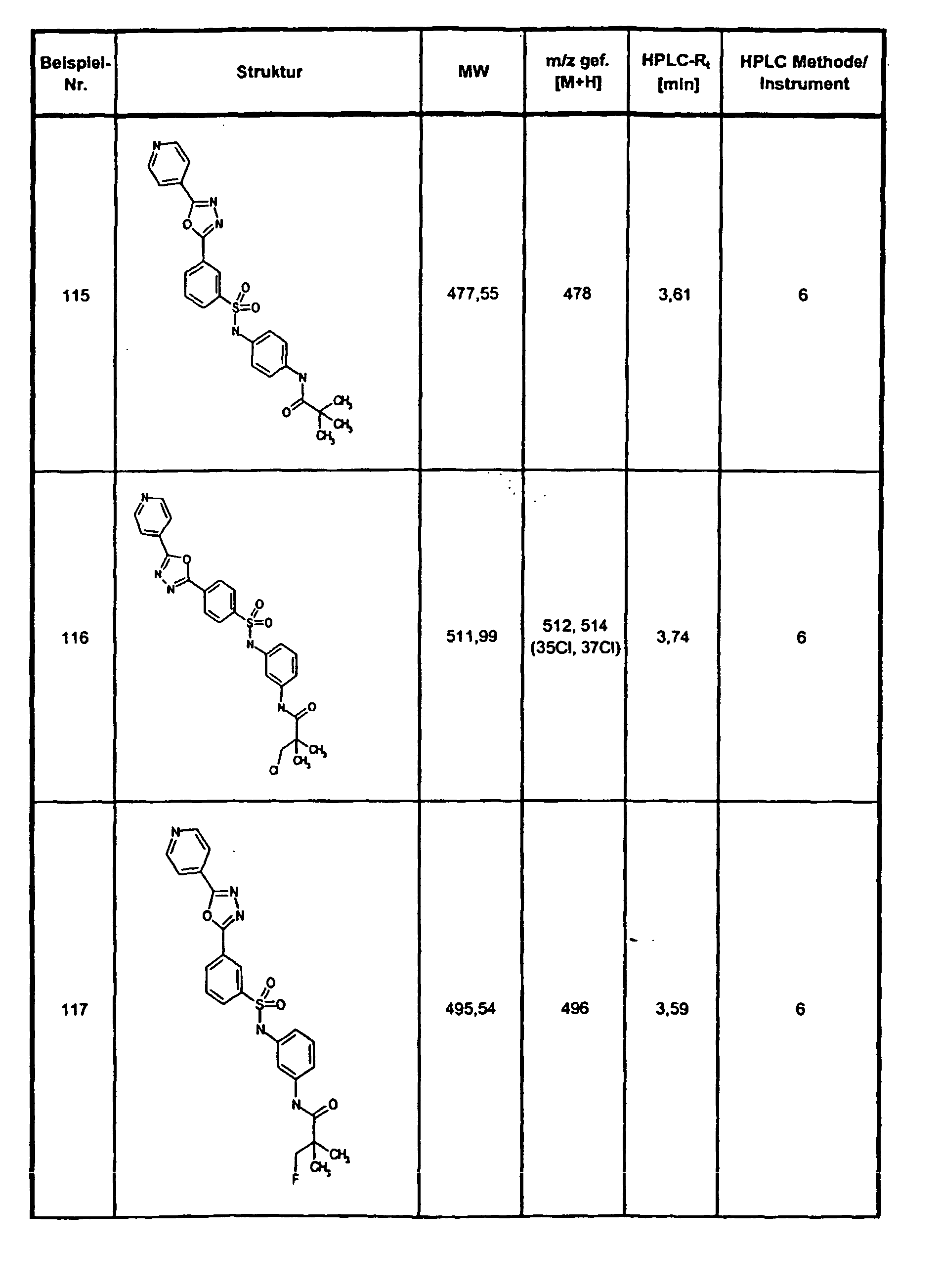

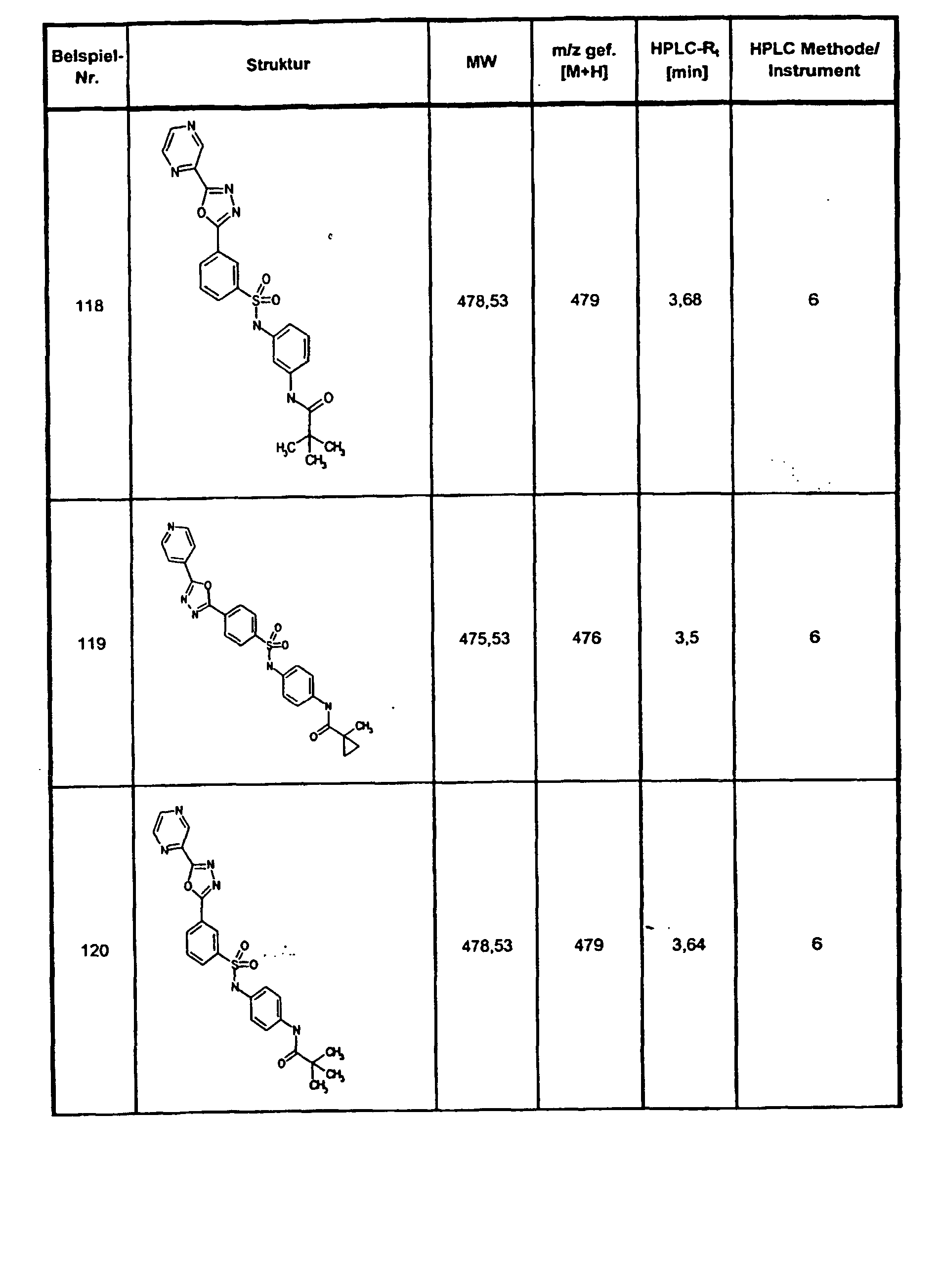

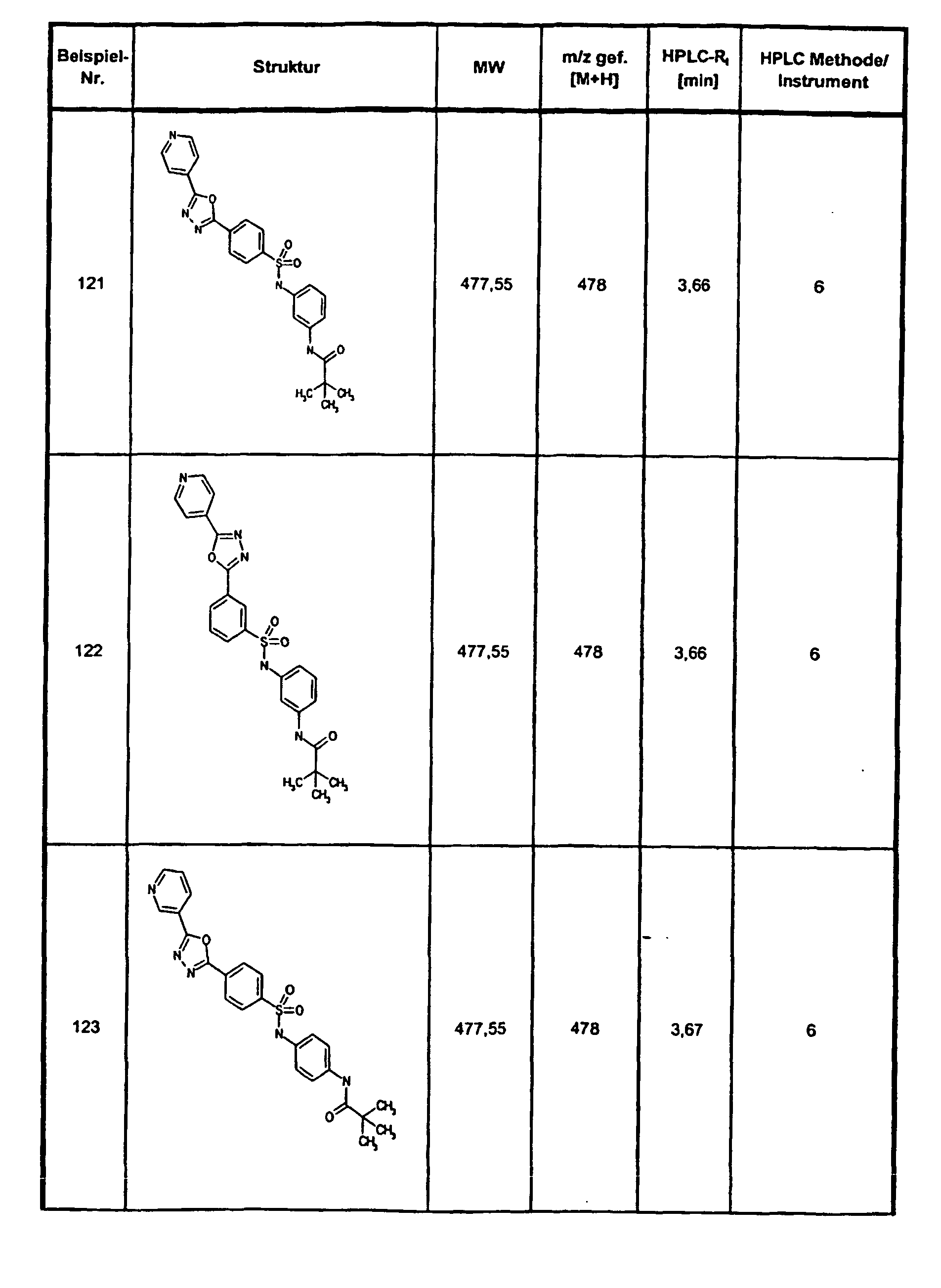

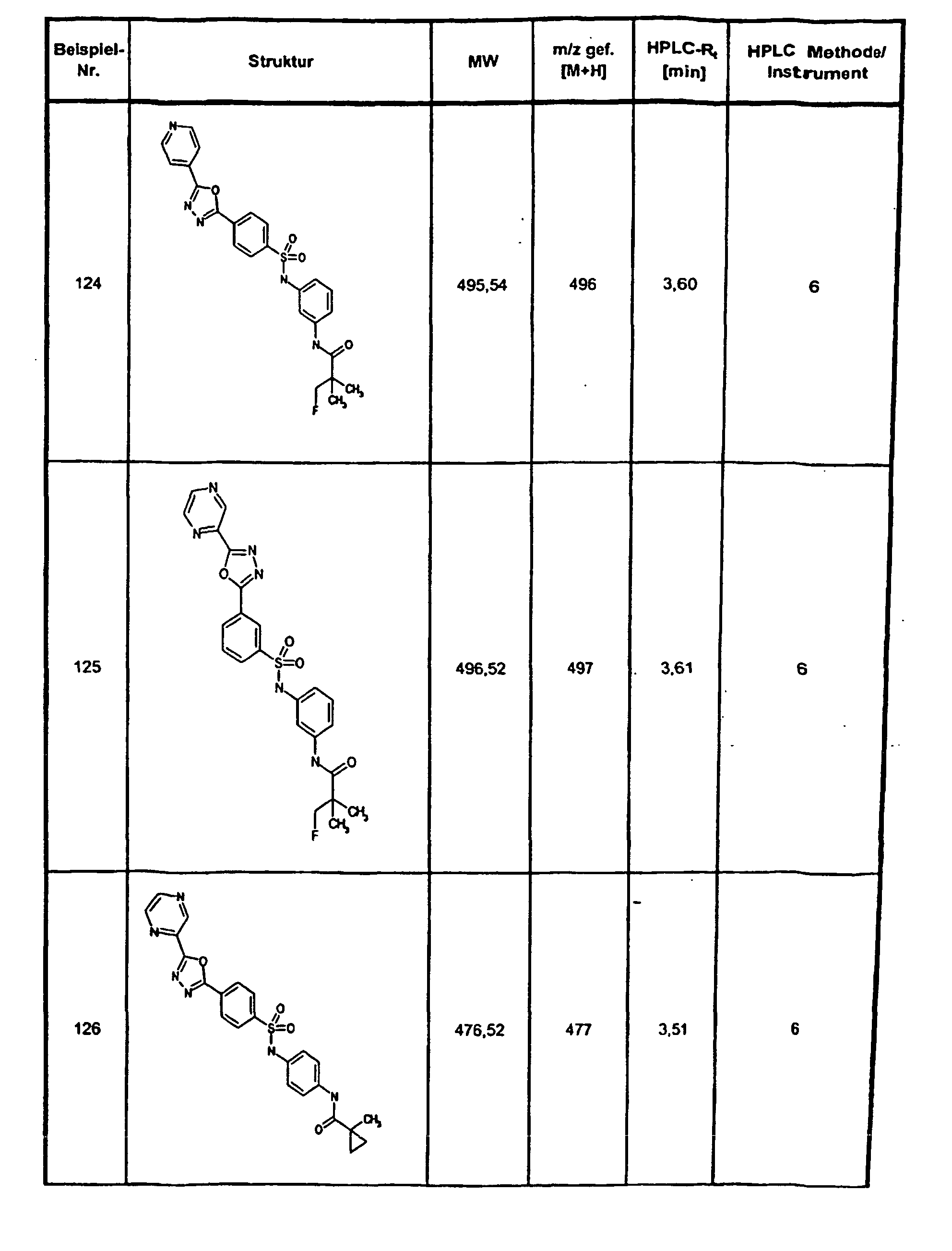

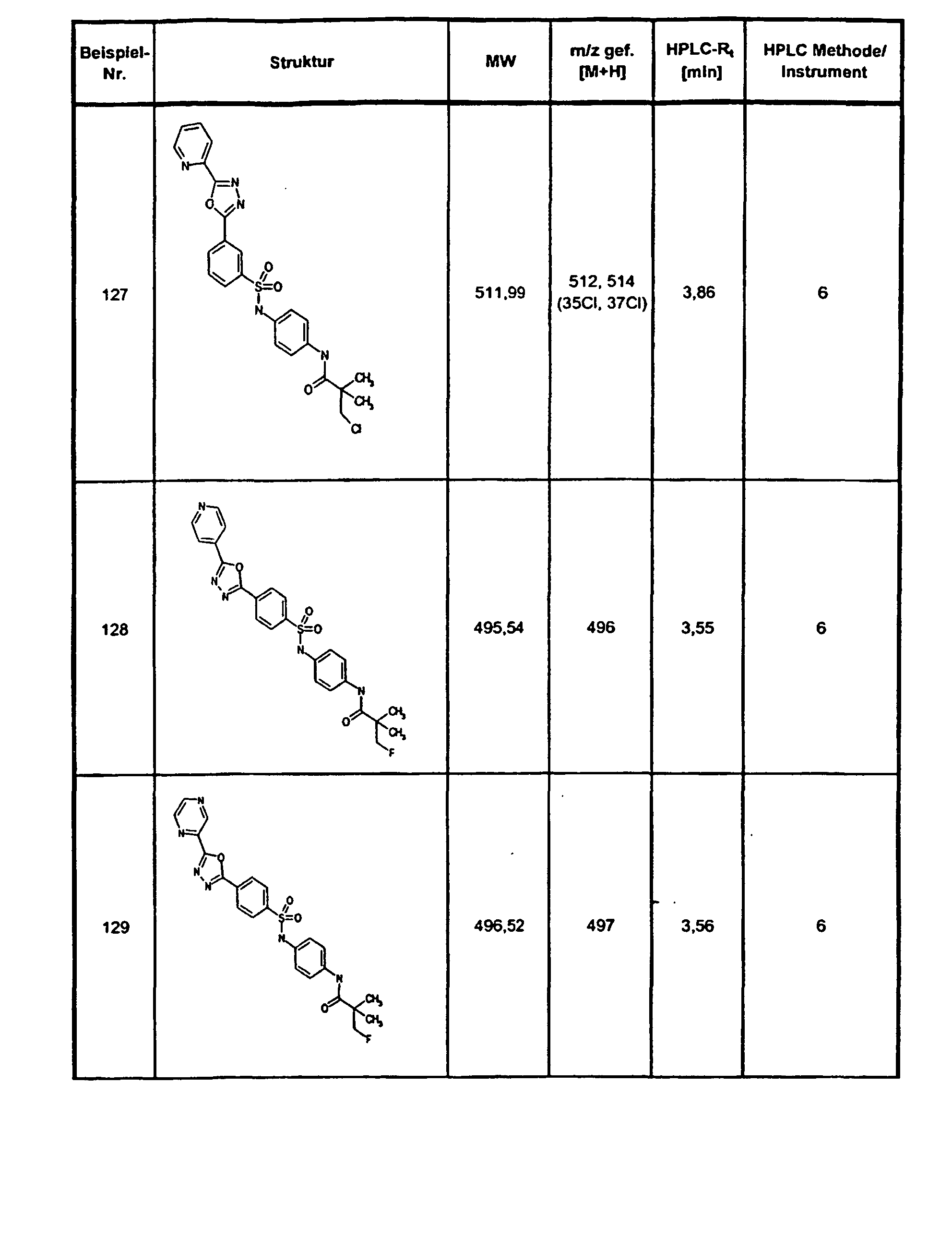

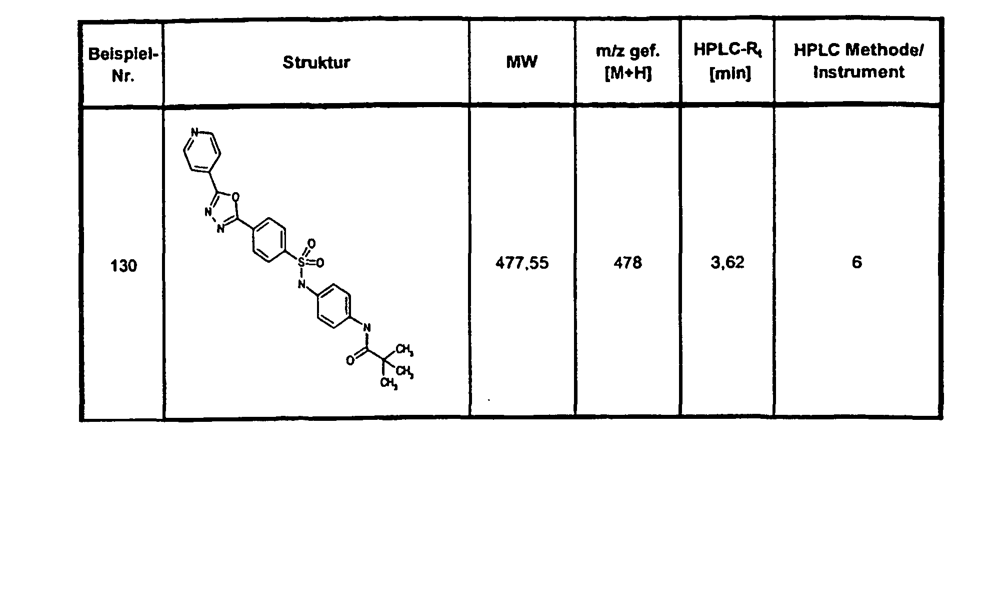

### Beispiel 131

### 1-Methyl-N-{4-[({3-[5-(2-pyridinyl)-1,3,4-thiadiazol-2-yl]phenyl}sulfonyl)amino]-phenyl}cyclopropancarboxamid

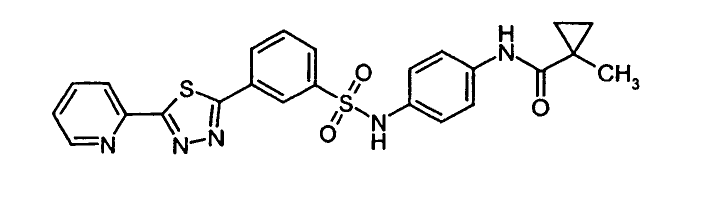

- MW 491,59;: m/z gef.: 490 (ESI-neg.)
- HPLC-Rt:: 4,03 min. (HPLC-Methode/Instrument: 5)
¹H-NMR (400 MHz, DMSO): δ = 0,56-0,59 (m, 2 H), 1,01-1,06 (m, 2 H), 1,34 (s, 3 H), 7,04 (d, 2 H), 7,48 (d, 2 H), 7,63-7,65 (m, 1 H), 7,76 (t, 3H), 7,88 (d, 1H), 8,09 (dt, 1H), 8,26 (dt, 1H), 8,34 (d, 1H), 8,44 (t, 1H), 8,75-8,77 (m, 1H), 10,10 (s, 1 H), 10,27 (s, 1 H).

### Beispiel 132

### N-(3-{[(4-{5-[6-(Acetylamino)-2-pyridinyl]-1,2,4-oxadiazol-3-yl}phenyl)sulfonyl]-amino}phenyl)-1-methylcyclopropancarboxamid

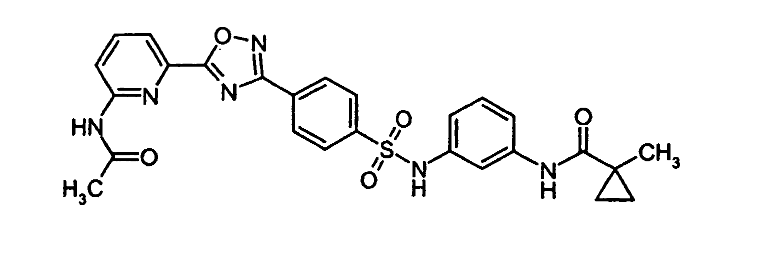

In 50 ml THF werden 8.1 g (20.85 mmol) des Amidoxims aus Beispiel VII vorgelegt, dann 4.13 g (22,94 mmol) 2-Aminoacetylpicolinsäure (Beispiel IX) und 16.28 g (31.28 mmol) PyBOP zugegeben und schließlich 2.96 g (22.94 mmol) N,N-Diisopropylethylamin zugetropft. Der Ansatz wird 16 h bei Raumtemperatur gerührt, dann das Reaktionsgemisch i. Vak. eingeengt, in Dichlormethan aufgenommen und nacheinander je einmal mit 1 N Salzsäure und ges. Kochsalz-Lösung gewaschen. Die organische Phase wird über Natriumsulfat getrocknet, filtriert und i. Vak. eingeengt. Der Rückstand (8.26 g) wird in 75 ml N,N-Dimethylformamid gelöst und 4 h bei 115°C gerührt. Nach dem Abkühlen werden 200 ml Ethylacetat zugegeben, 1x mit 1 N Salzsäure, 1x mit ges. Kochsalz-Lösung, 2x mit ges. Natriumhydrogencarbonat-Lösung und 1x mit ges. Kochsalz-Lösung gewaschen. Dabei setzt in der organischen Phase eine Kristallisation ein. Die organische Phase wird daher 30 min. stehen gelassen, die ausgefallenen Kristalle abgesaugt und mit Methanol nachgewaschen [1. Fraktion, Ausbeute: 5.04 g (23% d.Th.)]. Die Mutterlauge wird über Natriumsulfat getrocknet, filtriert und einrotiert. Durch Verrühren mit Dichlormethan erhält man zwei weitere Fraktionen kristallinen Produktes [Fraktion 2, Ausbeute: 3.5 g (16% d.Th.); Fraktion 3, Ausbeute: 1.1 g (5% d.Th.)]. Die restliche Mutterlauge enthält weiteres Produkt, welches chromatographisch gereinigt werden kann [Ausbeute: 1.01 g (5% d.Th.)].
HPLC: Rt = 4.42 min (HPLC-Methode/Instrument 3)
MW 550.59; m/z gef.: 551
¹H-NMR (200 MHz, DMSO-d₆): δ = 11.00 (s, 1H); 10.41 (s, 1H); 9.18 (s, 1H); 8.40 (dd, 1H); 8.24 (d, 2H); 8.14-9.97 (m, 4H); 7.57 (t, 1H); 7.27 (d, 1H); 7.12 (t, 1H); 6.78 (d, 1H); 2.15 (s, 3H); 1.37 (s, 3H); 1.08-1.03 (m, 2H); 0.62-0.57 (m, 2H).

### Beispiel 133

### N-(3- {[(4-{5-[6-(Acetylamino)-2-pyridinyl]-1,2,4-oxadiazol-3-yl}phenyl)sulfonyl]-amino}phenyl)-3-fluor-2,2-dimethylpropanamid

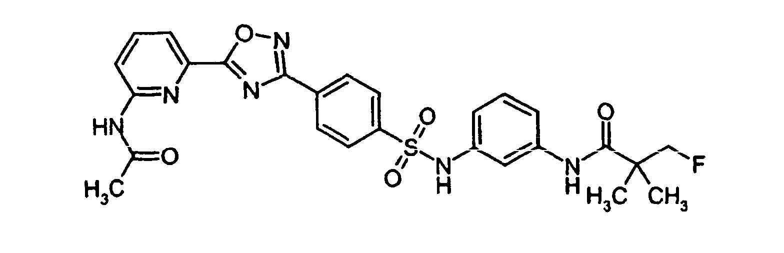

In 100 ml THF werden 7.80 g (19.1 mmol) des Amidoxims aus Beispiel V vorgelegt, dann 3.78 g (21.0 mmol) 2-Aminoacetylpicolinsäure und 14.91 g (28.6 mmol) PyBOP zugegeben und schließlich 2.71 g (21.0 mmol) N,N-Diisopropylethylamin zugetropft. Der Ansatz wird 16 h bei 40°C gerührt, dann das Reaktionsgemisch i. Vak. eingeengt, in Ethylacetat aufgenommen und nacheinander je zweimal mit 1 N Salzsäure und ges. Kochsalz-Lösung gewaschen. Die organische Phase wird über Natriumsulfat getrocknet, filtriert und i. Vak. eingeengt. Das Produkt wird durch Filtration an Kieselgel 60 mit Ethylacetat als Laufmittel gereinigt. Das so erhaltene Produkt (9.9 g) wird in 90 ml N,N-Dimethylformamid gelöst und 4 h bei 115°C gerührt. Nach dem Abkühlen wird das Lösungsmittel i. Vak. entfernt, 200 ml Ethylacetat zugegeben, 1x mit 1 N Salzsäure, 1x mit ges. Kochsalz-Lösung, 2x mit ges. Natriumhydrogencarbonat-Lösung und 1x mit ges. Kochsalz-Lösung gewaschen. Die organische Phase wird über Natriumsulfat getrocknet und anschließend das Löungsmittel entfernt. Beim Einengen bildet sich ein Niederschlag, worauf die Suspension mit Dichlormethan verdünnt wird, der Niederschlag abgetrennt und mit Dichlormethan nachgewaschen wird.
- Ausbeute:: 5.4 g (55% d.Th.)
- HPLC:: Rt = 4.44 min (HPLC-Methode/Instrument 3)
- MW 552.58;: m/z gef.: 553
¹H-NMR (200 MHz, DMSO-d₆): δ = 10.99 (s, 1H); 10.43 (s, 1H); 9.36 (s, 1H); 8.40 (dd, 1H); 8.25 (d, 2H); 8.13-7.98 (m, 4H); 7.59 (t, 1H); 7.30 (d, 1H); 7.15 (t, 1H); 6.81 (d, 1H); 4.48 (d, 1H); 2.16 (s, 3H); 1.21 (s, 6H).

### Beispiel 134

### N- {3-[({4-[5-(6-Amino-2-pyridinyl)-1,2,4-oxadiazol-3-yl]phenyl}sulfonyl)amino]-phenyl}-1-methylcyclopropancarboxamid

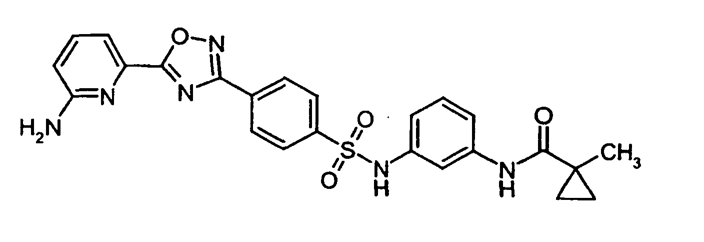

15 g (28.16 mmol) der Verbindung aus Beispiel 132 werden in 370 ml Ethanol suspendiert und mit 279 ml (281.7 mmol) 1 N Natronlauge versetzt. Der Ansatz wird 5 h bei 45°C gerührt (Suspension löst sich leicht auf), anschließend wird das Gemisch im Eisbad mit 1 N Salzsäure auf pH = 5 eingestellt, die ausgefallenen Kristalle abfiltriert, mit Wasser sowie mit Ethanol gewaschen und 16 h im Hochvakuum bei 80°C getrocknet.
- Ausbeute:: 12 g (85,5% d.Th.)
- HPLC:: Rt = 4.06 min (HPLC-Methode/Instrument 3)
- MW 490.54;: m/z gef.: 491
¹H-NMR (200 MHz, DMSO-d₆): δ = 10.40 (s, 1H); 9.15 (s, 1H); 8.21 (d, 2H); 7.96 (d, 2H); 7.68-7.43 (m, 3H); 7.25 (d, 1H); 7.10 (t, 1H); 6.76 (t, 2H); 6.56 (d, 2H); 1.36 (s, 3H); 1.08-1.03 (s, 2H); 0.62-0.57 (m, 2H).

### Beispiel 135

### N-{3-[({4-[5-(6-Amino-2-pyridinyl)-1,2,4-oxadiazol-3-yl]phenyl}sulfonyl)amino]-phenyl}-3-fluor-2,2-dimethylpropanamid

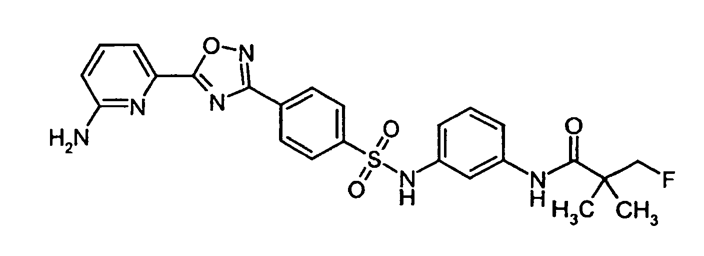

19.3 g (34.9 mmol) der Verbindung aus Beispiel 133 werden in 290 ml eines Gemisches Wasser / konz. Salzsäure (1:1 v/v) aufgenommen und die Suspension 4 h bei 100°C gerührt. Anschließend wird die Suspension filtriert, der Filterkuchen zwischen ges. Natriumhydrogencarbonat-Lösung und Ethylacetat verrührt, die organische Phase abgetrennt, einrotiert und das Rohprodukt chromatographisch gereinigt [Kieselgel 60, Laufmittel Toluol/Aceton (8:2 v/v)]. Um anhaftende Lösungsmittel-Rückstände zu entfemen, werden die sauberen Fraktionen vereinigt (6.8 g), in 130 ml 1 N Natronlauge bei 0°C gelöst (trübe Lösung) und diese Lösung mit 1 N Salzsäure auf pH = 5 angeäuert. Der Niederschlag wird abfiltriert, mit Wasser nachgewaschen und im Hochvakuum getrocknet.
- Ausbeute:: 6.4 g (36 % d.Th.)
- HPLC:: Rt = 4.09 min (HPLC-Methode/Instrument 3)
- MW 510.55;: m/z gef.: 511
¹H-NMR (500 MHz, DMSO-d₆): δ = 10.42 (s, 1H); 9.33 (s, 1H); 8.21 (d, 2H); 7.97 (d, 2H); 7.64 (t, 1H); 7.53 (s, 1H); 7.45 (d, 1H); 7.27 (d, 1H); 7.12 (t, 1H); 6.78 (d, 1H); 6.73 (d, 1H); 6.57 (s, 2H); 4.48 (d, 2H); 1.21 (s, 6H).

### Beispiel 136

### ({6-[3-(4-{[(3-{[(1-Methylcyclopropyl)carbonyl]amino}phenyl)amino]sulfonyl}-phenyl)-1,2,4-oxadiazol-5-yl]-2-pyridinyl}amino)(oxo)-essigsäureethylester

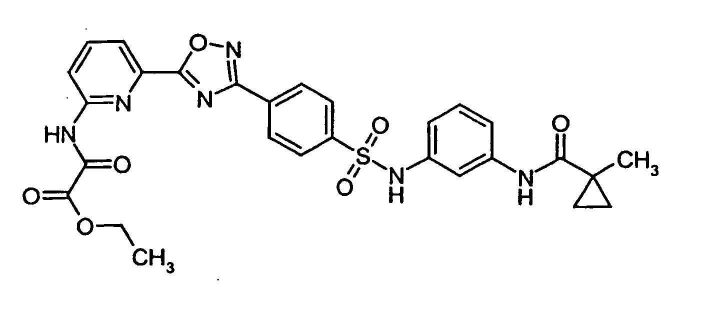

Unter Argon werden 400 mg (0.82 mmol) der Verbindung aus Beispiel 134 in 12 ml Dichlormethan gelöst und unter Rühren 70 mg (0.09 mmol) Pyridin sowie 150 mg (1.1 mmol) Oxalsäuremonoethylesterchlorid zugegeben. Die Lösung wird 30 min bei Raumtemperatur nachgerührt. Anschließend wird das Reaktionsgemisch auf 25 ml pH 7-Puffer gegeben, die wässrige Phase dreimal mit Dichlormethan extrahiert und die vereinigten organischen Phasen je zweimal mit ges. Kochsalz-Lösung, Natriumhydrogencarbonat-Lösung und ges. Kochsalz-Lösung gewaschen. Die organische Phase wird abgetrennt, über Natriumsulfat getrocknet und i. Vak. eingeengt. Das Rohprodukt wird durch Chromatographie an Kieselgel 60 mit Toluol/Ethylacetat (1:1 v/v) als Laufinittel gereinigt.
- Ausbeute:: 349 mg (72% d.Th.)
- HPLC:: Rt = 4.57 min (HPLC-Methode/Instrument 3)
- MW 590.61;: m/z gef.: 591
¹H-NMR (200 MHz, DMSO-d₆): δ = 11.41 (s, 1H); 10.42 (s, 1H); 9.20 (s, 1H); 8.28-8.16 (m, 6H); 8.00 (d, 1H); 7.60 (s, 1H); 7.28 (d, 1H); 7.13 (t, 1H); 6.79 (d, 1H); 4.32 (q, 2H); 1.37-1.29 (m, 6H); 1.08-1.03 (m, 2H); 0.63-0.58 (m, 2H).

### Beispiel 137

### ({6-[3-(4-{[(3-{[(1-Methylcyclopropyl)carbonyl]amino}phenyl)amino]sulfonyl}-phenyl)-1,2,4-oxadiazol-5-yl]-2-pyridinyl}amino)(oxo)-essigsäure

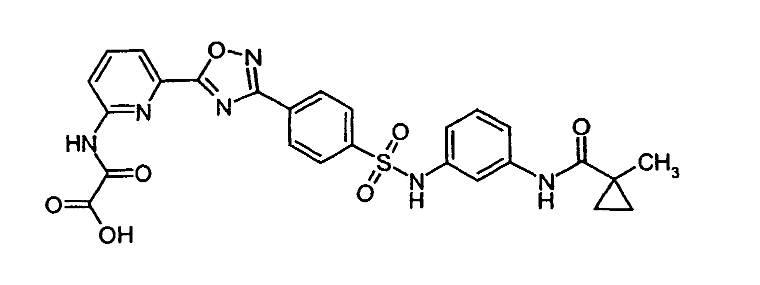

152 mg (0.26 mmol) der Verbindung aus Beispiel 136 werden in 7.5 ml Dioxan aufgenommen und mit 0.75 ml (0.75 mmol) 1 N Natronlauge versetzt. Es wird 16 h bei Raumtemperatur gerührt, anschließend mit 1 N Salzsäure vorsichtig auf pH = 7 angesäuert und das Lösungsmittel i. Vak. entfernt. Das Rohprodukt wird mittels präparativer HPLC (CromSil C18, 250x30 mm, Fluß 50 ml/min, Runtime 35 min, Detektion bei 254 nm, Gradient 10% Acetonitril @ 3 min -> 90% Acetonitril @ 31 min -> 90% Acetonitril @ 34 min -> 10% Acetonitril @ 34.01 min) gereinigt.
- Ausbeute:: 35 mg (24 % d.Th.)
- HPLC:: Rt = 4.23 min (HPLC-Methode/Instrument 3)
- MW 562,56;: m/z gef.: 563
¹H-NMR (200 MHz, DMSO-d₆): δ = 11.71 (s, 1H); 10.40 (s, 1H); 9.16 (s, 1H); 8.40 (d, 1H); 8.26 (d, 2H); 8.13 (t, 1H); 8.04-7.94 (m, 3H); 7.53 (s, 1H); 7.24 (d, 1H); 7.14 (t, 1H); 6.77 (d, 1H); 1.36 (s, 3H); 1.08-1.03 (m, 2H); 0.62-0.57 (m, 2H).

### Beispiel 138

### 1-Methyl-N-[3-({[4-(5-{6-[(methylsulfonyl)amino]-2-pyridinyl}-1,2,4-oxadiazol-3-yl)phenyl]sulfonyl}amino)phenyl]cyclopropancarboxamid

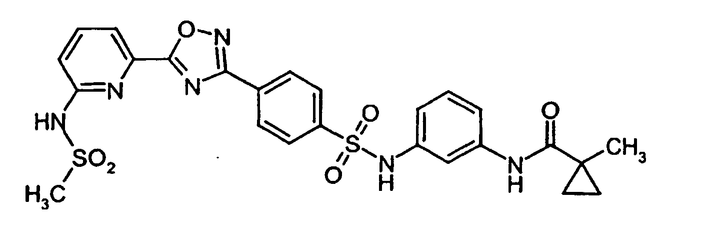

200 mg (0.38 mmol) der Verbindung aus Beispiel 134 werden in 10 ml THF gelöst und unter Argon mit 0.5 ml (6.18 mmol) Pyridin sowie 90 mg (0.75 mmol) Methansulfonsäurechlorid versetzt. Das Gemisch wird 16 h bei Raumtemperatur gerührt, dann i. Vak. das Lösungsmittel entfernt, der Rückstand in 5 ml Methanol aufgenommen, erneut i. Vak. eingeengt und das Rohprodukt mittels präparativer HPLC (CromSil C18, 250x30 mm, Fluß 50 ml/min, Runtime 35 min, Detektion bei 254 nm, Gradient 10% Acetonitril @ 3 min -> 90% Acetonitril @ 31 min -> 90% Acetonitril @ 34 min -> 10% Acetonitril @ 34.01 min) gereinigt.
- Ausbeute:: 82 mg (30% d.Th.)
- HPLC:: Rt = 4.30 min (HPLC-Methode/Instrument 3)
- MW 588.64;: m/z gef.: 589
¹H-NMR (200 MHz, DMSO-d₆): δ = 10.99 (br s, 1H); 10.48 (br s, 1H); 9.36 (s, 1H); 8.25 (d, 1H); 8.09-7.96 (m, 4H); 7.59-7.57 (m, 1H); 7.33-7.11 (m, 6H); 6.81 (d, 1H); 4.43 (d, 2H); 3.48 (s, 3H); 1.27-1.14 (m, 6H).

Weitere gemäß den erfindungsgemäßen Verfahren hergestellte, über die Position 3 verknüpfte 1,2,4-Oxadiazol-Derivate sind in Tabelle 4 aufgeführt:

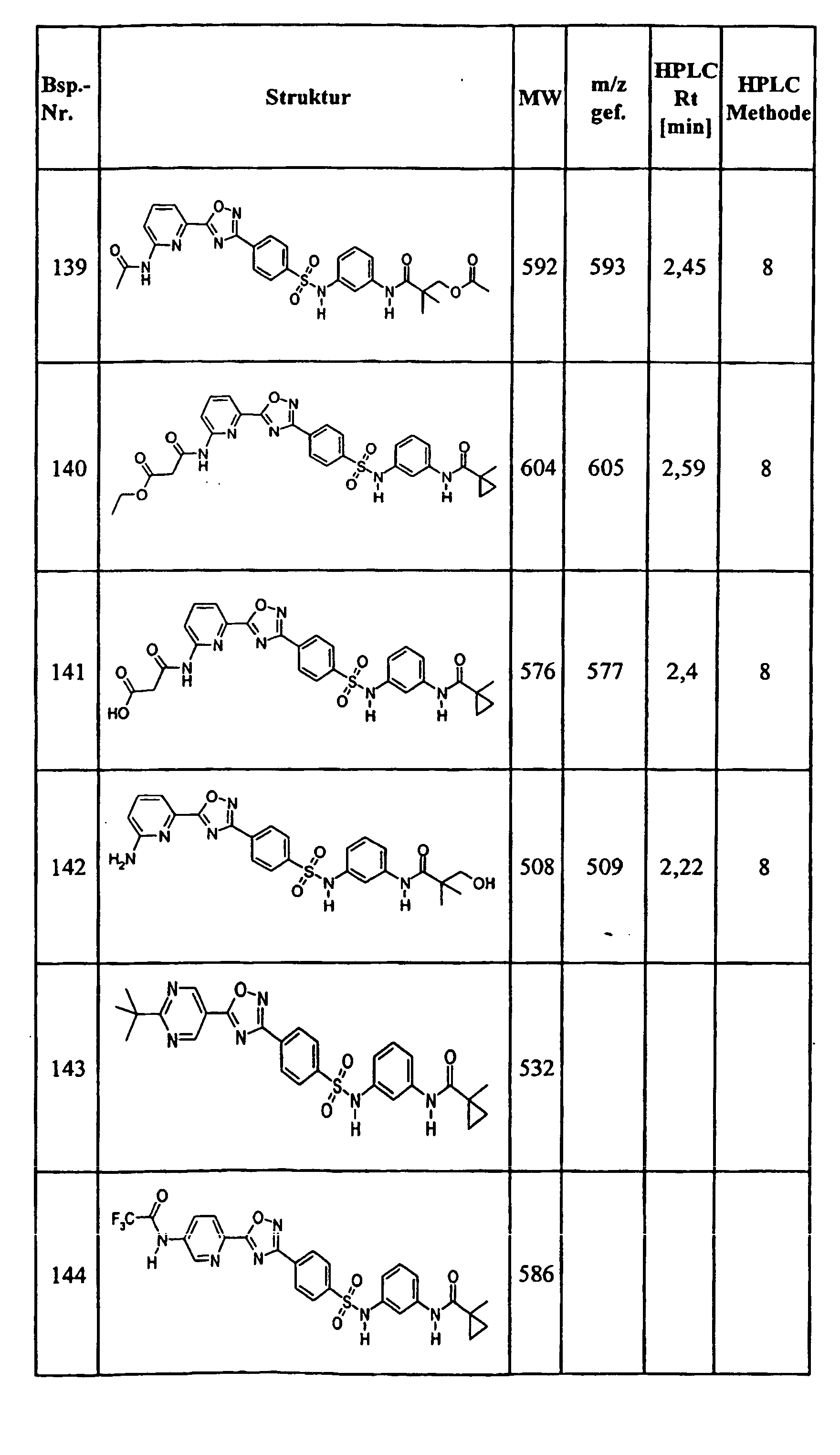

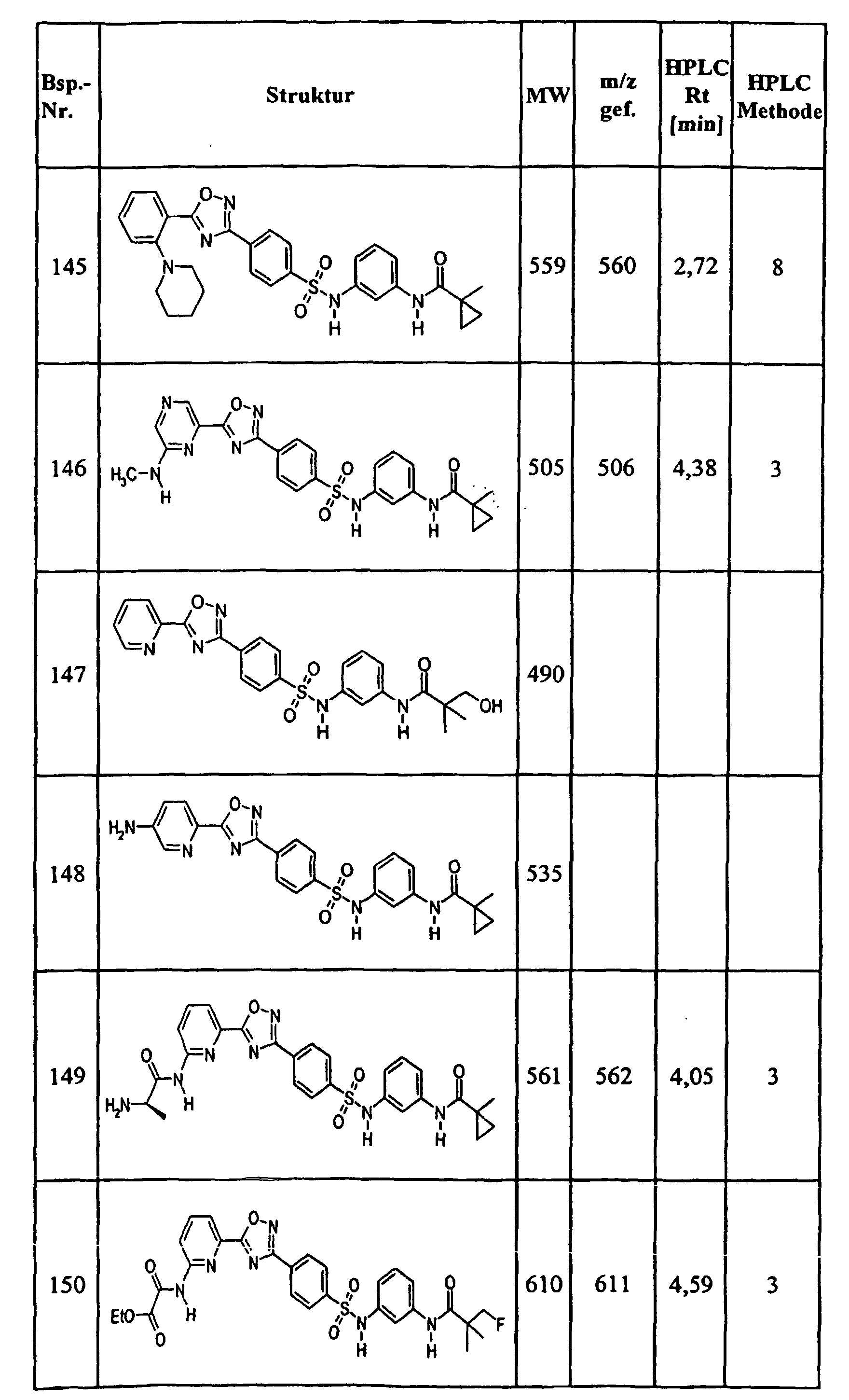

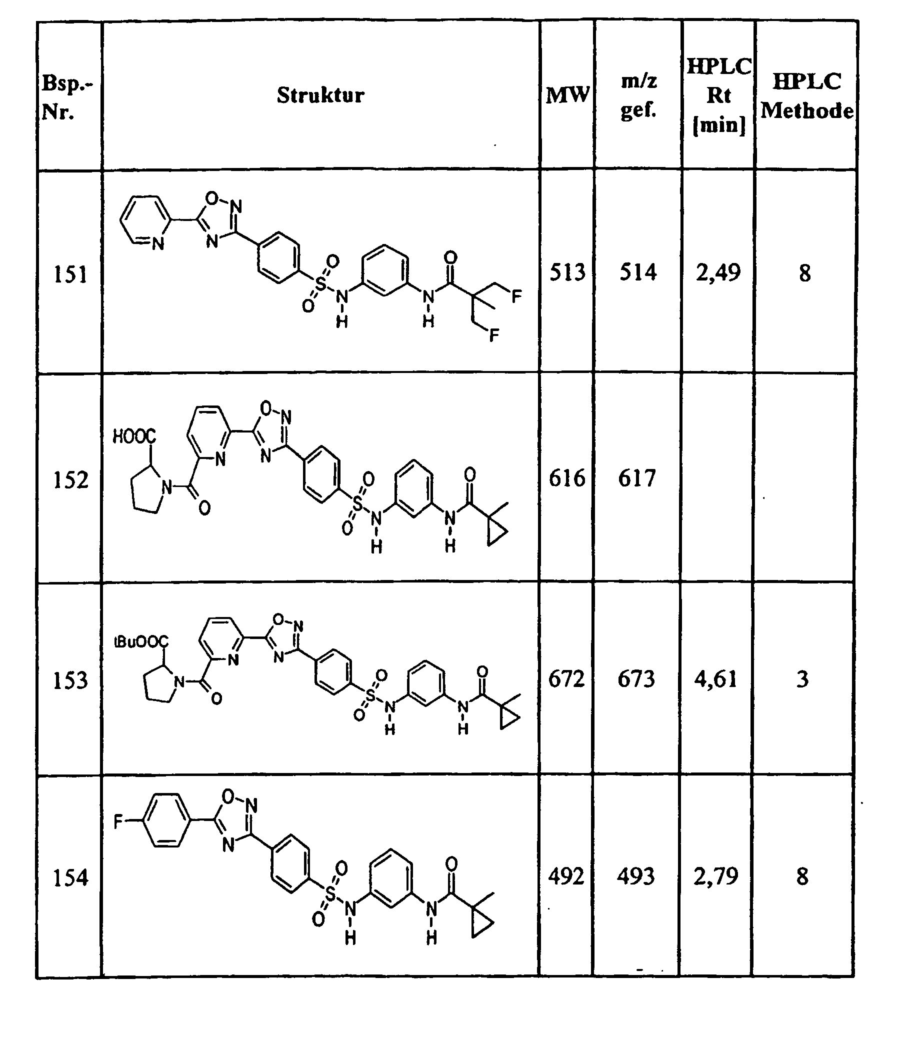

Die in den Ausführungsbeispielen und Tabellen aufgeführten Verbindungen wurden unter Anwendung der im folgenden beschriebenen LC-MS- und HPLC-Verfahren charakterisiert:

### Methode 1:

Säule: Kromasil C18, L-R Temperatur: 30°C, Fluss = 0.75 ml min⁻¹, Eluent: A = 0.01 M HClO₄, B = CH₃CN, Gradient: → 0.5 min 98 % A → 4.5 min 10 % A → 6.5 min 10 % A

### Methode 2:

Säule: Kromasil C18 60 x 2 mm, L-R Temperatur: 30°C, Fluss = 0.75 ml min⁻¹, Eluent: A = 0.01 M H₃PO₄, B = CH₃CN, Gradient: → 0.5 min 90 % A → 4.5 min 10 % A → 6.5 min 10%A

### Methode 3:

Säule: Kromasil C18 60 x 2 mm, L-R Temperatur: 30°C, Fluss = 0.75 ml min⁻¹, Eluent: A = 0.005 M HClO₄, B = CH₃CN, Gradient: → 0.5 min 98 % A → 4.5 min 10 % A → 6.5 min 10 % A

### Methode 4:

Säule: Symmetry C 18 2.1 x 150 mm, Säulenofen: 50°C, Fluss = 0.6 ml min⁻¹, Eluent: A = 0.6 g 30 %-ige HCl / 1 Wasser, B = CH₃CN, Gradient: 0.0 min 90 % A → 4.0 min 10 % A → 9 min 10%A

### Methode 5:

LC-MS: MHZ-2Q, Instrument Micromass Quattro LCZ
Säule: Symmetry C18 50 mm x 2.1 mm, 3.5 µm, Temperatur: 40°C, Fluss = 0.5 ml min⁻¹, Eluent A = CH₃CN + 0.1 % Ameisensäure, Eluent B = Wasser + 0.1 % Ameisensäure, Gradient: 0.0 min 10 % A → 4 min 90 % A → 6 min 90 % A

### Methode 6:

LC-MS: MHZ-2P, Instrument Micromass Platform LCZ
Säule: Symmetry C18 50 mm x 2.1 mm, 3.5 µm, Temperatur: 40°C, Fluss = 0.5 ml min⁻¹, Eluent A = CH₃CN + 0.1 % Ameisensäure, Eluent B = Wasser + 0.1 % Ameisensäure, Gradient: 0.0 min 10 % A → 4 min 90 % A → 6 min 90 % A

### Methode 7:

LC-MS: MHZ-7Q, Instrument Micromass Quattro LCZ
Säule: Symmetry C18 50 mm x 2.1 mm, 3.5 µm, Temperatur: 40°C, Fluss = 0.5 ml min⁻¹, Eluent A = CH₃CN + 0.1 % Ameisensäure, Eluent B = Wasser + 0.1 % Ameisensäure, Gradient: 0.0 min 5 % A → 1 min 5 % A → 5 min 90 % A → 6 min 90 % A

### Methode 8:

Säule: Symmetry C18 2.1 x 150 mm, Säulenofen: 50°C, Fluss = 0.9 ml min⁻¹, Eluent: A = 0.3 g 30 %-ige HCl / 1 Wasser, B = CH₃CN, Gradient: 0.0 min 90 % A → 3.0 min 10 % A → 6.0 min 10%A

### Methode 9:

HP1100, Säule: LiChroCart 75-5 LiChrospher 100 RP-18_5 µm, Säulenofen: 40°C, Fluss = 2.5 ml min⁻¹, Eluent: A = Wasser mit 0.05 % TFA, B = CH₃CN mit 0.05 % TFA, Gradient: 0.0 min 90 % A → 0.05 min 90 % A → 5.0 min 5 % A → 7.0 min 5 % A → 7.05 min 90 % A → 8.0 min 90 % A

## Patentansprüche

1. Verbindungen der allgemeinen Formel (I) 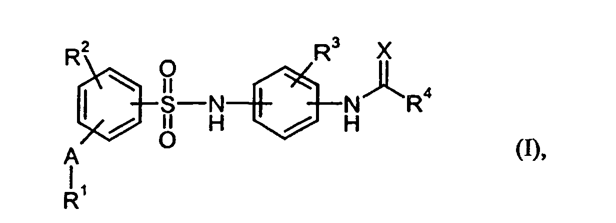 in der
R² und R³ gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 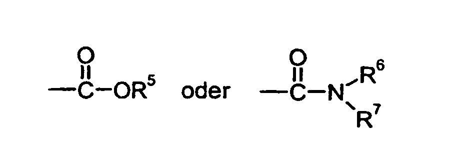 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
A für über ein C-Atom mit dem benachbarten Phenylring verknüpftes fünf- oder sechsgliedriges Heteroaryl mit ein bis drei Heteroatomen ausgewählt aus N, O und/oder S steht,
R¹ für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
R¹ substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heterocyclyl mit bis zu zwei Heteroatomen ausgewählt aus N, O und/oder S, 5- oder 6-gliedriges Heteroaryl mit einem oder mehreren Heteroatomen ausgewählt aus N, O und/oder S, -C(O)-O-R⁸, -C(O)-NR⁹R¹⁰, -NH-C(O)-R¹¹, -NH-C(O)-C(O)-R¹² und -NH-SO₂-R¹³ besteht,
wobei
R⁸, R⁹ und R¹⁰ gleich oder verschieden sind und jeweils für Wasserstoff oder (C₁-C₆)-Alkyl stehen,
oder
R⁹ und R¹⁰ zusammen mit dem Stickstoffatom, an das sie gebunden sind, einen 5- bis 6-gliedrigen Heterocyclus bilden, der ein weiteres Stickstoff- oder Sauerstoff-Heteroatom enthalten und ein- bis zweifach, gleich oder verschieden, durch (C₁-C₄)-Alkyl, welches seinerseits gegebenenfalls durch Hydroxy oder Amino substituiert ist, durch Amino, Hydroxy, (C₁-C₄)-Alkoxy, Oxo, Carboxyl oder durch (C₁-C₄)-Alkoxycarbonyl substituiert sein kann,
R¹¹ und R¹² gleich oder verschieden sind und jeweils für Trifluormethyl, (C₁-C₆)-Alkoxy, Hydroxy oder für (C₁-C₆)-Alkyl stehen, das gegebenen-falls ein- oder zweifach, gleich oder verschieden, durch Amino, (C₁-C₆)-Alkoxycarbonylamino, Mono-(C₁-C₆)-Acylamino, Hydroxy, Amidino, Guanidino, (C₁-C₆)-Alkoxycarbonyl, Carboxyl oder Phenyl substituiert ist,
und
R¹³ für (C₁-C₆)-Alkyl oder (C₆-C₁₀)-Aryl, die jeweils durch Halogen, Amino, Hydroxy, (C₁-C₄)-Alkoxy oder (C₁-C₄)-Alkyl substituiert sein können, steht,
R⁴ für (C₁-C₆)-Alkyl, das bis zu dreifach, gleich oder verschieden, durch Amino, Hydroxy, Halogen, (C₁-C₆)-Alkoxy, (C₁-C₅)-Alkanoyloxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
X für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

2. Verbindungen der allgemeinen Formel (I) nach Anspruch 1,
in der
R² und R³ gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 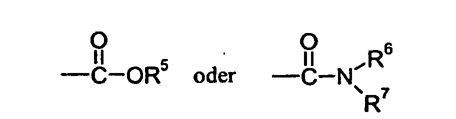 stehen,
in denen
R⁵, R⁶ und R⁷ gleich oder verschieden sind und jeweils für Wasser-stoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
A für über ein C-Atom mit dem benachbarten Phenylring verknüpftes fünf- oder sechsgliedriges Heteroaryl mit ein bis drei Heteroatomen ausgewählt aus N, O und/oder S steht,
R¹ für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
R¹ substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heteroaryl mit einem oder mehreren Heteroatomen ausgewählt aus N, O und/oder S, -C(O)-O-R⁸, -C(O)-NR⁹R¹⁰ und -NH-C(O)-R¹¹ besteht,
wobei
R⁸, R⁹ und R¹⁰ gleich oder verschieden sind und jeweils für Wasserstoff oder (C₁-C₆)-Alkyl stehen,
und
R¹¹ für (C₁-C₆)-Alkyl steht, das gegebenenfalls ein- oder zweifach, gleich oder verschieden, durch Amino, Hydroxy, Guanidino, Carboxyl oder Phenyl substituiert ist,
R⁴ für (C₁-C₆)-Alkyl, das bis zu dreifach, gleich oder verschieden, durch Amino, Hydroxy, Halogen, (C₁-C₆)-Alkoxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
X für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

3. Verbindungen der allgemeinen Formel (I) nach Anspruch 1,
in der
R² und R³ gleich oder verschieden sind und für Wasserstoff, Hydroxy, Halogen, Nitro, Cyano, Trifluormethyl, Trifluormethoxy, (C₁-C₆)-Alkyl, (C₁-C₆)-Alkoxy oder für eine Gruppe der Formel 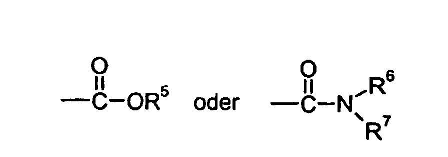 stehen,
in denen
R⁵, R⁶ und R⁷ gleich oder verschieden sind und jeweils für Wasserstoff oder (C₁-C₆)-Alkyl stehen, das seinerseits substituiert sein kann mit ein oder zwei Substituenten, ausgewählt aus der Gruppe bestehend aus Hydroxy, Halogen, Cyano, Trifluormethyl und Trifluormethoxy,
A für über ein C-Atom mit dem benachbarten Phenylring verknüpftes fünf- oder sechsgliedriges Heteroaryl mit ein bis drei Heteroatomen ausgewählt aus N, O und/oder S steht,
R¹ für (C₆-C₁₀)-Aryl, 5- bis 10-gliedriges Heteroaryl oder 5- bis 10-gliedriges Heterocyclyl mit jeweils ein bis drei Heteroatomen ausgewählt aus N, O und/oder S steht, wobei
R¹ substituiert sein kann mit bis zu drei Substituenten ausgewählt aus der Gruppe, die aus Hydroxy, Amino, Mono-(C₁-C₆)-Alkylamino, Di-(C₁-C₆)-Alkylamino, Halogen, Nitro, Cyano, Oxo, (C₁-C₆)-Alkyl, welches seinerseits durch Amino oder Hydroxy substituiert sein kann, (C₁-C₆)-Alkoxy, Phenyl, 5- oder 6-gliedriges Heteroaryl mit einem oder mehreren Heteroatomen ausgewählt aus N, O und/oder S, -C(O)-O-R⁸, -C(O)-NR⁹R¹⁰ und -NH-C(O)-R¹¹ besteht,
wobei
R⁸, R⁹ und R¹⁰ gleich oder verschieden sind und jeweils für Wasserstoff oder (C₁-C₆)-Alkyl stehen,
und
R¹¹ für (C₁-C₆)-Alkyl steht, das gegebenenfalls ein- oder zweifach, gleich oder verschieden, durch Amino, Hydroxy, Guanidino, Carboxyl oder Phenyl substituiert ist,
R⁴ für (C₁-C₆)-Alkyl, das bis zu dreifach, gleich oder verschieden, durch Amino, Hydroxy, Halogen, (C₁-C₆)-Alkoxy oder Phenyl, welches seinerseits gegebenenfalls ein- oder zweifach, gleich oder verschieden, durch Halogen, Nitro, Cyano, Amino oder Hydroxy substituiert ist, substituiert sein kann,
für (C₃-C₇)-Cycloalkyl, das bis zu dreifach, gleich oder verschieden, durch Amino, Hydroxy, Halogen, (C₁-C₆)-Alkoxy oder (C₁-C₆)-Alkyl, welches seinerseits gegebenenfalls bis zu dreifach, gleich oder verschieden, durch Amino, Hydroxy, Halogen oder (C₁-C₆)-Alkoxy substituiert ist, substituiert sein kann,
oder für (C₆-C₁₀)-Aryl, das gegebenenfalls ein- oder zweifach, gleich oder verschieden, durch Halogen, Nitro, Cyano, Amino oder Hydroxy substituiert ist, steht,
und in der
X für Sauerstoff steht,
und worin stickstoffhaltige Heterocyclen auch als N-Oxide vorliegen können, sowie deren Tautomere, Stereoisomere, stereoisomere Gemische und deren pharmakologisch verträglichen Salze.

4. Verbindungen der allgemeinen Formel (I) nach Anspruch 1,
in der
R² und R³ gleich oder verschieden sind und für Wasserstoff oder Halogen stehen,
A für den Rest (A-I) 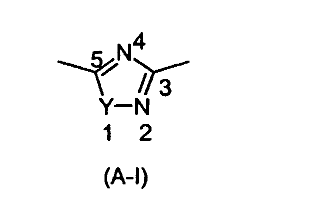 steht, der über eines der Kohlenstoffatome der Positionen 3 oder 5 mit dem benachbarten Phenylring verbunden ist,
und in dem
Y für Sauerstoff oder Schwefel steht,
oder
A für den Rest (A-II) 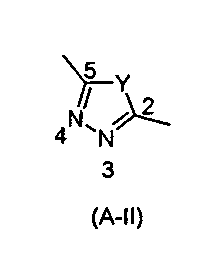 steht, der über eines der Kohlenstoffatome der Positionen 2 oder 5 mit dem benachbarten Phenylring verbunden ist,
und in dem
Y für Sauerstoff oder Schwefel steht,
R¹ für 5- bis 10-gliedriges Heteroaryl oder 5- oder 10-gliedriges Heterocyclyl mit jeweils bis zu drei Heteroatomen ausgewählt aus N, O und/oder S oder für Phenyl steht, wobei
R¹ substituiert sein kann mit ein bis drei Substituenten ausgewählt aus der Gruppe, die aus (C₁-C₄)-Alkyl, das seinerseits gegebenenfalls durch Hydroxy oder Amino substituiert ist, Hydroxy, Oxo, Halogen, Amino, Mono-(C₁-C₄)-alkylamino, Di-(C₁-C₄)-alkylamino und -NH-C(O)-R¹¹ besteht,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
R⁴ für (C₁-C₄)-Alkyl, das bis zu dreifach, gleich oder verschieden, durch Amino, Hydroxy, Fluor, Chlor oder (C₁-C₄)-Alkoxy substituiert sein kann, oder
für (C₃-C₅)-Cycloalkyl, das bis zu dreifach, gleich oder verschieden, durch Amino, Hydroxy, Fluor, Chlor, (C₁-C₄)-Alkoxy oder (C₁-C₄)-Alkyl, welches seinerseits gegebenenfalls bis zu dreifach, gleich oder verschieden, durch Amino, Hydroxy, Fluor, Chlor oder (C₁-C₄)-Alkoxy substituiert ist, substituiert sein kann, steht,
und in der
X für Sauerstoff oder Schwefel steht,
und worin stickstoffhaltige Heterocyclen auch als N-Oxide vorliegen können,
sowie deren Tautomere, Stereoisomere, stereoisomere Gemische und deren pharmakologisch verträglichen Salze.

5. Verbindungen der allgemeinen Formel (I) nach Anspruch 1,
in der
R² und R³ gleich oder verschieden sind und für Wasserstoff oder Halogen stehen,
A für den Rest (A-I) 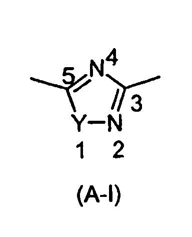 steht, der über eines der Kohlenstoffatome der Positionen 3 oder 5 mit dem benachbarten Phenylring verbunden ist,
und in dem
Y für Sauerstoff oder Schwefel steht,
oder
A für den Rest (A-II) 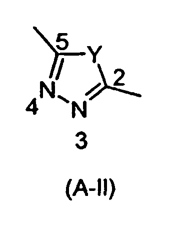 steht, der über eines der Kohlenstoffatome der Positionen 2 oder 5 mit dem benachbarten Phenylring verbunden ist,
und in dem
Y für Sauerstoff oder Schwefel steht,
R¹ für 5- bis 10-gliedriges Heteroaryl oder 5- oder 10-gliedriges Heterocyclyl mit jeweils bis zu drei Heteroatomen ausgewählt aus N, O und/oder S oder für Phenyl steht, wobei
R¹ substituiert sein kann mit ein bis drei Substituenten ausgewählt aus der Gruppe, die aus (C₁-C₄)-Alkyl, das seinerseits gegebenenfalls durch Hydroxy oder Amino substituiert ist, Hydroxy, Oxo, Halogen, Amino, Mono-(C₁-C₄)-alkylamino, Di-(C₁-C₄)-alkylamino und -NH-C(O)-R¹¹ besteht,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
R⁴ für (C₁-C₄)-Alkyl, das bis zu dreifach, gleich oder verschieden, durch Amino, Hydroxy, Fluor, Chlor oder (C₁-C₄)-Alkoxy substituiert sein kann, oder
für (C₃-C₅)-Cycloalkyl, das bis zu dreifach, gleich oder verschieden, durch Amino, Hydroxy, Fluor, Chlor, (C₁-C₄)-Alkoxy oder (C₁-C₄)-Alkyl, welches seinerseits gegebenenfalls bis zu dreifach, gleich oder verschieden, durch Amino, Hydroxy, Fluor, Chlor oder (C₁-C₄)-Alkoxy substituiert ist, substituiert sein kann, steht,
und in der
X für Sauerstoff steht,
sowie deren Tautomere, Stereoisomere, stereoisomere Gemische und deren pharmakologisch verträglichen Salze.

6. Verbindungen der allgemeinen Formel (I) nach Anspruch 1,
in der
R² und R³ für Wasserstoff stehen,
A für einen der Reste 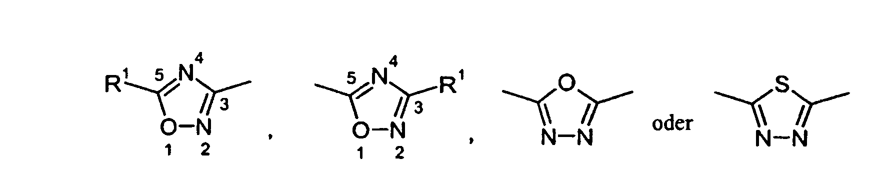 steht,
R¹ für einen Rest ausgewählt aus der Gruppe Phenyl, Pyridyl, Pyrazinyl, Thiazolyl, Thiadiazolyl, Chinolinyl, Isochinolinyl, Oxazolyl, Pyrazolyl, Imidazolyl, Pyrrolyl, Indolyl steht, wobei
R¹ substituiert sein kann mit ein bis zwei Substituenten ausgewählt aus der Gruppe Methyl, Aminomethyl, Hydroxy, Brom, Chlor, Fluor, Amino, Dimethylamino und -NH-C(O)-R¹¹,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
R⁴ für tert.-Butyl, das gegebenenfalls bis zu dreifach, gleich oder verschieden, durch Hydroxy, Fluor oder Chlor substituiert ist, oder
für Cyclopropyl oder Cyclobutyl steht, die durch Methyl substituiert sind, welches seinerseits gegebenenfalls durch Hydroxy, Fluor oder Chlor substituiert ist,
und in der
X für Sauerstoff steht,
und worin stickstoffhaltige Heterocyclen auch als N-Oxide vorliegen können,
sowie deren Tautomere, Stereoisomere, stereoisomere Gemische und deren pharmakologisch verträglichen Salze.

7. Verbindungen der allgemeinen Formel (I) nach Anspruch 1,
in der
R² und R³ für Wasserstoff stehen,
A für einen der Reste 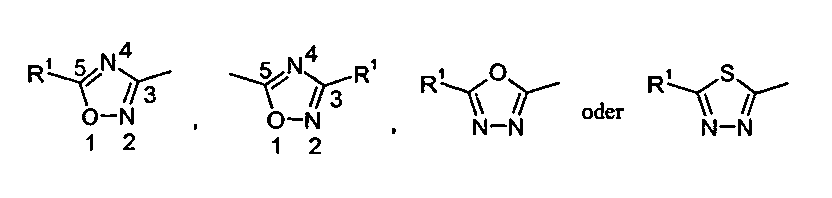 steht,
R¹ für einen Rest ausgewählt aus der Gruppe Phenyl, Pyridyl, Pyrazinyl, Thiazolyl, Thiadiazolyl, Chinolinyl, Isochinolinyl, Oxazolyl, Pyrazolyl, Imidazolyl, Pyrrolyl, Indolyl steht, wobei
R¹ substituiert sein kann mit ein bis zwei Substituenten ausgewählt aus der Gruppe Methyl, Aminomethyl, Hydroxy, Brom, Chlor, Fluor, Amino, Dimethylamino und -NH-C(O)-R¹¹,
wobei
R¹¹ für (C₁-C₆)-Alkyl steht, das gegebenenfalls ein- oder zweifach, gleich oder verschieden, durch Amino, Hydroxy, Guanidino oder Carboxyl substituiert ist,
R⁴ für tert.-Butyl, das gegebenenfalls bis zu dreifach, gleich oder verschieden, durch Hydroxy, Fluor oder Chlor substituiert ist, oder
für Cyclopropyl oder Cyclobutyl steht, die durch Methyl substituiert sind, welches seinerseits gegebenenfalls durch Hydroxy, Fluor oder Chlor substituiert ist,
und in der
X für Sauerstoff steht.

8. Verbindungen nach Anspruch 1 der allgemeinen Formel (Ia) 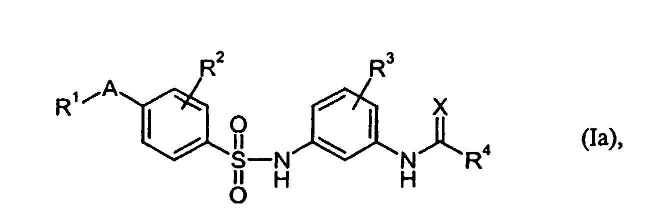 in der
R¹, R⁴, A und X die oben angegebenen Bedeutungen haben,
und
R² und R³ gleich oder verschieden sind und für Wasserstoff, Hydroxy, Halogen, Nitro, Cyano, Trifluormethyl, Trifluormethoxy, (C₁-C₆)-Alkyl oder (C₁-C₆)-Alkoxy stehen.

9. Verbindungen der allgemeinen Formel (I) nach Anspruch 1,
in denen
R⁴ für einen der Reste 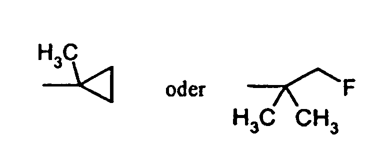
steht.

10. Verbindungen der allgemeinen Formel (I) nach Anspruch 1,
in denen
A für ein 3-verknüpftes 1,2,4-Oxadiazol steht.

11. Verbindungen nach Anspruch 1, die ausgewählt sind aus der Gruppe der folgenden Verbindungen:

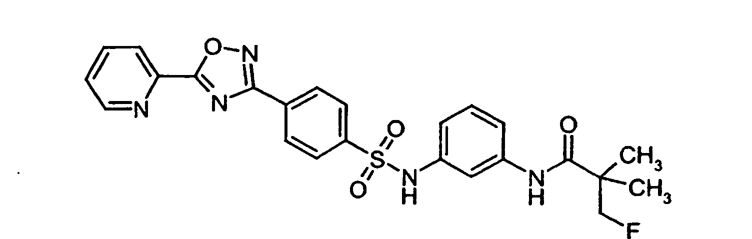

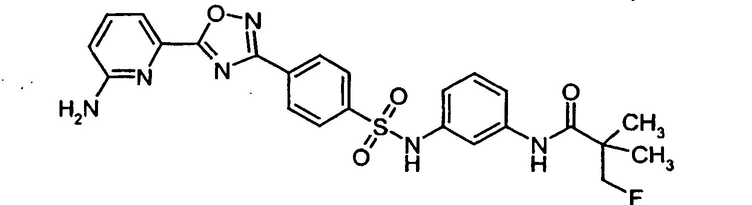

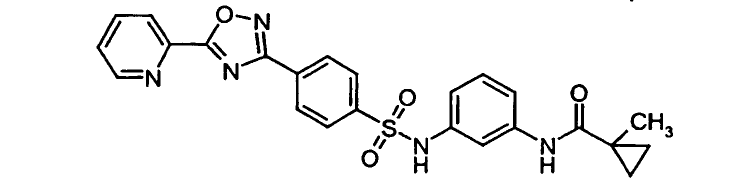

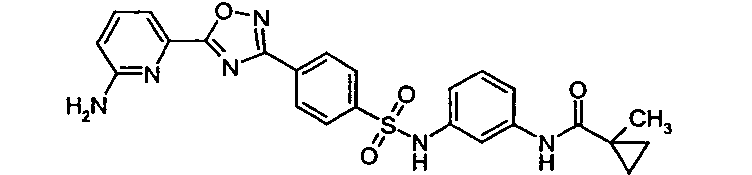

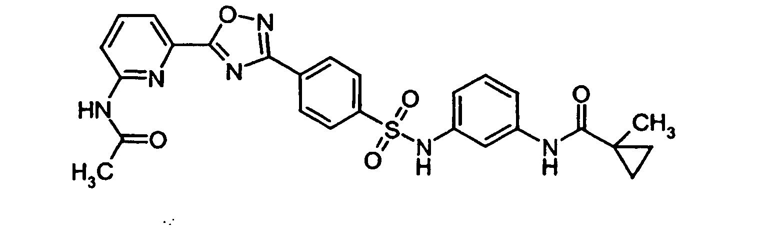

12. Verfahren zur Herstellung von Verbindungen der allgemeinen Formel (I) gemäß Anspruch 1, worin
A für den Rest (A-I) 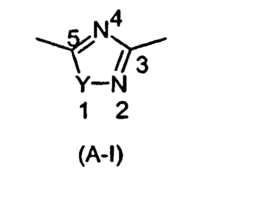
steht, der über eines der Kohlenstoffatome der Positionen 3 oder 5 mit dem benachbarten Phenylring verbunden ist, und in dem
Y für Sauerstoff steht,
indem man
Amidoxime der allgemeinen Formel [D-1] 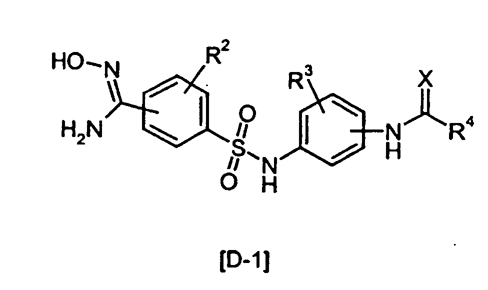 worin
X, R², R³ und R⁴ eine der oben angegebenen Bedeutungen haben,
mit einer Carbonsäure [E-1]
R¹-COOH [E-1]
worin R¹ die oben angegebene Bedeutung hat,
umsetzt,
oder
Sulfonamide der allgemeinen Formel [F-3] 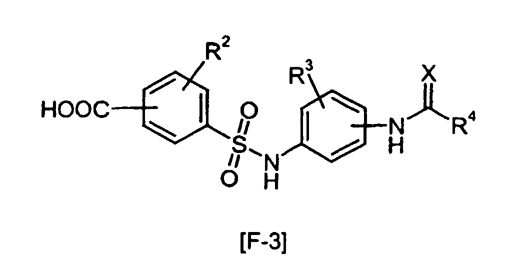 worin
X, R², R³ und R⁴ eine der oben angegebenen Bedeutungen haben,
mit einem Amid-Oxim der allgemeinen Formel [G-1] 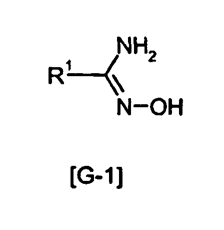 worin
R¹ die oben angegebene Bedeutung hat,
zu Verbindungen der allgemeinen Formel [G-2] kondensiert, 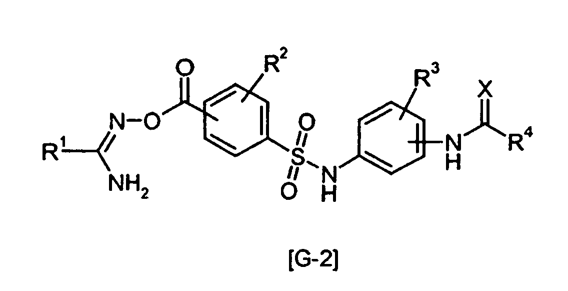 worin
R¹, R², R³, R⁴ und X die oben angegebene Bedeutung haben,
und die Verbindungen [G-2] nachfolgend unter Wasserabspaltung zu den Verbindungen der allgemeinen Formel (I) cyclisiert.

13. Verfahren zur Herstellung von Verbindungen der allgemeinen Formel (I) gemäß Anspruch 1, worin
A für den Rest (A-II) 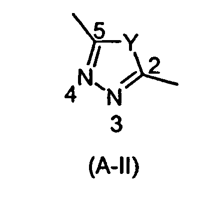
steht, der über eines der Kohlenstoffatome der Positionen 2 oder 5 mit dem benachbarten Phenylring verbunden ist,
und in dem
Y für Sauerstoff steht,
indem man Hydrazide der allgemeinen Formel [H-2] 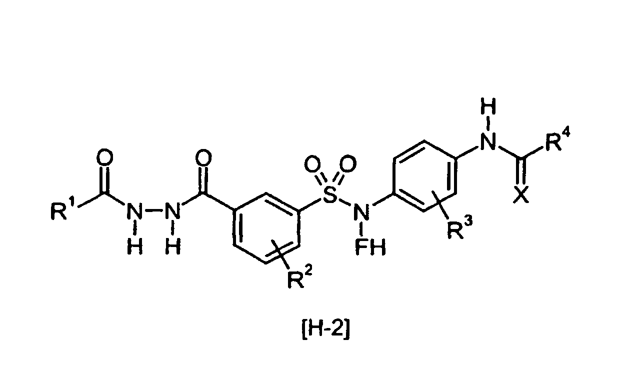 worin X, R¹, R², R³, R⁴ eine der oben angegebenen Bedeutungen hat, und
FH für Wasserstoff, eine Amino-Schutzgruppe, oder einen polymeren Träger steht,
unter Wasserabspaltung zu den Verbindungen der allgemeinen Formel (I) cyclisiert.

14. Verfahren zur Herstellung von Verbindungen der allgemeinen Formel (I) gemäß Anspruch 1, worin
X für Sauerstoff,
A für den Rest (A-II) 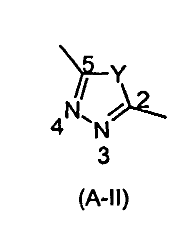 steht, der über eines der Kohlenstoffatome der Positionen 2 oder 5 mit dem benachbarten Phenylring verbunden ist,
und in dem
Y für Schwefel steht,
indem man Hydrazide der allgemeinen Formel [H-3] 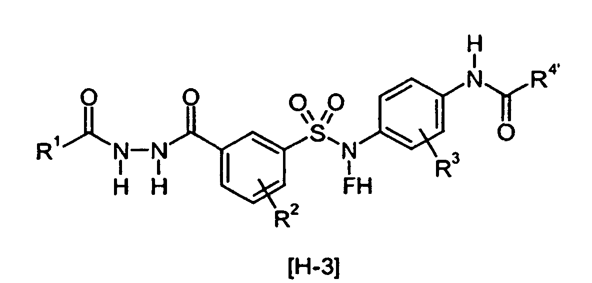 worin R¹, R², R³ die oben angegebene Bedeutung haben,
FH für Wasserstoff, eine Amino-Schutzgruppe oder einen polymeren Träger steht,
und
R^{4'} für (C₁-C₆)-Alkoxy, (C₁-C₆)-Alkenoxy oder Aralkoxy steht,
in Gegenwart eines Thio-Donors, vorzugsweise Lawesson's Reagenz, zu solchen Verbindungen der allgemeinen Formel (I) cyclisiert, in denen Y für Schwefel steht, dann die Gruppe -C(O)-R^{4'} abspaltet, und abschließend mit Verbindungen der allgemeinen Formel 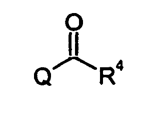 worin R⁴ und Q die oben angegebene Bedeutung haben,
umsetzt.

15. Verwendung von Verbindungen der allgemeinen Formel (I) gemäß irgendeinem der Ansprüche 1 bis 11 zur Herstellung von Arzneimitteln zur Prophylaxe oder Behandlung von Krankheiten.

16. Verwendung von Verbindungen der allgemeinen Formel (I) gemäß irgendeinem der Ansprüche 1 bis 11 zur Herstellung von Arzneimitteln.

17. Verwendung von Verbindungen der allgemeinen Formel (I) nach Anspruch 16, worin die Arzneimittel zur Bekämpfung viraler Erkrankungen sind.

18. Verwendung von Verbindungen der allgemeinen Formel (I) nach Anspruch 16 oder 17, worin die Arzneimittel zur Bekämpfung von Cytomegalievirus-Infektionen sind.

19. Arzneimittel enthaltend Verbindungen der allgemeinen Formel (I) nach Anspruch 1.

20. Verbindung der allgemeinen Formel (Ia) gemäß Anspruch 1 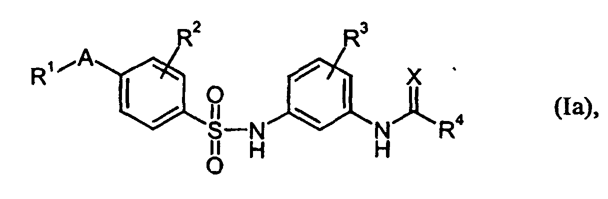 in der
R¹, R², R³, R⁴, A und X die oben angegebenen Bedeutungen haben.

## Claims

1. Compounds of the general formula (I) 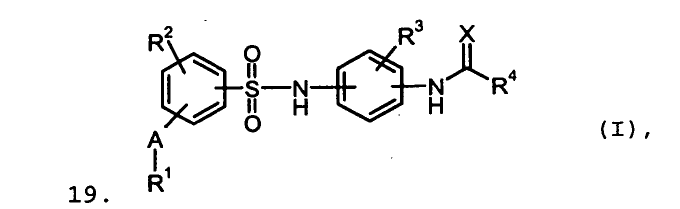 in which
R² and R³ are identical or different and represent hydrogen, hydroxyl, halogen, nitro, cyano, trifluoromethyl, trifluoromethoxy, (C₁-C₆)-alkyl, (C₁-C₆)-alkoxy or represent a group of the formula 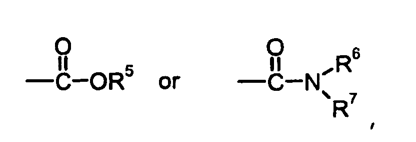 in which
R⁵, R⁶ and R⁷ are identical or different and each represents hydrogen or (C₁-C₆)-alkyl which for its part may be substituted by one or two substituents selected from the group consisting of hydroxyl, halogen, cyano, trifluoromethyl and trifluoromethoxy,
A represents five- or six-membered heteroaryl, which is attached to the adjacent phenyl ring via a C atom and has one to three heteroatoms selected from the group consisting of N, O and S,
R¹ represents (C₆-C₁₀)-aryl, 5- to 10-membered heteroaryl or 5- to 10-membered heterocyclyl having in each case one to three heteroatoms selected from the group consisting of N, O and S, where
R¹ may be substituted by up to three substituents selected from the group consisting of hydroxyl, amino, mono-(C₁-C₆)-alkylamino, di-(C₁-C₆)-alkylamino, halogen, nitro, cyano, oxo, (C₁-C₆)-alkyl, which for its part may be substituted by amino or hydroxyl, (C₁-C₆)-alkoxy, phenyl, 5- or 6-membered heterocyclyl having up to two heteroatoms selected from the group consisting of N, O and S, 5- or 6-membered heteroaryl having one or more heteroatoms selected from the group consisting of N, O and S, -C(O)-O-R⁸, -C(O)-NR⁹R¹⁰, -NH-C(O)-R¹¹, -NH-C(O)-C(O)-R¹² and -NH-SO₂-R¹³,
where
R⁸, R⁹ and R¹⁰ are identical or different and each represents hydrogen or (C₁-C₆)-alkyl,
or
R⁹ and R¹⁰ together with the nitrogen atom to which they are attached form a 5- to 6-membered heterocycle which may contain a further nitrogen or oxygen heteroatom and which may be mono- to disubstituted by identical or different substituents from the group consisting of (C₁-C₄)-alkyl, which for its part is optionally substituted by hydroxyl or amino, amino, hydroxyl, (C₁-C₄)-alkoxy, oxo, carboxyl and (C₁-C₄)-alkoxycarbonyl,
R¹¹ and R¹² are identical or different and each represents trifluoromethyl, (C₁-C₆)-alkoxy, hydroxyl or represents (C₁-C₆)-alkyl, which is optionally mono- or disubstituted by identical or different constituents from the group consisting of amino, (C₁-C₆)-alkoxycarbonylamino, mono-(C₁-C₆)-acylamino, hydroxyl, amidino, guanidino, (C₁-C₆)-alkoxycarbonyl, carboxyl and phenyl,
and
R¹³ represents (C₁-C₆)-alkyl or (C₆-C₁₀)-aryl which may in each case be substituted by halogen, amino, hydroxyl, (C₁-C₄)-alkoxy or (C₁-C₄)-alkyl,
R⁴ represents (C₁-C₆)-alkyl which may be substituted up to three times by identical or different substituents from the group consisting of amino, hydroxyl, halogen, (C₁-C₆)-alkoxy, (C₁-C₅)-alkanoyloxy and phenyl, which for its part is optionally mono- or disubstituted by identical or different substituents from the group consisting of halogen, nitro, cyano, amino and hydroxyl,
represents (C₃-C₇)-cycloalkyl which may be substituted up to three times by identical or different substituents from the group consisting of amino, hydroxyl, halogen, (C₁-C₆)-alkoxy and (C₁-C₆)-alkyl, which for its part is optionally substituted up to three times by identical or different substituents from the group consisting of amino, hydroxyl, halogen and (C₁-C₆)-alkoxy,
or represents (C₆-C₁₀)-aryl which is optionally mono- or disubstituted by identical or different substituents from the group consisting of halogen, nitro, cyano, amino and hydroxyl,
and in which
X represents oxygen or sulphur,
and in which nitrogen-containing heterocycles may also be present as N-oxides,
and their tautomers, stereoisomers, stereoisomer mixtures and their pharmacological acceptable salts.

2. Compounds of the general formula (I) according to Claim 1,
in which
R² and R³ are identical or different and represent hydrogen, hydroxyl, halogen, nitro, cyano, trifluoromethyl, trifluoromethoxy, (C₁-C₆)-alkyl, (C₁-C₆)-alkoxy or represent a group of the formula 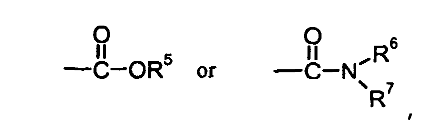 in which
R⁵, R⁶ and R⁷ are identical or different and each represents hydrogen or (C₁-C₆)-alkyl, which for its part may be substituted by one or two substituents selected from the group consisting of hydroxyl, halogen, cyano, trifluoromethyl and trifluoromethoxy,
A represents five- or six-membered heteroaryl, which is attached to the adjacent phenyl ring via a C atom and has one to three heteroatoms selected from the group consisting of N, O and S,
R¹ represents (C₆-C₁₀)-aryl, 5- to 10-membered heteroaryl or 5- to 10-membered heterocyclyl having in each case one to three heteroatoms selected from the group consisting of N, O and S, where
R¹ may be substituted by up to three substituents selected from the group consisting of hydroxyl, amino, mono-(C₁-C₆)-alkylamino, di-(C₁-C₆)-alkylamino, halogen, nitro, cyano, oxo, (C₁-C₆)-alkyl, which for its part may be substituted by amino or hydroxyl, (C₁-C₆)-alkoxy, phenyl, 5- or 6-membered heteroaryl having one or more heteroatoms selected from the group consisting of N, O and S, -C(O)-O-R⁸, -C(O)-NR⁹R¹⁰ and -NH-C(O)-R¹¹,
where
R⁸, R⁹ and R¹⁰ are identical or different and each represents hydrogen or (C₁-C₆)-alkyl,
and
R¹¹ represents (C₁-C₆)-alkyl which is optionally mono- or disubstituted by identical or different substituents from the group consisting of amino, hydroxyl, guanidino, carboxyl and phenyl,
R⁴ represents (C₁-C₆)-alkyl which may be substituted up to three times by identical or different substituents from the group consisting of amino, hydroxyl, halogen, (C₁-C₆)-alkoxy and phenyl, which for its part is optionally mono- or disubstituted by identical or different substituents from the group consisting of halogen, nitro, cyano, amino and hydroxyl,
represents (C₃-C₇)-cycloalkyl which may be substituted up to three times by identical or different substituents from the group consisting of amino, hydroxyl, halogen, (C₁-C₆)-alkoxy and (C₁-C₆)-alkyl, which for its part is optionally substituted up to three times by identical or different substituents from the group consisting of amino, hydroxyl, halogen and (C₁-C₆)-alkoxy,
or represents (C₆-C₁₀)-aryl which is optionally mono- or disubstituted by identical or different substituents from the group consisting of halogen, nitro, cyano, amino and hydroxyl,
and in which
X represents oxygen or sulphur,
and in which nitrogen-containing heterocycles may also be present as N-oxides,
and their tautomers, stereoisomers, stereoisomer mixtures and their pharmacologically acceptable salts.

3. Compounds of the general formula (I) according to Claim 1,
in which
R² and R³ are identical or different and represent hydrogen, hydroxyl, halogen, nitro, cyano, trifluoromethyl, trifluoromethoxy, (C₁-C₆)-alkyl, (C₁-C₆)-alkoxy or represent a group of the formula 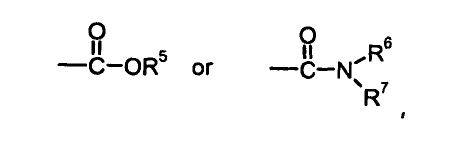 in which
R⁵, R⁶ and R⁷ are identical or different and each represents hydrogen or (C₁-C₆)-alkyl, which for its part may be substituted by one or two substituents selected from the group consisting of hydroxyl, halogen, cyano, trifluoromethyl and trifluoromethoxy,
A represents five- or six-membered heteroaryl, which is attached to the adjacent phenyl ring via a C atom and has one to three heteroatoms selected from the group consisting of N, O and S,
R¹ represents (C₆-C₁₀)-aryl, 5- to 10-membered heteroaryl or 5- to 10-membered heterocyclyl having in each case one to three heteroatoms selected from the group consisting of N, O and S, where
R¹ may be substituted by up to three substituents selected from the group consisting of hydroxyl, amino, mono-(C₁-C₆)-alkylamino, di-(C₁-C₆)-alkylamino, halogen, nitro, cyano, oxo, (C₁-C₆)-alkyl, which for its part may be substituted by amino or hydroxyl, (C₁-C₆)-alkoxy, phenyl, 5- or 6-membered heteroaryl having one or more heteroatoms selected from the group consisting of N, O and S, -C(O)-O-R⁸, -C(O)-NR⁹R¹⁰ and -NH-C(O)-R¹¹,
where
R⁸, R⁹ and R¹⁰ are identical or different and each represents hydrogen or (C₁-C₆)-alkyl,
and
R¹¹ represents (C₁-C₆)-alkyl, which is optionally mono- or disubstituted by identical or different substituents from the group consisting of amino, hydroxyl, guanidino, carboxyl and phenyl,
R⁴ represents (C₁-C₆)-alkyl which may be substituted up to three times by identical or different substituents from the group consisting of amino, hydroxyl, halogen, (C₁-C₆)-alkoxy and phenyl, which for its part is optionally mono- or disubstituted by identical or different substituents from the group consisting of halogen, nitro, cyano, amino and hydroxyl,
represents (C₃-C₇)-cycloalkyl which may be substituted up to three times by identical or different substituents from the group consisting of amino, hydroxyl, halogen, (C₁-C₆)-alkoxy and (C₁-C₆)-alkyl, which for its part is optionally substituted up to three times by identical or different substituents from the group consisting of amino, hydroxyl, halogen and (C₁-C₆)-alkoxy,
or represents (C₆-C₁₀)-aryl which is optionally mono- or disubstituted by identical or different substituents from the group consisting of halogen, nitro, cyano, amino and hydroxyl,
and in which
X represents oxygen,
and in which nitrogen-containing heterocycles may also be present as N-oxides,
and their tautomers, stereoisomers, stereoisomer mixtures and their pharmacologically acceptable salts.

4. Compounds of the general formula (I) according to Claim 1,
in which
R² and R³ are identical or different and represent hydrogen or halogen,
A represents the radical (A-I) 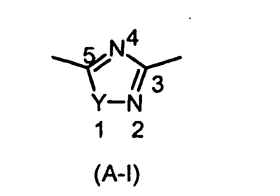 which is attached to the adjacent phenyl ring via one of the carbon atoms in position 3 or 5,
and in which
Y represents oxygen or sulphur,
or
A represents the radical (A-II) 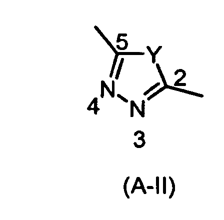 which is attached to the adjacent phenyl ring via one of the carbon atoms in position 2 or 5,
and in which
Y represents oxygen or sulphur,
R¹ represents 5- to 10-membered heteroaryl or 5- or 10-membered heterocyclyl having in each case up to three heteroatoms selected from the group consisting of N, O and S, or represents phenyl, where
R¹ may be substituted by one to three substituents selected from the group consisting of (C₁-C₄)-alkyl, which for its part is optionally substituted by hydroxyl or amino, hydroxyl, oxo, halogen, amino, mono-(C₁-C₄)-alkylamino, di-(C₁-C₄)-alkylamino and -NH-C(O)-R¹¹,
where
R¹¹ represents (C₁-C₆)-alkyl which is optionally mono- or disubstituted by identical or different substituents from the group consisting of amino, hydroxyl, guanidino and carboxyl,
R⁴ represents (C₁-C₄)-alkyl which may be substituted up to three times by identical or different substituents from the group consisting of amino, hydroxyl, fluorine, chlorine and (C₁-C₄)-alkoxy,
represents (C₃-C₅)-cycloalkyl, which may be substituted up to three times by identical or different substituents from the group consisting of amino, hydroxyl, fluorine, chlorine, (C₁-C₄)-alkoxy and (C₁-C₄)-alkyl, which for its part is optionally substituted up to three times by identical or different substituents from the group consisting of amino, hydroxyl, fluorine, chlorine and (C₁-C₄)-alkoxy,
and in which
X represents oxygen or sulphur,
and in which nitrogen-containing heterocycles may also be present as N-oxides,
and their tautomers, stereoisomers, stereoisomer mixtures and their pharmacologically acceptable salts.

5. Compounds of the general formula (I) according to Claim 1, in which
R² and R³ are identical or different and represent hydrogen or halogen,
A represents the radical (A-I) 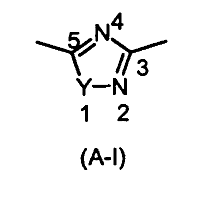 which is attached to the adjacent phenyl ring via one of the carbon atoms in position 3 or 5,
and in which
Y represents oxygen or sulphur,
or
A represents the radical (A-II) 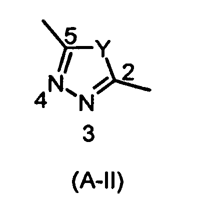 which is attached to the adjacent phenyl ring via one of the carbon atoms in position 2 or 5,
and in which
Y represents oxygen or sulphur,
R¹ represents 5- to 10-membered heteroaryl or 5- or 10-membered heterocyclyl having in each case up to three heteroatoms selected from the group consisting of N, O and S, or represents phenyl, where
R¹ may be substituted by one to three substituents selected from the group consisting of (C₁-C₄)-alkyl, which for its part is optionally substituted by hydroxyl or amino, hydroxyl, oxo, halogen, amino, mono-(C₁-C₄)-alkylamino, di-(C₁-C₄)-alkylamino and -NH-C(O)-R¹¹,
where
R¹¹ represents (C₁-C₆)-alkyl which is optionally mono- or disubstituted by identical or different substituents from the group consisting of amino, hydroxyl, guanidino and carboxyl,
R⁴ represents (C₁-C₄)-alkyl which may be substituted up to three times by identical or different substituents from the group consisting of amino, hydroxyl, fluorine, chlorine and (C₁-C₄)-alkoxy, or
represents (C₃-C₅)-cycloalkyl which may be substituted up to three times by identical or different substituents from the group consisting of amino, hydroxyl, fluorine, chlorine, (C₁-C₄)-alkoxy and (C₁-C₄)-alkyl, which for its part is optionally substituted up to three times by identical or different substituents from the group consisting of amino, hydroxyl, fluorine, chlorine and (C₁-C₄)-alkoxy,
and in which
X represents oxygen,
and their tautomers, stereoisomers, stereoisomer mixtures and their pharmacologically acceptable salts.

6. Compounds of the general formula (I) according to Claim 1,
in which
R² and R³ represent hydrogen,
A represents one of the radicals 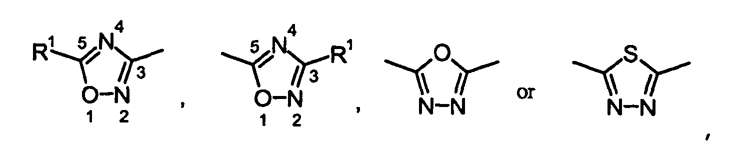
R¹ represents a radical selected from the group consisting of phenyl, pyridyl, pyrazinyl, thiazolyl, thiadiazolyl, quinolinyl, isoquinolinyl, oxazolyl, pyrazolyl, imidazolyl, pyrrolyl and indolyl, where
R¹ may be substituted by one or two substituents selected from the group consisting of methyl, aminomethyl, hydroxyl, bromine, chlorine, fluorine, amino, dimethylamino and -NH-C(O)-R¹¹,
where
R¹¹ represents (C₁-C₆)-alkyl which is optionally mono- or disubstituted by identical or different substituents from the group consisting of amino, hydroxyl, guanidino and carboxyl,
R⁴ represents tert-butyl which is optionally substituted up to three times by identical or different substituents from the group consisting of hydroxyl, fluorine and chlorine, or
represents cyclopropyl or cyclobutyl which are substituted by methyl, which for its part is optionally substituted by hydroxyl, fluorine or chlorine,
and in which
X represents oxygen,
and in which nitrogen-containing heterocycles may also be present as N-oxides,
and their tautomers, stereoisomers, stereoisomer mixtures and their pharmacologically acceptable salts.

7. Compounds of the general formula (I) according to Claim 1,
in which
R² and R³ represent hydrogen,
A represents one of the radicals 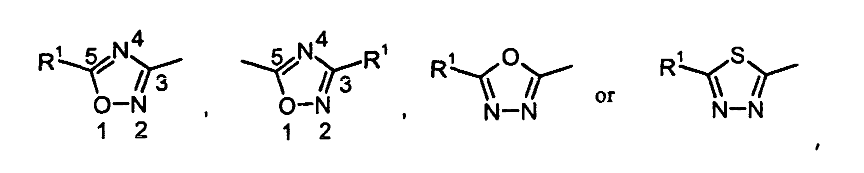
R¹ represents a radical selected from the group consisting of phenyl, pyridyl, pyrazinyl, thiazolyl, thiadiazolyl, quinolinyl, isoquinolinyl, oxazolyl, pyrazolyl, imidazolyl, pyrrolyl and indolyl, where
R¹ may be substituted by one to two substituents selected from the group consisting of methyl, aminomethyl, hydroxyl, bromine, chlorine, fluorine, amino, dimethylamino and -NH-C(O)-R¹¹,
where
R¹¹ represents (C₁-C₆)-alkyl which is optionally mono- or disubstituted by identical or different substituents from the group consisting of amino, hydroxyl, guanidino and carboxyl,
R⁴ represents tert-butyl which is optionally substituted up to three times by identical or different substituents from the group consisting of hydroxyl, fluorine and chlorine, or
represents cyclopropyl or cyclobutyl which are substituted by methyl, which for its part is optionally substituted by hydroxyl, fluorine or chlorine,
and in which
X represents oxygen.

8. Compounds according to Claim 1 of the general formula (Ia) 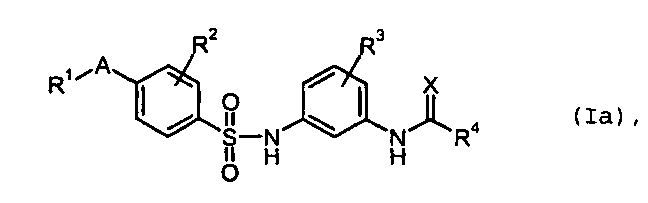 in which
R¹, R⁴, A and X are as defined above,
and
R² and R³ are identical or different and represent hydrogen, hydroxyl, halogen, nitro, cyano, trifluoromethyl, trifluoromethoxy, (C₁-C₆)-alkyl or (C₁-C₆)-alkoxy.

9. Compounds of the general formula (I) according to Claim 1,
in which
R⁴ represents one of the radicals

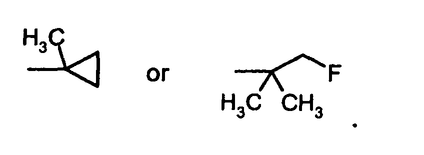

10. Compounds of the general formula (I) according to Claim 1,
in which
A represents a 1,2,4-oxadiazole attached via the 3-position.

11. Compounds according to Claim 1, selected from the group of the following compounds:

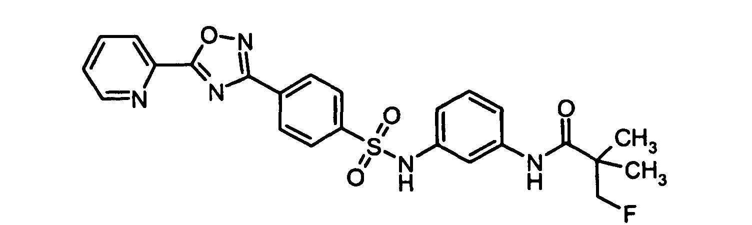

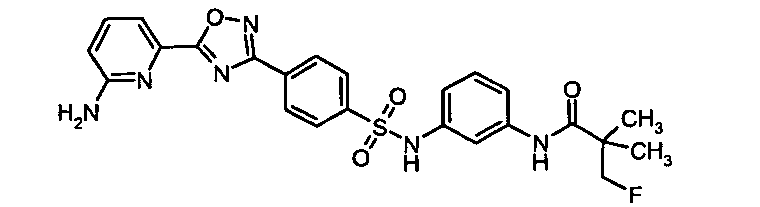

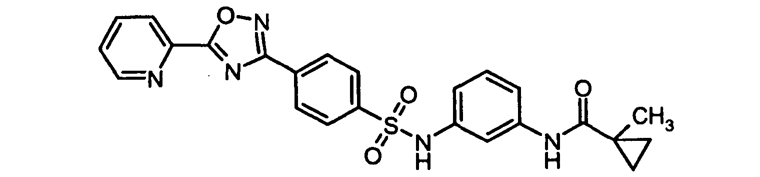

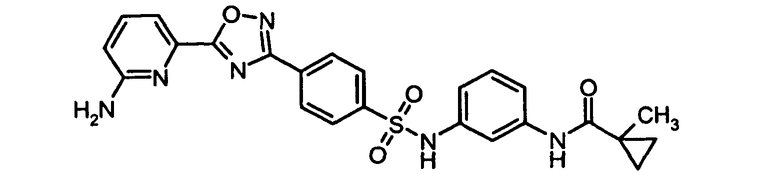

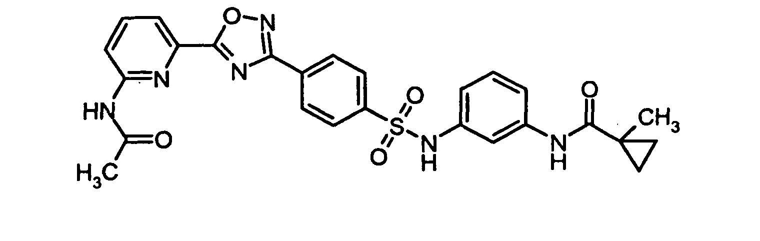

12. Process for preparing compounds of the general formula (I) according to Claim 1, in which
A represents the radical (A-I) 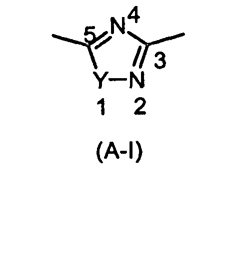 which is attached to the adjacent phenyl ring via one of the carbon atoms in position 3 or 5, and in which
Y represents oxygen,
by
reacting amidoximes of the general formula [D-1] 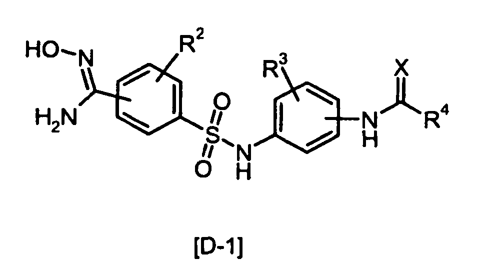 in which
X, R², R³ and R⁴ are as defined above,
with a carboxylic acid [E-1]
R¹-COOH [E-1]
in which R¹ is as defined above,
or
condensing sulphonamides of the general formula [F-3] 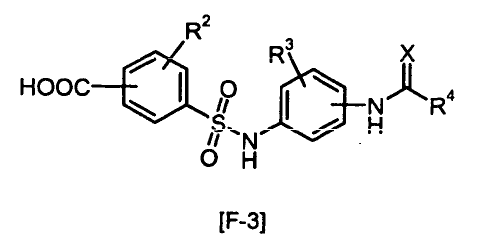 in which
X, R², R³ and R⁴ are as defined above,
with an amidoxime of the general formula [G-1] 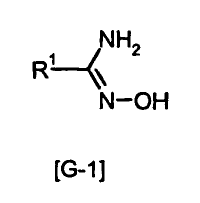 in which
R¹ is as defined above,
giving compounds of the general formula [G-2], 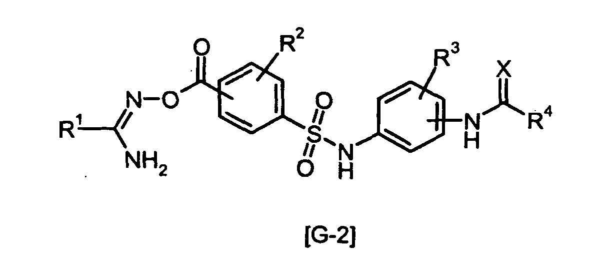 in which
R¹, R², R³, R⁴ and X are as defined above,
and subsequently cyclizing the compounds [G-2] with elimination of water, giving the compounds of the general formula (I).

13. Process for preparing compounds of the general formula (I) according to Claim 1 in which
A represents the radical (A-II) 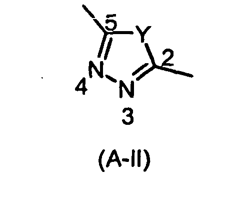
which is attached to the adjacent phenyl ring via one of the carbon atoms in position 2 or 5,
and in which
Y represents oxygen,
by cyclizing hydrazides of the general formula [H-2] 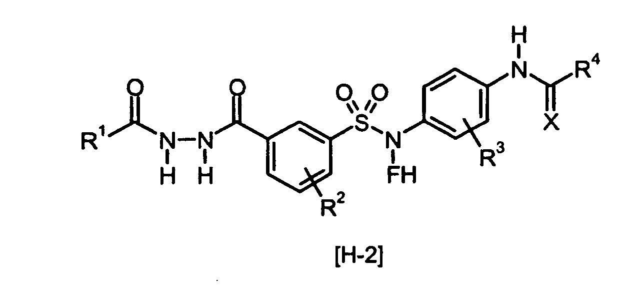 in which X, R¹, R², R³ and R⁴ are as defined above, and
FH represents hydrogen, an amino protective group or a polymeric support,
with elimination of water, to give compounds of the general formula (I).

14. Process for preparing compounds of the general formula (I) according to Claim 1, in which
X represents oxygen,
A represents the radical (A-II) 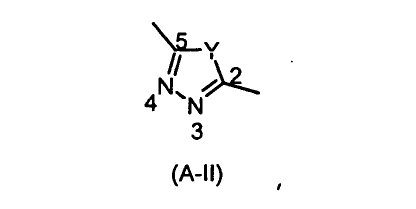
which is attached to the adjacent phenyl ring via one of the carbon atoms of position 2 or 5,
and in which
Y represents sulphur,
by cyclizing hydrazides of the general formula [H-3] 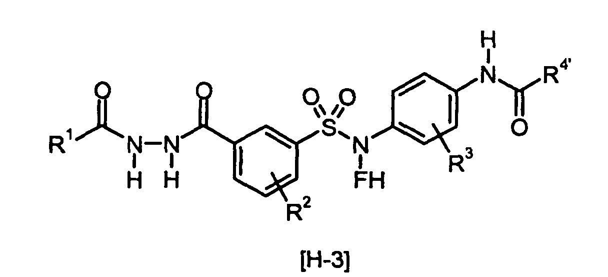 in which R¹, R², R³ are as defined above,
FH represents hydrogen, an amino protective group or a polymeric support,
and
R^{4'} represents (C₁-C₆)-alkoxy, (C₁-C₆)-alkenoxy or aralkoxy,
in the presence of a thio donor, preferably Lawesson's reagent, to give compounds of the general formula (I) in which Y represents sulphur, then removing group -C(O)-R^{4'} and finally reacting with compounds of the general formula 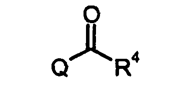 in which R⁴ and Q are as defined above.

15. Use of compounds of the general formula (I) according to any of Claims 1 to 11 for preparing medicaments for the prophylaxis or treatment of diseases.

16. Use of compounds of the general formula (I) according to any of Claims 1 to 11 for preparing medicaments.

17. Use of compounds of the general formula (I) according to Claim 16, where the medicaments are for controlling viral disorders.

18. Use of compounds of the general formula (I) according to Claim 16 or 17, where the medicaments are for controlling cytomegalovirus infections.

19. Medicaments, comprising compounds of the general formula (I) according to Claim 1.

20. Compounds of the general formula (Ia) according to Claim 1 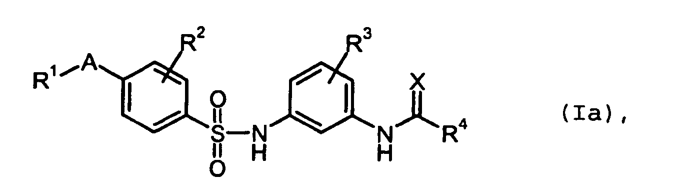 in which
R¹, R², R³, R⁴, A and X are as defined above.

## Revendications

1. Composés de formule générale (I) 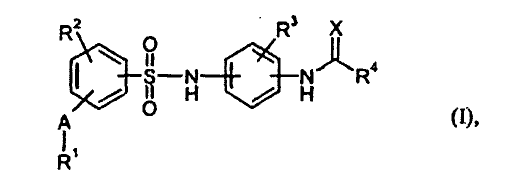 dans laquelle
R² et R³ sont identiques ou différents et représentent l'hydrogène, un groupe hydroxy, un halogène, un groupe nitro, cyano, un reste trifluorométhyle, trifluorométhoxy, alkyle en C₁ à C₆, alkoxy en C₁ à C₆ ou un groupe de formule 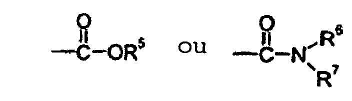 dans laquelle
R⁵, R⁶ et R⁷ sont identiques ou différents et représentent chacun l'hydrogène ou un reste alkyle en C₁ à C₆ qui peut lui-même porter un ou deux substituants choisis dans le groupe constitué d'hydroxy, halogène, cyano, trifluorométhyle et trifluorométhoxy,
A est un reste hétéroaryle pentagonal ou hexagonal lié au noyau phényle contigu par l'intermédiaire d'un atome de carbone et ayant un à trois hétéroatomes choisis entre N, O et/ou S,
R¹ est un reste aryle en C₆ à C₁₀, un reste hétéroaryle pentagonal à décagonal ou un reste hétérocyclyle pentagonal à décagonal ayant chacun jusqu'à trois hétéroatomes choisis entre N, O et/ou S,
R¹ pouvant porter jusqu'à trois substituants choisis dans le groupe comprenant hydroxy, amino, monoalkylamino en C₁ à C₆, di (alkyle en C₁ à C₆)amino, halogène, nitro, cyano, oxo, alkyle en C₁ à C₆ qui peut lui-même porter un substituant amino ou hydroxy, alkoxy en C₁ à C₆, phényle, hétérocyclyle pentagonal ou hexagonal ayant jusqu'à deux hétéroatomes choisis entre N, O et/ou S, hétéroaryle pentagonal ou hexagonal ayant un ou plusieurs hétéroatomes choisis entre N, O et/ou S, -C(O)-O-R⁸, -C-(O)-NR⁹R¹⁰, -NH-C(O)-R¹¹, -NH-C(O)-C(O)-R¹² et -NH-SO₂-R¹³,
R⁸, R⁹ et R¹⁰ étant identiques ou différents et représentant chacun un reste hydrogène ou alkyle en C₁ à C₆,
ou bien
R⁹ et R¹⁰ forment, conjointement avec l'atome d'azote auquel ils sont liés, un hétérocycle pentagonal ou hexagonal qui peut contenir un autre hétéroatome d'azote ou d'oxygène et qui peut être substitué une ou deux fois identiques ou différentes par un radical alkyle en C₁ à C₄ pouvant lui-même le cas échéant porter un substituant hydroxy ou amino, par un groupe amino, hydroxy, un reste alkoxy en C₁ à C₄, un groupe oxo, carboxyle ou par un reste (alkoxy en C₁ à C₄)carbonyle,
R¹¹ et R¹² sont identiques ou différents et représentent chacun un reste trifluorométhyle, alkoxy en C₁ à C₆, un groupe hydroxy ou un reste alkyle en C₁ à C₆, qui est éventuellement substitué une ou deux fois identiques ou différentes par un radical amino, (alkoxy en C₁ à C₆)carbonyle, monoacylamino en C₁ à C₆, hydroxy, amidino, guanidino, (alkoxy en C₁ à C₆)carbonyle, carboxyle ou phényle,
et
R¹³ représente un reste alkyle en C₁ à C₆ ou un reste aryle en C₆ à C₁₀ qui peuvent être substitués chacun par un radical halogéno, amino, hydroxy, alkoxy en C₁ à C₄ ou alkyle en C₁ à C₄,
R⁴ est un reste alkyle en C₁ à C₆ qui peut être substitué jusqu'à trois fois identiques ou différentes par un groupe amino, hydroxy, un halogène, un reste alkoxy en C₁ à C₆, alcanoyloxy en C₁ à C₅ ou un reste phényle, qui est lui-même éventuellement substitué une ou deux fois identiques ou différentes par un radical halogéno, nitro, cyano, amino ou hydroxy,
un reste cycloalkyle en C₃ à C₇ qui peut être substitué jusqu'à trois fois identiques ou différentes par un radical amino, hydroxy, halogéno, un reste alkoxy en C₁ à C₆ ou alkyle en C₁ à C₆ qui est lui-même éventuellement substitué jusqu'à trois fois identiques ou différentes par un radical amino, hydroxy, halogéno ou alkoxy en C₁ à C₆,
ou bien un reste aryle en C₆ à C₁₀ qui est éventuellement substitué une ou deux fois identiques ou différentes par un radical halogéno, nitro, cyano, amino ou hydroxy,
et dans laquelle
X représente l'oxygène ou le soufre,
et où des hétérocycles contenant de l'azote peuvent aussi exister comme N-oxydes,
ainsi que leurs tautomères, leurs stéréoisomères, leurs mélanges stéréoisomères et leurs sels acceptables du point de vue pharmaceutique.

2. Composés de formule générale (I) suivant la revendication 1,
formule dans laquelle
R² et R³ sont identiques ou différents et représentent l'hydrogène, un groupe hydroxy, un halogène, un groupe nitro, cyano, un reste trifluorométhyle, trifluorométhoxy, alkyle en C₁ à C₆, alkoxy en C₁ à C₆ ou un groupe de formule 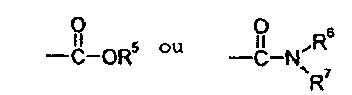 dans laquelle
R⁵, R⁶ et R⁷ sont identiques ou différents et représentent chacun l'hydrogène ou un reste alkyle en C₁ à C₆ qui peut lui-même porter un ou deux substituants choisis dans le groupe constitué d'hydroxy, halogène, cyano, trifluorométhyle et trifluorométhoxy,
A est un reste hétéroaryle pentagonal ou hexagonal lié au noyau phényle contigu par l'intermédiaire d'un atome de carbone et ayant un à trois hétéroatomes de la série N, O et/ou S,
R¹ est un reste aryle en C₆ à C₁₀, un reste hétéroaryle pentagonal à décagonal ou un reste hétérocyclyle pentagonal à décagonal ayant chacun un à trois hétéroatomes choisis en N, O et/ou S,
R¹ pouvant porter jusqu'à trois substituants choisis dans le groupe formé d'hydroxy, amino, monoalkylamino en C₁ à C₆, di(alkyle en C₁ à C₆)amino, halogéno, nitro, cyano, oxo, alkyle en C₁ à C₆ pouvant porter lui-même un substituant amino ou hydroxy, alkoxy en C₁ à C₆, phényle, hétéroaryle pentagonal ou hexagonal ayant un ou deux hétéroatomes choisis entre N, O et/ou S, -C(O)-O-R⁸, -C(O)-NR⁹R¹⁰ et -NH-C(O)-R¹¹,
R⁸, R⁹ et R¹⁰ étant identiques ou différents et représentant chacun l'hydrogène ou un reste alkyle en C₁ à C₆,
et
R¹¹ étant un reste alkyle en C₁ à C₆ qui est éventuellement substitué une ou deux fois identiques ou différentes par un radical amino, hydroxy, guanidino, carboxyle ou phényle,
R⁴ représente un reste alkyle en C₁ à C₆ qui peut être substitué jusqu'à trois fois identiques ou différentes par un groupe amino, hydroxy, un halogène, un reste alkoxy en C₁ à C₆, ou phényle, qui peut être lui-même substitué, le cas échéant, une ou deux fois identiques ou différentes par un radical halogéno, nitro, cyano, amino ou hydroxy,
un reste cycloalkyle en C₃ à C₇ qui peut être substitué jusqu'à trois fois identiques ou différentes par un radical amino, hydroxy, halogéno, alkoxy en C₁ à C₆ ou alkyle en C₁ à C₆, qui peut lui-même porter le cas échéant jusqu'à trois substituants amino, hydroxy, halogéno ou alkoxy en C₁ à C₆ identiques ou différents,
ou un reste aryle en C₆ à C₁₀, qui est éventuellement substitué une ou deux fois identiques ou différentes par un radical halogéno, nitro, cyano, amino ou hydroxy,
et dans laquelle
X représente l'oxygène ou le soufre,
et où des hétérocycles contenant de l'azote peuvent aussi se présenter sous forme de N-oxydes,
ainsi que leurs tautomères, leurs stéréoisomères, leurs mélanges stéréoisomères et leurs sels acceptables du point de vue pharmaceutique.

3. Composés de formule générale (I) suivant la revendication 1,
formule dans laquelle
R² et R³ sont identiques ou différents et représentent l'hydrogène, un groupe hydroxy, halogéno, nitro, cyano, un reste trifluorométhyle, trifluorométhoxy, alkyle en C₁ à C₆, alkoxy en C₁ à C₆ ou un groupe de formule 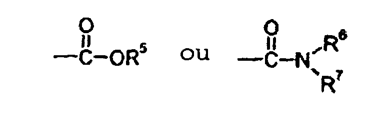 dans laquelle
R⁵, R⁶ et R⁷ sont identiques ou différents et représentent chacun l'hydrogène ou un reste alkyle en C₁ à C₆ qui peut lui-même porter un ou deux substituants choisis dans le groupe des substituants hydroxy, halogéno, cyano, trifluorométhyle et trifluorométhoxy,
A représente un reste hétéroaryle pentagonal ou hexagonal lié au noyau phényle contigu par l'intermédiaire d'un atome de carbone et ayant un à trois hétéroatomes choisis entre N, O et/ou S,
R¹ est un reste aryle en C₆ à C₁₀, hétéroaryle pentagonal à décagonal ou hétérocyclyle pentagonal à décagonal, ayant chacun un à trois hétéroatomes choisis entre N, O et/ou S,
R¹ pouvant porter jusqu'à trois substituants choisis dans le groupe des substituants hydroxy, amino, monoalkylamino en C₁ à C₆, di(alkyle en C₁ à C₆)amino, halogéno, nitro, cyano, oxo, alkyle en C₁ à C₆ qui peut lui-même porter un substituant amino ou hydroxy, alkoxy en C₁ à C₆, phényle, hétéroaryle pentagonal ou hexagonal ayant un ou plusieurs hétéroatomes choisis entre N, O et/ou S, -C(O)-O-R⁸, -C(O)-NR⁹R¹⁰ et -NH-C(O)-R¹¹,
R⁸, R⁹ et R¹⁰ étant identiques ou différents et représentant chacun l'hydrogène ou un reste alkyle en C₁ à C₆,
et
R¹¹ étant un reste alkyle en C₁ à C₆, qui porte éventuellement un ou deux substituants, identiques ou différents, amino, hydroxy, guanidino, carboxyle ou phényle,
R⁴ représente un reste alkyle en C₁ à C₆, qui peut être substitué une à trois fois identiques ou différentes par un substituant amino, hydroxy, halogéno, alkoxy en C₁ à C₆ ou phényle, qui est lui-même substitué éventuellement une ou deux fois identiques ou différentes par un radical halogéno, nitro, cyano, amino ou hydroxy,
un reste cycloalkyle en C₃ à C₇, qui peut être substitué jusqu'à trois fois identiques ou différentes par un radical amino, hydroxy, halogéno, alkoxy en C₁ à C₆ ou alkyle en C₁ à C₆ qui est lui-même substitué le cas échéant jusqu'à trois fois identiques ou différentes par un radical amino, hydroxy, halogéno ou alkoxy en C₁ à C₆,
ou un reste aryle en C₆ à C₁₀, qui est éventuellement substitué une ou deux fois identiques ou différentes par un radical halogéno, nitro, cyano, amino ou hydroxy,
et dans laquelle
X représente l'oxygène,
et où des hétérocycles contenant de l'azote peuvent aussi se présenter comme N-oxydes,
ainsi que leurs tautomères, leurs stéréoisomères, leurs mélanges stéréoisomères et leurs sels acceptables du point de vue pharmacologique.

4. Composés de formule générale (I) suivant la revendication 1,
formule dans laquelle
R² et R³ sont identiques ou différents et représentent l'hydrogène ou un halogène,
A représente le reste (A-I) 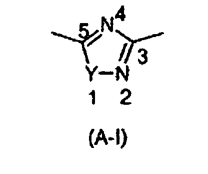 qui est lié au noyau phényle contigu par l'intermédiaire de l'un des atomes de carbone des positions 3 ou 5,
et dans lequel
Y représente l'oxygène ou le soufre,
ou bien
A représente le reste (A-II) 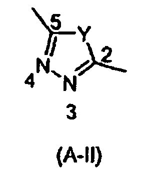 qui est lié au noyau phényle contigu par l'intermédiaire de l'un des atomes de carbone des positions 2 ou 5,
et dans lequel
Y représente l'oxygène ou le soufre,
R¹ est un reste hétéroaryle pentagonal à décagonal ou un reste hétérocyclyle pentagonal ou décagonal ayant chacun jusqu'à trois hétéroatomes choisis entre N, O et/ou S ou un reste phényle;
R¹ pouvant porter un à trois substituants choisis dans le groupe constitué d'alkyle en C₁ à C₄, qui est lui-même éventuellement substitué par un radical hydroxy ou amino, hydroxy, oxo, halogéno, amino, monoalkylamino en C₁ à C₄, di(alkyle en C₁ à C₄)amino et -NH-C(O)-R¹¹,
R¹¹ représentant un reste alkyle en C₁ à C₆ qui est éventuellement substitué une ou deux fois identiques ou différentes par un radical amino, hydroxy, guanidino ou carboxyle,
R⁴ étant un reste alkyle en C₁ à C₄ qui peut être substitué jusqu'à trois fois identiques ou différentes par un radical amino, hydroxy, fluoro, chloro ou alkoxy en C₁ à C₄, ou bien un reste cycloalkyle en C₃ à C₅ qui peut être substitué jusqu'à trois fois identiques ou différentes par un radical amino, hydroxy, fluoro, chloro, alkoxy en C₁ à C₄ ou par un radical alkyle en C₁ à C₄, qui est lui-même substitué le cas échéant jusqu'à trois fois identiques ou différentes par un radical amino, hydroxy, fluoro, chloro ou alkoxy en C₁ à C₄,
et dans laquelle
X représente l'oxygène ou le soufre,
et où des hétérocycles contenant de l'azote peuvent aussi exister sous forme de N-oxydes.
ainsi que leurs tautomères, leurs stéréoisomères, leurs mélanges stéréoisomères et leurs sels acceptables du point de vue pharmacologique.

5. Composés de formule générale (I) suivant la revendication 1,
formule dans laquelle
R² et R³ sont identiques ou différents et représentent l'hydrogène ou un halogène,
A représente le reste (A-I) 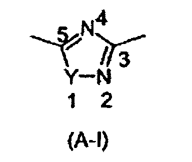 qui est lié au noyau phényle contigu par l'intermédiaire de l'un des atomes de carbone des positions 3 ou 5,
et dans lequel
Y représente l'oxygène ou le soufre,
ou bien
A représente le reste (A-II) 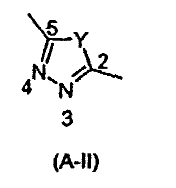 qui est lié au noyau phényle contigu par l'intermédiaire de l'un des atomes de carbone des positions 2 ou 5,
et dans lequel
Y représente l'oxygène ou le soufre,
R¹ est un reste hétéroaryle pentagonal à décagonal ou un reste hétérocyclyle pentagonal ou décagonal ayant chacun jusqu'à trois hétéroatomes choisis entre N, O et/ou S ou le reste phényle,
R¹ pouvant porter un à trois substituants choisis dans le groupe constitué des restes alkyle en C₁ à C₄, portant lui-même éventuellement un radical hydroxy ou amino, hydroxy, oxo, halogéno, amino, monoalkylamino en C₁ à C₄, di(alkyle en C₁ à C₄)amino et -NH-C(O)-R¹¹,
R¹¹ étant un reste alkyle en C₁ à C₆ qui est éventuellement substitué une ou deux fois identiques ou différentes par un radical amino, hydroxy, guanidino ou carboxyle,
R⁴ est un reste alkyle en C₁ à C₄, qui peut être substitué jusqu'à trois fois identiques ou différentes par un radical amino, hydroxy, fluoro, chloro ou alkoxy en C₁ à C₄, ou bien
un reste cycloalkyle en C₃ à C₅, qui peut être substitué jusqu'à trois fois identiques ou différentes par un radical amino, hydroxy, fluoro, chloro, alkoxy en C₁ à C₄ ou alkyle en C₁ à C₄ qui est lui-même éventuellement substitué jusqu'à trois fois identiques ou différentes par un radical amino, hydroxy, fluoro, chloro ou alkoxy en C₁ à C₄,
et dans laquelle
X représente l'oxygène
ainsi que leurs tautomères, leurs stéréoisomères, leurs mélanges stéréoisomères et leurs sels acceptables du point de vue pharmacologique.

6. Composés de formule générale (I) suivant la revendication 1,
formule dans laquelle
R² et R³ représentent l'hydrogène,
A représente l'un des restes 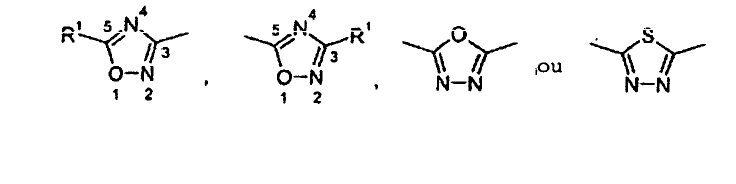
R¹ représente un reste choisi dans le groupe des restes phényle, pyridyle, pyrazinyle, thiazolyle, thiadiazolyle, chinolinyle, isochinolinyle, oxazolyle, pyrazolyle, imidazolyle, pyrrolyle, indolyle,
R¹ pouvant porter un ou deux substituants choisis dans le groupe des substituants méthyle, aminométhyle, hydroxy, bromo, chloro, fluoro, amino, diméthylamino et -NH-C(O)-R¹¹,
R¹¹ étant un reste alkyle en C₁ à C₆ qui est éventuellement substitué une ou deux fois identiques ou différentes par un radical amino, hydroxy, guanidino ou carboxyle,
R⁴ est un reste tertiobutyle, qui est éventuellement substitué jusqu'à trois fois identiques ou différentes par un radical hydroxy, fluoro ou chloro, ou bien
un reste cyclopropyle ou un reste cyclobutyle, qui sont substitués par un radical méthyle, lui-même éventuellement substitué par un radical hydroxy, fluoro ou chloro,
et dans laquelle
X représente l'oxygène,
et où des hétérocycles contenant de l'azote peuvent aussi exister comme N-oxydes,
ainsi que leur tautomères, leurs stéréoisomères, leurs mélanges stéréoisomères et leurs sels acceptables du point de vue pharmacologique.

7. Composés de formule générale (I) suivant la revendication 1,
dans laquelle
R² et R³ représentent l'hydrogène,
A représente l'un des restes 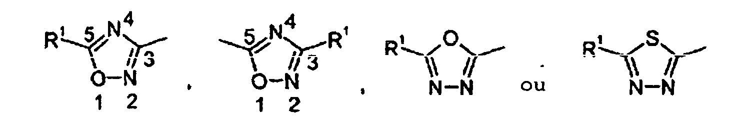
R¹ représente un reste choisi dans le groupe des restes phényle, pyridyle, pyrazinyle, thiazolyle, thiadiazolyle, chinolinyle, isochinolinyle, oxazolyle, pyrazolyle, imidazolyle, pyrrolyle, indolyle,
R¹ pouvant porter un ou deux substituants choisis dans le groupe des substituants méthyle, aminométhyle, hydroxy, bromo, chloro, fluoro, amino, diméthylamino ou -NH-C (O)-R¹¹,
R¹¹ représentant un reste alkyle en C₁ à C₆, qui est éventuellement substitué une ou deux fois identiques ou différentes par un radical amino, hydroxy, guanidino ou carboxyle,
R⁴ est un reste tertiobutyle, qui est éventuellement substitué jusqu'à trois fois identiques ou différentes par un radical hydroxy, fluoro ou chloro, ou bien
un reste cyclopropyle ou un reste cyclobutyle, qui sont substitués par un radical méthyle lui-même éventuellement substitué par un radical hydroxy, fluoro ou chloro,
et dans laquelle
X représente l'oxygène.

8. Composés suivant la revendication 1, de formule générale (Ia) 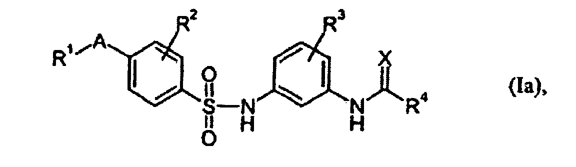 dans laquelle
R¹, R⁴, A et X ont les définitions indiquées ci-dessus,
et
R² et R³ sont identiques ou différents et représentent l'hydrogène, un groupe hydroxy, un halogène, un groupe nitro, cyano, un reste trifluorométhyle, trifluorométhoxy, alkyle en C₁ à C₆ ou alkoxy en C₁ à C₆.

9. Composés de formule générale (I) suivant la revendication 1,
dans lesquels
R⁴ représente l'un des restes

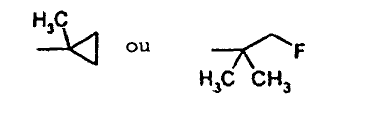

10. Composés de formule générale (I) suivant la revendication 1,
dans lesquels
A représente un 1,2,4-oxadiazole lié en position 3.

11. Composés suivant la revendication 1, qui sont choisis dans le groupe des composés suivants :

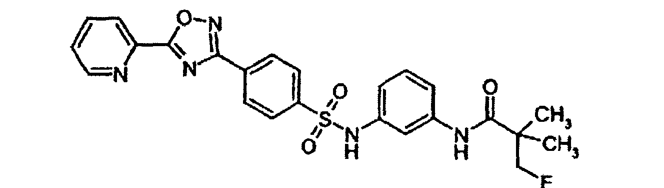

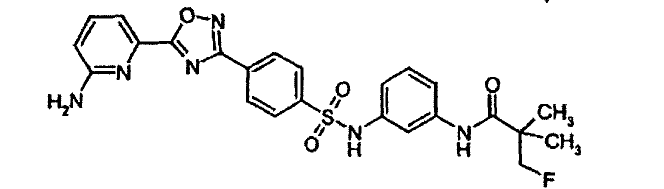

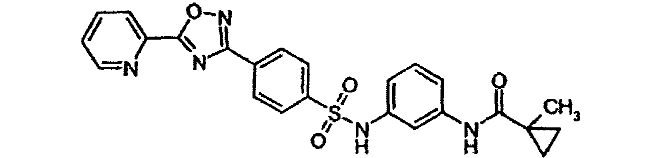

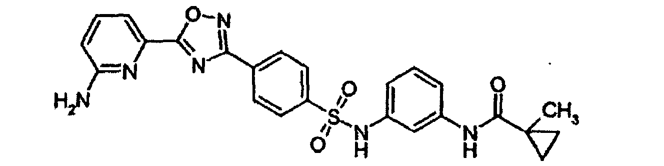

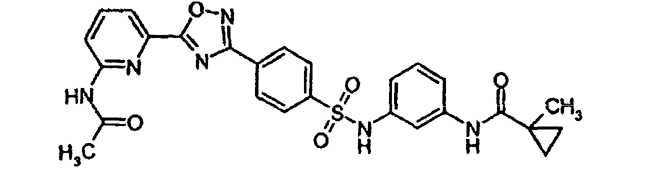

12. Procédé de production de composés de formule générale (I) suivant la revendication 1, formule dans laquelle
A représente le reste (A-I) 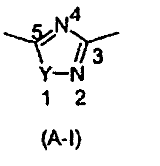
qui est lié au noyau phényle contigu par l'intermédiaire de l'un des atomes de carbone des positions 3 ou 5, et dans lequel
Y représente l'oxygène,
dans le procédé dans lequel
on fait réagir des amidoximes de formule générale [D-1] 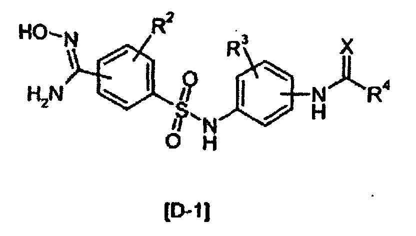 dans laquelle
X, R², R³ et R⁴ ont les définitions indiquées ci-dessus, avec un acide carboxylique [E-1]
R¹-COOH [E-1]
dans lequel R¹ a la définition indiquée ci-dessus,
ou bien
on condense des sulfonamides de formule générale [F-3] 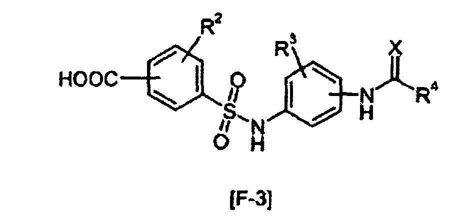 dans laquelle
X, R², R³ et R⁴ ont l'une des définitions indiquées ci-dessus,
avec une oxyme d'amide de formule générale [G-1] 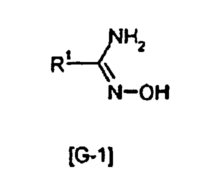 dans laquelle
R¹ a la définition indiquée ci-dessus,
pour obtenir des composés de formule générale [G-2] 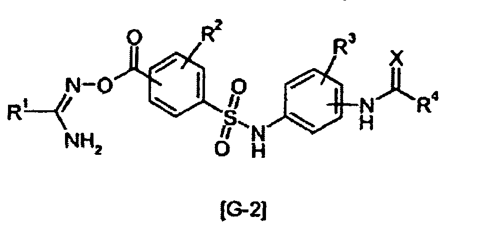 dans laquelle
R¹, R², R³, R⁴ et X ont la définition indiquée ci-dessus,
puis on cyclise les composés [G-2], avec élimination d'eau, en composés de formule générale (I).

13. Procédé de production de composés de formule générale (I) suivant la revendication 1, formule dans laquelle
A représente le reste (A-II) 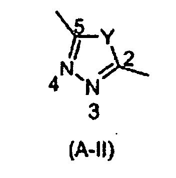
qui est lié au noyau phényle contigu par l'intermédiaire de l'un des atomes de carbone des positions 2 ou 5,
et dans lequel
Y représente l'oxygène,
procédé dans lequel on cyclise des hydrazides de formule générale [H-2] 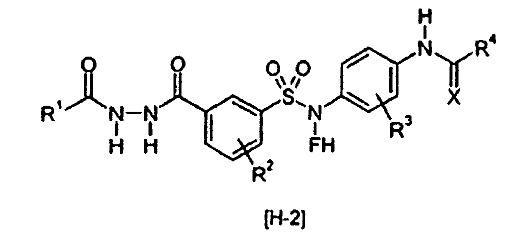 dans laquelle X, R¹, R², R³, R⁴ ont l'une des définitions indiquées ci-dessus, et
FH représente l'hydrogène, un groupe protégeant la fonction amino ou un support polymère,
en composés de formule générale (I) avec élimination d'eau.

14. Procédé de production de composés de formule générale (I) suivant la revendication 1, formule dans laquelle
X représente l'oxygène
A représente le reste (A-II) 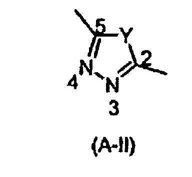
qui est lié au noyau phényle contigu par l'intermédiaire de l'un des atomes de carbone des positions 2 ou 5,
et dans lequel
Y représente le soufre,
procédé dans lequel on cyclise des hydrazides de formule générale [H-3] 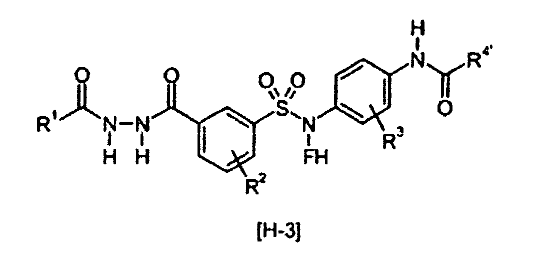 dans laquelle R¹, R², R³ ont la définition indiquée ci-dessus,
FH représente l'hydrogène, un groupe protégeant la fonction amino ou un support polymère,
et
R^{4'} est un reste alkoxy en C₁ à C₆, alcényloxy en C₁ à C₆ ou aralkoxy,
en présence d'un donneur de fonction thio, avantageusement en présence du réactif de Lawesson, pour obtenir des composés de formule générale (I) dans lesquels Y représente le soufre, puis on élimine le groupe -C(O)-R⁴', et on conduit finalement la réaction avec des composés de formule générale 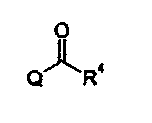 dans laquelle R⁴ et Q ont la définition indiquée ci-dessus.

15. Utilisation de composés de formule générale (I) suivant l'une quelconque des revendications 1 à 11 pour la préparation de médicaments destinés à la prophylaxie ou au traitement de maladies.

16. Utilisation de composés de formule générale (I) suivant l'une quelconque des revendications 1 à 11 pour la préparation de médicaments.

17. Utilisation de composés de formule générale (I) suivant la revendication 16, dans laquelle les médicaments sont destinés à lutter contre des maladies virales.

18. Utilisation de composés de formule générale (I) suivant la revendication 16 ou 17, dans laquelle les médicaments sont destinés à la lutte contre des infections à cytomégalovirus.

19. Médicament contenant des composés de formule générale (I) suivant la revendication 1.

20. Composé de formule générale (Ia) selon la revendication 1 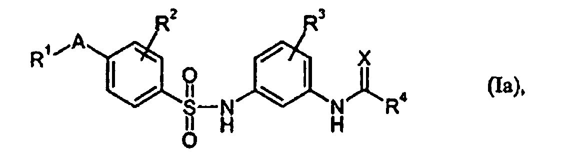 formule dans laquelle
R¹, R², R³, R⁴, A et X ont les définitions indiquées ci-dessus.
